(19) Europäisches Patentamt
European Patent Office
Office européen des brevets

(11) **EP 4 775 134 A1**

(12) **EUROPEAN PATENT APPLICATION**

(43) Date of publication:
**15.07.2026 Bulletin 2026/29**

(21) Application number: **26151036.6**

(22) Date of filing: **09.01.2026**

(51) International Patent Classification (IPC):
**A61B 5/00** (2006.01)   **A61B 5/1455** (2006.01)

(52) Cooperative Patent Classification (CPC):
**A61B 5/0075; A61B 5/1455; A61B 5/14551;
A61B 5/14552;** A61B 2562/04

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **09.01.2025 US 202563743499 P**

(71) Applicant: **NIRSense, Inc.
Richmond, VA 23223 (US)**

(72) Inventors:
• **ROUMENGOUS DE FESTES, Thibault Pierre
Thierry
Richmond, 23223 (US)**
• **RODENHAVER, Daniel C.
Richmond, 23223 (US)**
• **BOUTWELL, Ryan Casey
Richmond, 23223 (US)**

(74) Representative: **Lavoix
Bayerstraße 83
80335 München (DE)**

(54) **SYSTEMS AND METHODS FOR MONITORING OXIMETRY PARAMETERS OF TISSUE**

(57)   A system for detecting oximetry parameters of a user may include a reusable, wireless, and flexible near-infrared spectroscopy (NIRS) monitoring wearable system. The NIRS system may include a first component having a plurality of light sources configured to emit visible and infrared light at a plurality of wavelengths. The first component may have a first detector configured to detect a first plurality of signals from the plurality of light sources at a first source-detector distance of approximately 6 millimeters (mm) to 10 mm. The NIRS system may include a second component having a plurality of second detectors configured to detect a second plurality of signals from the plurality of light sources at multiple source-detector distances ranging from about 25 mm to about 45 mm. The NIRS system may include a memory, rechargeable battery, and processor configured to execute multi-depth tissue analysis.

EP 4 775 134 A1

**Description**

CROSS-REFERENCE TO RELATED APPLICATIONS

This application claims priority under 35 U.S.C. § 119 to U.S. Provisional Patent Application No. 63/743,499, filed January 9, 2025, entitled "WEARABLE TISSUE OXIMETER," the entire contents of which are fully incorporated herein by reference for all purposes.

FIELD OF INVENTION

The present disclosure relates to non-invasive physiological monitoring systems using optical spectroscopy, and more particularly to wearable near-infrared spectroscopy (NIRS) devices configured for continuous multi-depth tissue oxygenation measurement and assessment.

BACKGROUND

Near-infrared spectroscopy (NIRS) devices interrogate biological tissue using a selection of light wavelengths in the red and near-infrared (NIR) region of the electromagnetic spectrum. These wavelengths are particularly well suited for deep light penetration through tissue, versus lower wavelengths of light that are scattered or absorbed by confounding factors in the body and thus cannot reach the tissue depth of these red and NIR wavelengths. NIRS devices generally feature at least two wavelengths of light output in this range and at least one detector, and including additional optical elements can allow different depths of sensing.

Red and NIR wavelengths are particularly effective for non-invasively sensing different molecular states of hemoglobin in various body tissues. Unfortunately, existing NIRS devices are typically expensive, large desktop units with disintegrated sensor and processing systems. This lack of portability limits the usefulness of NIRS outside of the surgical suite, laboratory, and research environments. Some portable solutions include a sensor-only patch with wired communication to a separate portable, pocketable, or head-worn processing and communications unit. These changes represent only a nominal improvement, as the processing unit is itself not fully wearable and risks physically detaching from the sensor unit through movement or cable weight. These limitations greatly decrease the wearability and utility of such systems. These semi-ambulatory systems are also typically not designed to be used in parallel, where individual NIRS sensor systems work in tandem across the body or across a population to continually sense physiological features at multiple places using a common interface. Non-ambulatory systems can have more sensor inputs, but these are limited by the total number of ports designed into the physical system itself. Therefore, there exists a need for integrated NIRS systems and methods of using those systems to interrogate biological tissue.

Additionally, existing tissue oximeters continuously and non-invasively estimate tissue oxygenation (StO2) using NIRS technology. Photoplethysmography (PPG) monitors periodic oscillations of reflected light from the pulsation of arterial blood to continuously estimate arterial oxygen saturation through pulse oximetry (SpO2) as well as pulse rate (PR), respiratory rate (RR), perfusion, blood pressure, and other metrics using red and NIR optical components.

SUMMARY

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

In some embodiments, a system for detecting oximetry parameters of a user is disclosed. The system may include a reusable, wireless, and flexible near-infrared spectroscopy (NIRS) monitoring wearable system configured to be placed on a body part of a user. The NIRS system may include a first component having a plurality of light sources configured to emit visible and infrared light at a plurality of wavelengths including at least five distinct wavelengths. The first component may further have a first detector configured to detect a first plurality of signals from the plurality of light sources at a first source-detector distance of approximately 6 millimeters (mm) to 10 mm, wherein the first component has a first length of about 25 mm to about 45 mm, a first width of about 25 mm to about 45 mm, a first height of about 8 mm to about 16 mm, and comprises integrated optical isolation barriers. The NIRS system may include a second component having a plurality of second detectors configured to detect a second plurality of signals from the plurality of light sources at multiple source-detector distances ranging from about 25 mm to about 45 mm, wherein the second component has a second length of about 25 mm to about 45 mm, a second width of about 25 mm to about 45 mm, and a second height of about 8 mm to about 16 mm. The NIRS system may include a processor configured to execute one or more multi-depth tissue analysis algorithms, a memory configured to utilize a multi-threading processing approach and comprising dedicated buffering for continuous data streaming, and a rechargeable battery having a lifespan of at least two days with power management circuitry for adaptive power consumption. The NIRS system may be configured to continuously process the plurality of signals using differential path length factor corrections, and responsive to continuously processing the plurality of signals, simultaneously assess characteristics of optical tissue density at a plurality of tissue depths of the body part, and calculate one or more biometric properties of the user.

In some embodiments, a system for continuous physiological monitoring of a user is disclosed. The system may include a wearable near-infrared spectroscopy (NIRS) device configured to be positioned on a body part of the user. The NIRS device may include a plurality of light emitting diodes (LEDs) configured to emit light at multiple wavelengths including green, red, and near-infrared wavelengths, wherein the plurality of LEDs are arranged in a spatially distributed pattern with integrated thermal management and wavelength-specific power control. The NIRS device may include a plurality of photodetectors positioned at varying distances from the plurality of LEDs to enable multi-depth tissue analysis, wherein the plurality of photodetectors comprise photodiodes with dynamic gain adjustment. The NIRS device may include a flexible substrate connecting the plurality of LEDs and the plurality of photodetectors, wherein the flexible substrate enables the NIRS device to conform to contours of the body part. The NIRS device may include a processor configured to receive optical signals from the plurality of photodetectors with simultaneous multi-channel acquisition, apply signal processing algorithms to the optical signals to determine tissue oxygenation levels, and generate real-time physiological measurements based on the tissue oxygenation levels. The NIRS device may include a wireless communication module configured to transmit the real-time physiological measurements to an external device using encrypted data transmission with adaptive bandwidth management. The NIRS device may include a power source configured to provide continuous power to the NIRS device for extended monitoring periods with intelligent power management.

In some embodiments, a system for multi-parameter physiological assessment is disclosed. The system may include a portable near-infrared spectroscopy (NIRS) monitoring device configured for attachment to a user's body. The NIRS monitoring device may include an optical sensor array having multiple light sources and multiple photodetectors arranged in a predetermined pattern configured to enable multi-depth tissue analysis. The NIRS monitoring device may include optical isolation structures positioned between adjacent light sources to minimize optical crosstalk, wherein the optical isolation structures include wavelength-selective barriers with gradient refractive index profiles. The NIRS monitoring device may include a processor configured to selectively activate the multiple light sources and collect corresponding signals from the multiple photodetectors with synchronized timing control and adaptive exposure adjustment, and process the collected signals. Responsive to processing the collected signals, the processor may be configured to determine multiple physiological parameters including tissue oxygenation, and provide continuous monitoring output with automated alert generation based on physiological thresholds. The NIRS monitoring device may include a data storage component configured to store physiological measurements and analysis results with compression algorithms for extended data retention, and a user interface configured to display real-time physiological status information with customizable visualization modes and alerts.

The foregoing general description of the illustrative embodiments and the following detailed description thereof are merely exemplary aspects of the teachings of this disclosure and are not restrictive.

BRIEF DESCRIPTION OF FIGURES

Non-limiting and non-exhaustive examples are described with reference to the following figures.

FIG. 1 depicts an embodiment of a system used for detecting oximetry parameters of a user, in accordance with the present disclosure.

FIG. 2 depicts an embodiment of a substrate of the system of FIG. 1, in accordance with the present disclosure.

FIG. 3 depicts an embodiment of an electronics module of the system of FIG. 1, in accordance with the present disclosure.

FIG. 4 depicts a top perspective view of a NIRS monitoring wearable system, in accordance with the present disclosure.

FIG. 5 depicts a bottom perspective view of the NIRS monitoring wearable system of FIG. 4, in accordance with the present disclosure.

FIG. 6 depicts a side view of the NIRS monitoring wearable system of FIG. 4, in accordance with the present disclosure.

FIG. 7 depicts a bottom perspective view of the NIRS monitoring wearable system of FIG. 4, in accordance with the present disclosure.

FIG. 8 depicts a bottom view of the NIRS monitoring wearable system of FIG. 4, in accordance with the present disclosure.

FIG. 9 provides examples of the NIRS monitoring system of FIG. 4 adhered to different parts of a body of a user, in accordance with the present disclosure.

FIG. 10 is a flowchart of a method for detecting oximetry parameters of tissue, in accordance with the present disclosure.

FIG. 11 is a flowchart of a method for detecting oximetry parameters of tissue, in accordance with the present disclosure.

FIG. 12 is a flowchart of a method for detecting oximetry parameters of tissue, in accordance with the present disclosure.

DETAILED DESCRIPTION

This disclosure is not limited to the particular systems, devices, and methods described, as these may vary. The terminology used in the description is for the purpose of describing the particular versions or embodiments only, and is not intended to limit the scope of the disclosure.

The following terms shall have, for the purposes of this application, the respective meanings set forth below. Unless otherwise defined, all technical and scientific terms used herein have the same meanings as commonly understood by one of ordinary skill in the art. Nothing in this disclosure is to be construed as an admission that the embodiments described in this disclosure are not entitled to antedate such disclosure by virtue of prior invention.

As used herein, the singular forms "a," "an," and "the" include plural references, unless the context clearly dictates otherwise. Thus, for example, reference to a "fiber" is a reference to one or more fibers and equivalents thereof known to those skilled in the art, and so forth.

As used herein, the term "about" means plus or minus 10% of the numerical value of the number with which it is being used. For example, about 50 mm means in the range of 45 mm to 55 mm.

As used herein, the term "consists of" or "consisting of" means that the device or method includes only the elements, steps, or ingredients specifically recited in the particular claimed embodiment or claim.

In embodiments or claims where the term "comprising" is used as the transition phrase, such embodiments can also be envisioned with replacement of the term "comprising" with the terms "consisting of" or "consisting essentially of."

It is to be understood that the mention of one or more method steps does not preclude the presence of additional method steps or intervening method steps between those steps expressly identified. It is also to be understood that the listing of one or more method steps in any order does not preclude the performance of such one or more methods steps in alternative orders, nor does it preclude the simultaneous performance of any such one or more method steps. Similarly, it is also to be understood that the mention of one or more components in a device or system does not preclude the presence of additional components or intervening components between those components expressly identified.

It will be understood by those within the art that, in general, terms used herein are generally intended as "open" terms (for example, the term "including" should be interpreted as "including but not limited to," the term "having" should be interpreted as "having at least," the term "includes" should be interpreted as "includes but is not limited to," et cetera). While various compositions, methods, and devices are described in terms of "comprising" various components or steps (interpreted as meaning "including, but not limited to"), the compositions, methods, and devices can also "consist essentially of" or "consist of" the various components and steps, and such terminology should be interpreted as defining essentially closed-member groups.

In addition, even if a specific number is explicitly recited, those skilled in the art will recognize that such recitation should be interpreted to mean at least the recited number (for example, the bare recitation of "two components," without other modifiers, means at least two components, or two or more components). Furthermore, in those instances where a convention analogous to "at least one of A, B, and C, et cetera" is used, in general such a construction is intended in the sense one having skill in the art would understand the convention (for example, "a system having at least one of A, B, and C" would include but not be limited to systems that have A alone, B alone, C alone, A and B together, A and C together, B and C together, and/or A, B, and C together, et cetera). In those instances where a convention analogous to "at least one of A, B, or C, et cetera" is used, in general such a construction is intended in the sense one having skill in the art would understand the convention (for example, "a system having at least one of A, B, or C" would include but not be limited to systems that have A alone, B alone, C alone, A and B together, A and C together, B and C together, and/or A, B, and C together, et cetera). It will be further understood by those within the art that virtually any disjunctive word and/or phrase presenting two or more alternative terms, whether in the description, sample embodiments, or drawings, should be understood to contemplate the possibilities of including one of the terms, either of the terms, or both terms. For example, the phrase "A or B" will be understood to include the possibilities of "A" or "B" or "A and B."

Furthermore, where features of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, et cetera. As a non-limiting example, each range discussed herein can be readily broken down into a lower third, middle third and upper third, et cetera. As will also be understood by one skilled in the art all language such as "up to," "at least," and the like include the number recited and refer to ranges that can be subsequently broken down into subranges as discussed above. Finally, as will be understood by one skilled in the art, a range includes each individual member. Thus, for example, a group having 1-3 components refers to groups having 1, 2, or 3 components. Similarly, a group having 1-5 components refers to groups having 1, 2, 3, 4, or 5 components, and so forth.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity.

Near-infrared spectroscopy (NIRS) represents a non-invasive optical technique for measuring tissue oxygenation and perfusion in biological tissues. NIRS-based systems may analyze the absorption of specific wavelengths of near-infrared light to estimate the oxygenation of microvascular tissues at depths of up to approximately 5 centimeters (cm) (e.g., up to approximately 5.0 cm, 4.5 cm, 4.0 cm, 3.5 cm, 3.0 cm, 2.5 cm, 2.0 cm, etc.) below the skin surface. Unlike pulse oximetry, which relies on pulsatile blood to estimate arterial oxygen saturation, NIRS-based tissue oximetry may be sensitive to changes in venous and capillary blood oxygen content, thereby providing a more comprehensive assessment of tissue oxygenation status.

Photoplethysmography (PPG) represents another optical monitoring technique that may monitor periodic oscillations in tissue optical density. PPG-based systems may continuously estimate non-invasive arterial oxygen saturation values through pulse oximetry ($SpO_2$) as well as pulse rate (PR) and respiratory rate (RR) using red and near-infrared optical components. The combination of NIRS and PPG technologies into a single monitoring platform may enable simultaneous measurement of both deep tissue oxygenation and arterial blood oxygenation, providing complementary measures of cardiopulmonary function.

Wearable NIRS monitoring systems may be configured to detect backscattered light from multiple tissue depths, enabling continuous assessment of changes in optical density from underlying tissue. In some cases, such systems may include multiple red and near-infrared light sources positioned at varying distances from optical detectors. The calculated tissue oxygenation may be a function of the difference between the amount of light emitted and collected by the sensor. Periodic oscillations in tissue optical density may be visible in PPG signals and may correlate with pulse rate and respiratory rate measurements.

Tissue oximeters using NIRS technology may estimate non-invasive tissue oxygenation values by measuring the absorption characteristics of hemoglobin at different wavelengths. Oxygenated hemoglobin and deoxygenated hemoglobin exhibit different absorption spectra in the near-infrared range, and NIRS systems may exploit these differences to calculate tissue oxygen saturation ($StO_2$). In some cases, NIRS systems may also measure changes in relative hemoglobin concentrations, providing additional information about tissue perfusion and blood volume.

Pulse oximetry may provide an estimation of arterial blood oxygen saturation recorded from a PPG signal that is sensitive to the pulsatile component in arterial blood. This reliance upon pulsatility may result in PPG data being limited to signals related to arterial blood. $SpO_2$ measurements may be collected at peripheral sites such as the finger in transmission mode or the wrist in reflectance mode, and may serve as a systemic measure of lung and heart performance. In contrast, NIRS-based tissue oximetry may use multi-wavelength infrared light sources with longer source-detector distances to allow deep oxygenation monitoring below the skin surface.

The combination of PPG and NIRS sensing modalities into a unified monitoring system may allow for comprehensive monitoring of oxygenated blood supply, demand, and consumption in both superficial and deep tissues simultaneously. Pulse oximetry may allow continuous assessment of the ability of a patient's lungs to deliver oxygen to arterial hemoglobin, while tissue oximetry may enable continuous monitoring of cardiovascular function to deliver oxygenated blood to target tissues. Together, these features may allow comprehensive and non-invasive monitoring of a user's physical state.

In some embodiments, a NIRS monitoring wearable system, as disclosed herein, may be reuseable, wireless, and/or flexible such that it may conform to a body part of a user (e.g., sternum, arm, leg, ankle, etc.). The NIRS system may also be waterproof (e.g., according to Ingress Protection (IP) x7, with '7' meaning immersion up to about 1 meter for approximately 30 minutes), crush resistant, drop resistant, and/or low pressure over-molded, and/or be made from biocompatible materials. The NIRS system may comprise a processor configured to execute one or more multi-depth tissue analysis algorithms. The processor may be in electronic communication with a plurality of light sources and a plurality of detectors, and may be mounted on a substrate. The NIRS system may be configured to continuously process the plurality of signals using differential path length factor corrections to account for variations in optical path lengths through tissue at different wavelengths and source-detector separations. Upon processing the signals, the NIRS system may be configured to assess characteristics of optical tissue density at a plurality of tissue depths of the body part of the user, and calculate one or more biometric properties of the user.

Reference will now be made in detail to example embodiments of the disclosed technology that are illustrated in the accompanying drawings and disclosed herein. Wherever convenient, the same reference numbers will be used throughout the drawings to refer to the same or like parts.

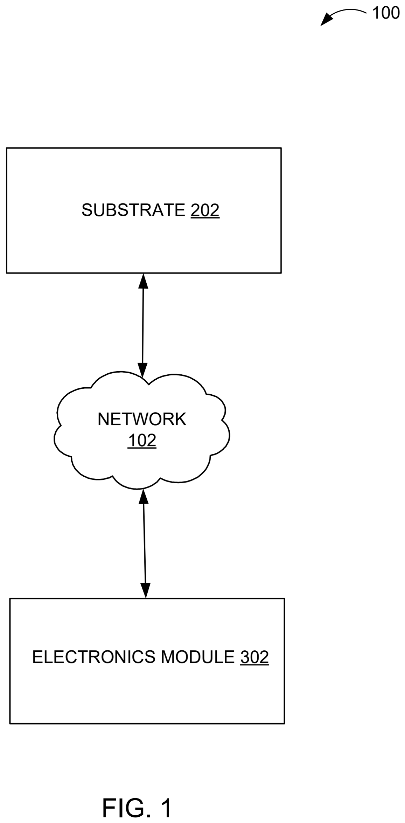

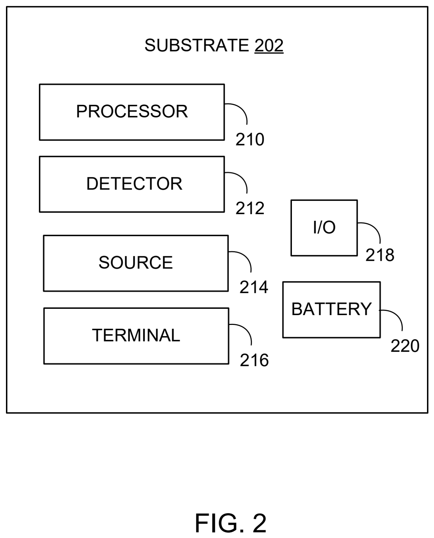

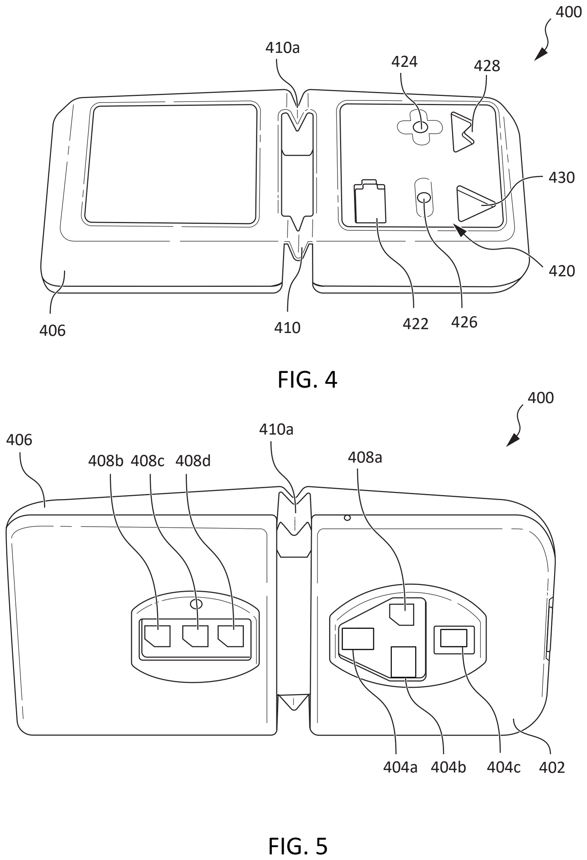

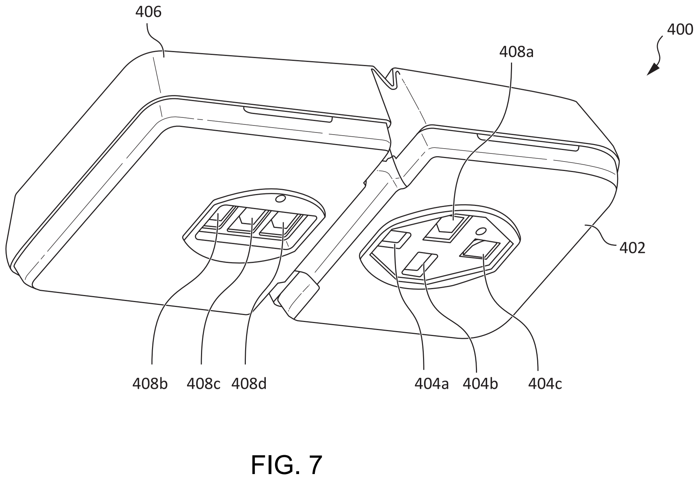

FIG. 1 depicts an embodiment of a system 100 used for monitoring oximetry parameters of a user, in accordance with the present disclosure. The system 100 may include a substrate 202 configured to communicate with an electronics module 302 over a network 402, as further discussed below with respect to FIGS. 2-3. It should be understood that while FIGS. 1-3 provide general schematics respectively associated with a system, substrate, and electronics module, it should be understood that one or more components or features of the system 100, substrate 202, and/or electronics module 302 may be modified when providing a specific form factor of a NIRS monitoring system, as shown in and described further below with respect to FIGS. 4-8. For example, in one example NIRS monitoring system, the utilized substrate may not include its own components (e.g., processor, battery) independent from the electronics module. Instead, the electronics may all be included within the electronics module that is disposed on or integrated into a substrate. As another example, a NIRS monitoring system may utilize a substrate housing at least one or more detectors and one or more sensors, and an electronics module housing at least a processor, memory, and battery. In such example, the electronics module and substrate may be communicatively and removably coupled to each other.

FIG. 2 depicts an embodiment of a substrate 202 used for monitoring oximetry parameters of a user, in accordance with the present disclosure. In some embodiments, the substrate 202 may be configured to be placed on a body part of a user (e.g., a patient). For example, substrate 202 may be coupled to (e.g., placed on top of or embedded within) a flexible material, such that substrate 202 may be placed (e.g., with an adhesive) on a body part of a user and conform to the shape of that body part (e.g., a forehead, sternum, leg, etc.). In some embodiments, the substrate 202 and/or the flexible material may also provide for electrical insulation and/or optical isolation of any NIRS and/or PPG optics, and/or any electrical connection lines to electrodes mounted on substrate 202.

In some embodiments, the substrate 202 may include one or more materials, such as silicone, nylon, epoxy, a bioinert polymer, a biocompatible polymer, a woven or nonwoven textile, an adhesive film, a flexible circuit board, flexible sensors and electronics, or a combination thereof. In some embodiments, the substrate 202 and any components mounted onto the substrate 202 may be configured to provide mechanical flexibility, allowing the substrate 202 to conform to and/or adhere to a surface. In some embodiments, the substrate 202 may be configured to be integrated into clothing or other equipment designed to be worn or applied to a user.

In some embodiments, the shape and/or dimensions of the substrate 202 may be different depending on the specific patient and/or use case, as further discussed below. Additionally, substrate 202 may also be configured of any size, for example, having any of the dimensions described herein (e.g., the NIRS system 400).

As shown in FIG. 2, the substrate 202 may include a processor 210, a sensor or detector 212, a source 214 (e.g., a light source, an electrode, an ultrasound transducer), a terminal 216, an input/output (I/O) device 218, and/or a power source 220 (e.g., a battery) configured to power the substrate 202.

Detector 212 may be configured to sense or detect signals associated with oximetry parameters of a user, such as SpO2 and StO2, as further discussed herein. Detector 212 may be configured to detect such signals at various tissue depths associated with the applicable body part, as discussed herein.

The I/O device 218 may be configured to connect the substrate 202 to one or more other components of system 100 or one or more components external to system 100, such as a computing device (e.g., a laptop or other "smart" device).

The source 214 may include a single light source. In other embodiments, the source 214 may include multiple light sources, such as 2 light sources, 3 light sources, 4 light sources, 5 light sources, and so on. In some embodiments, each light source may include one or more light emitting diodes (LEDs). In some embodiments, each light source may include a single tunable light source such as a broadband LED coupled with a miniature monochromator. In some embodiments, each light source may include one or more laser diodes. In an embodiment, the source 214 may include a light source driver capable of selecting between the different light sources or selecting a wavelength from a tunable light source. In some embodiments, the source 214 may include ultrasound or electrode sources.

In some embodiments, the source 214 may be capable of emitting a first set of wavelengths of red, green, infrared, near-infrared, and/or visible light. In some embodiments, each light source within the source 214 may be capable of independently emitting a wavelength. The first set of wavelengths may comprise 1 wavelength, 2 wavelengths, 3 wavelengths, 4 wavelengths, 5 wavelengths, 6 wavelengths, 7 wavelengths, 8 wavelengths, 9 wavelengths, 10 wavelengths, or any other number of wavelengths known in the art. In some embodiments, each wavelength within the first set of wavelengths may independently be from about 450 nm to about 950 nm. Each wavelength may be, for example, about 450 nm, about 455 nm, about 460 nm, about 465 nm, about 470 nm, about 475 nm, about 480 nm, about 485 nm, about 490 nm, about 495 nm, about 500 nm, about 505 nm, about 510 nm, about 515 nm, about 520 nm, about 525 nm, about 530 nm, about 535 nm, about 540 nm, about 545 nm, about 550 nm, about 555 nm, about 560 nm, about 565 nm, about 570 nm, about 575 nm, about 580 nm, about 585 nm, about 590 nm, about 595 nm, about 600 nm, about 605 nm, about 610 nm, about 615 nm, about 620 nm, about 625 nm, about 630 nm, about 635 nm, about 640 nm, about 645 nm, about 650 nm, about 655 nm, about 660 nm, about 665 nm, about 670 nm, about 675 nm, about 680 nm, about 685 nm, about 690 nm, about 695 nm, about 700 nm, about 705 nm, about 710 nm, about 715 nm, about 720 nm, about 725 nm, about 730 nm, about 735 nm, about 740 nm, about 745 nm, about 750 nm, about 755 nm, about 760 nm, about 765 nm, about 770 nm, about 775 nm, about 780 nm, about 785 nm, about 790 nm, about 795 nm, about 800 nm, about 805 nm, about 810 nm, about 815 nm, about 820 nm, about 825 nm, about 830 nm, about 835 nm, about 840 nm, about 845 nm, about 850 nm, about 855 nm, about 860 nm, about 865 nm, about 870 nm, about 875 nm, about 880 nm, about 885 nm, about 890 nm, about 895 nm, about 900 nm, about 905 nm, about 910 nm, about 915 nm, about 920 nm, about 925 nm, about 930 nm, about 935 nm, about 940 nm, about 945 nm, about 950 nm, or any range between any two of these values, including endpoints.

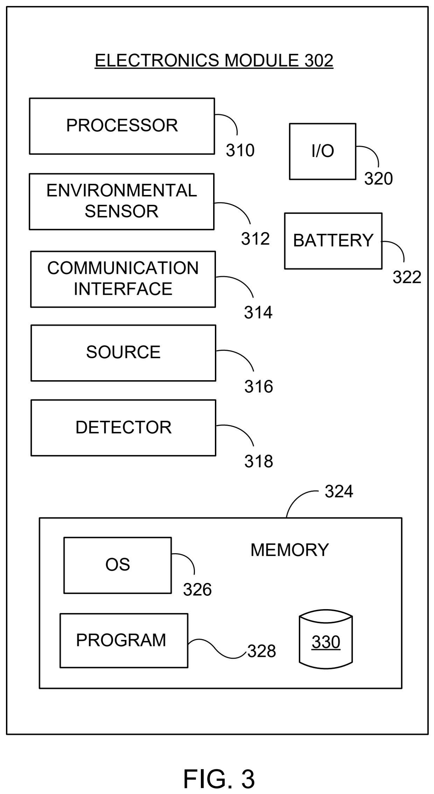

Turning to FIG. 3, system 100 may include one or more electronics modules 302. Each electronics module 302 may be configured with the same or similar components; however, in some embodiments, different electronics modules 302 may be configured to detect and process different types of parameters of a user. For example, a first electronics module 302 may be configured to detect and process optical biometric properties of a user, while a second electronics module 302 may be configured to detect and process non-optical biometric properties of a user. In some embodiments, system 100 may include a single electronics module 302 that may include the hardware and functionality of a first and second electronics module 302, and may be configured to carry out the program instructions for both electronics modules within a single, integrated package.

In some embodiments, electronics module 302 may be communicatively coupled to the substrate 202 (e.g., via network 402, as shown in FIG. 1), and may include a processor 310, a memory device 324, and instructions stored on the memory device 324 that may direct the processor 310 to perform one or more actions. The electronics module 302 may be configured to receive a signal from one or more detectors 212 mounted on the substrate 202, and to process the received signal based on one or more predefined algorithms.

As shown in FIG. 3, the electronics module 302 may further include one or more environmental sensors 312, a communication interface 314, a light source 316, a detector 318, an I/O device 320, and/or an energy storage device 322 (e.g., a battery) configured to power the electronics module 302. Memory device 324 may include an operating system (OS) 326 and program 328, and a database 330. Operating system 326 may be a real-time operating system (RTOS) or program instructions in system firmware operating on the processor 310. Program 328 may include stored instructions that direct the processor 310 to perform one or more steps toward processing signals received from substrate 202, as discussed herein.

In some embodiments, the communication or connection interface 314 (which may also or instead be included on substrate 202) can facilitate connections that can be, for example, a wireless connection, a wired connection, a Bluetooth connection, a near-field communication (NFC) connection, a radio frequency identification (RFID) connection, or a combination thereof. In some embodiments, data processing and real-time feedback may occur within the components onboard the substrate 202, or offboard through communication with an external computing device, as discussed below. The external computing device may comprise, for example, a smartphone, a charging or communications base station, a display screen, a tablet, a computer, a mobile or web-based application, or another device.

FIGS. 4-8 provide different views of an embodiment of a NIRS wearable monitoring system 400 used for detecting oximetry parameters of a user, and specifically a tissue of a body part of the user. It should be understood that while FIGS. 4-8 provide an embodiment of a form factor including one or more of the components and features discussed above with respect to FIGS. 1-3, other form factors are contemplated to perform the functions disclosed herein.

In some embodiments, the NIRS system 400 may be configured to continuously process a plurality of signals using differential path length factor corrections, and, responsive to continuously processing the plurality of signals, assess characteristics of optical tissue density at a plurality of tissue depths of the body part. The multi-depth tissue analysis algorithms (further discussed herein) executed by the processor may analyze signals from detectors positioned at different distances from the light sources to determine oxygenation levels at corresponding tissue depths. The NIRS system 400 may further be configured to calculate one or more biometric properties of the user based on the assessed characteristics in optical tissue density, including StO2, SpO2, pulse rate (PR), heart rate, respiratory rate (RR), perfusion, blood pressure, blood volume, total hemoglobin, and/or macrovasular and/or microvascular hemodynamic coherence, and/or trends of any of the foregoing properties.

In some embodiments, the NIRS system 400 may be configured to generate real-time physiological measurements, provide predictive analytics (e.g., diagnostic trends), and/or provide continuous monitoring output (e.g., with automated alert generation based on physiological thresholds) based on the monitored biometric properties and trends.

In some embodiments, the NIRS system 400 may be wearable, portable, reusable, wireless, flexible, waterproof, crush resistant, drop resistant, and/or low pressure over-molded. The NIRS system 400 may also be made from biocompatible materials. The NIRS system 400 may have an Ingress Protection (IP) rating of at least IP67, and may have one or more hermetically sealed optical components that can be redesigned in many configurations, for example, as a modular design.

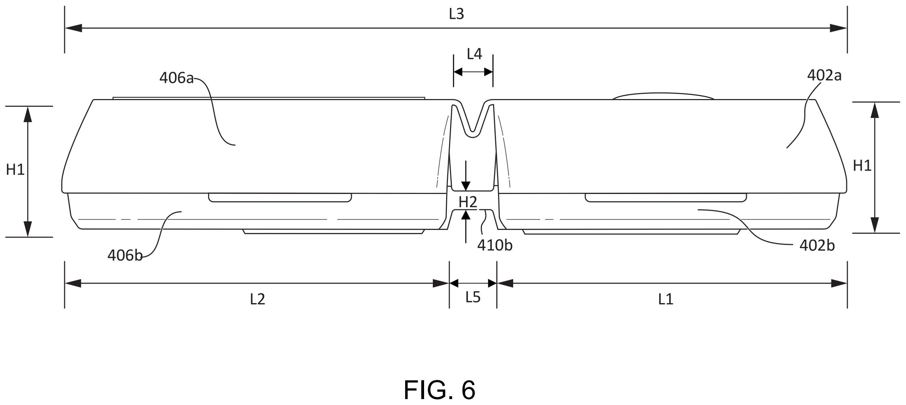

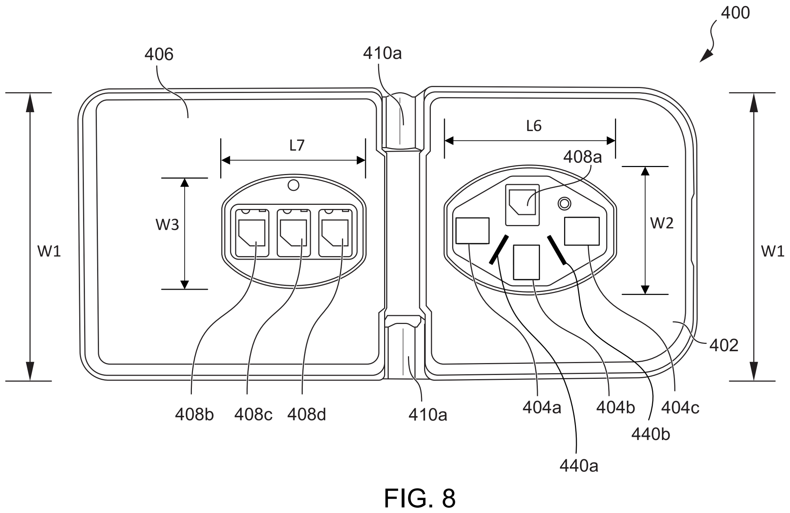

In some embodiments, as particularly shown in FIGS. 6 and 8, the NIRS system 400 may have an overall length L3 (FIG. 6) of about 50 millimeters (mm) to about 108 mm (e.g., about 50.0 mm, about 50.2 mm, about 50.4 mm, about 50.6 mm, about 50.8 mm, about 51.0 mm, about 51.2 mm, about 51.4 mm, about 51.6 mm, about 51.8 mm, about 52.0 mm, about 52.2 mm, about 52.4 mm, about 52.6 mm, about 52.8 mm, about 53.0 mm, about 53.2 mm, about 53.4 mm, about 53.6 mm, about 53.8 mm, about 54.0 mm, about 54.2 mm, about 54.4 mm, about 54.6 mm, about 54.8 mm, about 55.0 mm, about 55.2 mm, about 55.4 mm, about 55.6 mm, about 55.8 mm, about 56.0 mm, about 56.2 mm, about 56.4 mm, about 56.6 mm, about 56.8 mm, about 57.0 mm, about 57.2 mm, about 57.4 mm, about 57.6 mm, about 57.8 mm, about 58.0 mm, about 58.2 mm, about 58.4 mm, about 58.6 mm, about 58.8 mm, about 59.0 mm, about 59.2 mm, about 59.4 mm, about 59.6 mm, about 59.8 mm, about 60.0 mm, about 60.2 mm, about 60.4 mm, about 60.6 mm, about 60.8 mm, about 61.0 mm, about 61.2 mm, about 61.4 mm, about 61.6 mm, about 61.8 mm, about 62.0 mm, about 62.2 mm, about 62.4 mm, about 62.6 mm, about 62.8 mm, about 63.0 mm, about 63.2 mm, about 63.4 mm, about 63.6 mm, about 63.8 mm, about 64.0 mm, about 64.2 mm, about 64.4 mm, about 64.6 mm, about 64.8 mm, about 65.0 mm, about 65.2 mm, about 65.4 mm, about 65.6 mm, about 65.8 mm, about 66.0 mm, about 66.2 mm, about 66.4 mm, about 66.6 mm, about 66.8 mm, about 67.0 mm, about 67.2 mm, about 67.4 mm, about 67.6 mm, about 67.8 mm, about 68.0 mm, about 68.2 mm, about 68.4 mm, about 68.6 mm, about 68.8 mm, about 69.0 mm, about 69.2 mm, about 69.4 mm, about 69.6 mm, about 69.8 mm, about 70.0 mm, about 70.2 mm, about 70.4 mm, about 70.6 mm, about 70.8 mm, about 71.0 mm, about 71.2 mm, about 71.4 mm, about 71.6 mm, about 71.8 mm, about 72.0 mm, about 72.2 mm, about 72.4 mm, about 72.6 mm, about 72.8 mm, about 73.0 mm, about 73.2 mm, about 73.4 mm, about 73.6 mm, about 73.8 mm, about 74.0 mm, about 74.2 mm, about 74.4 mm, about 74.6 mm, about 74.8 mm, about 75.0 mm, about 75.2 mm, about 75.4 mm, about 75.6 mm, about 75.8 mm, about 76.0 mm, about 76.2 mm, about 76.4 mm, about 76.6 mm, about 76.8 mm, about 77.0 mm, about 77.2 mm, about 77.4 mm, about 77.6 mm, about 77.8 mm, about 78.0 mm, about 78.2 mm, about 78.4 mm, about 78.6 mm, about 78.8 mm, about 79.0 mm, about 79.2 mm, about 79.4 mm, about 79.6 mm, about 79.8 mm, about 80.0 mm, about 80.2 mm, about 80.4 mm, about 80.6 mm, about 80.8 mm, about 81.0 mm, about 81.2 mm, about 81.4 mm, about 81.6 mm, about 81.8 mm, about 82.0 mm, about 82.2 mm, about 82.4 mm, about 82.6 mm, about 82.8 mm, about 83.0 mm, about 83.2 mm, about 83.4 mm, about 83.6 mm, about 83.8 mm, about 84.0 mm, about 84.2 mm, about 84.4 mm, about 84.6 mm, about 84.8 mm, about 85.0 mm, about 85.2 mm, about 85.4 mm, about 85.6 mm, about 85.8 mm, about 86.0 mm, about 86.2 mm, about 86.4 mm, about 86.6 mm, about 86.8 mm, about 87.0 mm, about 87.2 mm, about 87.4 mm, about 87.6 mm, about 87.8 mm, about 88.0 mm, about 88.2 mm, about 88.4 mm, about 88.6 mm, about 88.8 mm, about 89.0 mm, about 89.2 mm, about 89.4 mm, about 89.6 mm, about 89.8 mm, about 90.0 mm, about 90.2 mm, about 90.4 mm, about 90.6 mm, about 90.8 mm, about 91.0 mm, about 91.2 mm, about 91.4 mm, about 91.6 mm, about 91.8 mm, about 92.0 mm, about 92.2 mm, about 92.4 mm, about 92.6 mm, about 92.8 mm, about 93.0 mm, about 93.2 mm, about 93.4 mm, about 93.6 mm, about 93.8 mm, about 94.0 mm, about 94.2 mm, about 94.4 mm, about 94.6 mm, about 94.8 mm, about 95.0 mm, about 95.2 mm, about 95.4 mm, about 95.6 mm, about 95.8 mm, about 96.0 mm, about 96.2 mm, about 96.4 mm, about 96.6 mm, about 96.8 mm, about 97.0 mm, about 97.2 mm, about 97.4 mm, about 97.6 mm, about 97.8 mm, about 98.0 mm, about 98.2 mm, about 98.4 mm, about 98.6 mm, about 98.8 mm, about 99.0 mm, about 99.2 mm, about 99.4 mm, about 99.6 mm, about 99.8 mm, about 100.0 mm, about 100.2 mm, about 100.4 mm, about 100.6 mm, about 100.8 mm, about 101.0 mm, about 101.2 mm, about 101.4 mm, about 101.6 mm, about 101.8 mm, about 102.0 mm, about 102.2 mm, about 102.4 mm, about 102.6 mm, about 102.8 mm, about 103.0 mm, about 103.2 mm, about 103.4 mm, about 103.6 mm, about 103.8 mm, about 104.0 mm, about 104.2 mm, about 104.4 mm, about 104.6 mm, about 104.8 mm, about 105.0 mm, about 105.2 mm, about 105.4 mm, about 105.6 mm, about 105.8 mm, about 106.0 mm, about 106.2 mm, about 106.4 mm, about 106.6 mm, about 106.8 mm, about 107.0 mm, about 107.2 mm, about 107.4 mm, about 107.6 mm, about 107.8 mm, about 108.0 mm).

The NIRS system 400 may have an overall height H1 (FIG. 6) of about 8 mm to about 16 mm (e.g., about 8.0 mm, about 8.2 mm, about 8.4 mm, about 8.6 mm, about 8.8 mm, about 9.0 mm, about 9.2 mm, about 9.4 mm, about 9.6 mm, about 9.8 mm, about 10.0 mm, about 10.2 mm, about 10.4 mm, about 10.6 mm, about 10.8 mm, about 11.0 mm, about 11.2 mm, about 11.4 mm, about 11.6 mm, about 11.8 mm, about 12.0 mm, about 12.2 mm, about 12.4 mm, about 12.6 mm, about 12.8 mm, about 13.0 mm, about 13.2 mm, about 13.4 mm, about 13.6 mm, about 13.8 mm, about 14.0 mm, about 14.2 mm, about 14.4 mm, about 14.6 mm, about 14.8 mm, about 15.0 mm, about 15.2 mm, about 15.4 mm, about 15.6 mm, about 15.8 mm, about 16.0 mm).

The NIRS system 400 may have an overall width W1 (FIG. 8) of about 20 mm to about 50 mm (e.g., about 20.0 mm, about 20.2 mm, about 20.4 mm, about 20.6 mm, about 20.8 mm, about 21.0 mm, about 21.2 mm, about 21.4 mm, about 21.6 mm, about 21.8 mm, about 22.0 mm, about 22.2 mm, about 22.4 mm, about 22.6 mm, about 22.8 mm, about 23.0 mm, about 23.2 mm, about 23.4 mm, about 23.6 mm, about 23.8 mm, about 24.0 mm, about 24.2 mm, about 24.4 mm, about 24.6 mm, about 24.8 mm, about 25.0 mm, about 25.2 mm, about 25.4 mm, about 25.6 mm, about 25.8 mm, about 26.0 mm, about 26.2 mm, about 26.4 mm, about 26.6 mm, about 26.8 mm, about 27.0 mm, about 27.2 mm, about 27.4 mm, about 27.6 mm, about 27.8 mm, about 28.0 mm, about 28.2 mm, about 28.4 mm, about 28.6 mm, about 28.8 mm, about 29.0 mm, about 29.2 mm, about 29.4 mm, about 29.6 mm, about 29.8 mm, about 30.0 mm, about 30.2 mm, about 30.4 mm, about 30.6 mm, about 30.8 mm, about 31.0 mm, about 31.2 mm, about 31.4 mm, about 31.6 mm, about 31.8 mm, about 32.0 mm, about 32.2 mm, about 32.4 mm, about 32.6 mm, about 32.8 mm, about 33.0 mm, about 33.2 mm, about 33.4 mm, about 33.6 mm, about 33.8 mm, about 34.0 mm, about 34.2 mm, about 34.4 mm, about 34.6 mm, about 34.8 mm, about 35.0 mm, about 35.2 mm, about 35.4 mm, about 35.6 mm, about 35.8 mm, about 36.0 mm, about 36.2 mm, about 36.4 mm, about 36.6 mm, about 36.8 mm, about 37.0 mm, about 37.2 mm, about 37.4 mm, about 37.6 mm, about 37.8 mm, about 38.0 mm, about 38.2 mm, about 38.4 mm, about 38.6 mm, about 38.8 mm, about 39.0 mm, about 39.2 mm, about 39.4 mm, about 39.6 mm, about 39.8 mm, about 40.0 mm, about 40.2 mm, about 40.4 mm, about 40.6 mm, about 40.8 mm, about 41.0 mm, about 41.2 mm, about 41.4 mm, about 41.6 mm, about 41.8 mm, about 42.0 mm, about 42.2 mm, about 42.4 mm, about 42.6 mm, about 42.8 mm, about 43.0 mm, about 43.2 mm, about 43.4 mm, about 43.6 mm, about 43.8 mm, about 44.0 mm, about 44.2 mm, about 44.4 mm, about 44.6 mm, about 44.8 mm, about 45.0 mm, about 45.2 mm, about 45.4 mm, about 45.6 mm, about 45.8 mm, about 46.0 mm, about 46.2 mm, about 46.4 mm, about 46.6 mm, about 46.8 mm, about 47.0 mm, about 47.2 mm, about 47.4 mm, about 47.6 mm, about 47.8 mm, about 48.0 mm, about 48.2 mm, about 48.4 mm, about 48.6 mm, about 48.8 mm, about 49.0 mm, about 49.2 mm, about 49.4 mm, about 49.6 mm, about 49.8 mm, about 50.0 mm).

The disclosed NIRS system 400 provides important, critical, and unexpected results in terms of its data storage and processing capabilities, as well as its achievable lifespan, given the above-described overall dimensions. That is, the NIRS system 400 provides unexpected storage and processing capacity, as well as lifespan (e.g., about 70 to 85 hours) compared to conventional NIRS monitoring systems given the small compact size of the NIRS system 400.

As shown in FIGS. 4-8, the NIRS system 400 may include a first component 402 (e.g., made of a rigid, semi rigid, or flexible material) that may house a plurality of light sources 404 (e.g., 404a, 404b, 404c) configured to emit visible, infrared, near-infrared, green, and/or red light at a plurality of wavelengths, for example, including at least three distinct wavelengths, at least four distinct wavelengths, or at least five distinct wavelengths. The plurality of light sources may be configured as a plurality of light emitting diodes (LEDs) configured to emit light at multiple wavelengths including visible and infrared wavelengths. In some embodiments, the plurality of wavelengths may include a first wavelength at approximately 735 nm (e.g., approximately 705 nm, 710 nm, 715 nm, 720 nm, 725 nm, 730 nm, 735 nm, 740 nm, 745 nm, 750 nm, 755 nm, 760 nm, 765 nm), a second wavelength at approximately 850 nm (e.g., 830 nm, 835 nm, 840 nm, 845 nm, 850 nm, 855 nm, 860 nm, 865 nm), a third wavelength at approximately 535 nm (e.g., 505 nm, 510 nm, 515 nm, 520 nm, 525 nm, 530 nm, 535 nm, 540 nm, 545 nm, 550 nm), a fourth wavelength at approximately 660 nm (e.g., 635 nm, 640 nm, 645 nm, 650 nm, 655 nm, 660 nm, 665 nm, 670 nm, 675 nm, 680 nm, 685 nm), and a fifth wavelength at approximately 940 nm (e.g., 900 nm, 905 nm, 910 nm, 915 nm, 920 nm, 925 nm, 930 nm, 935 nm, 940 nm, 945 nm, 950 nm, 955 nm, 960 nm). These wavelengths may be selected based on the absorption characteristics of oxygenated hemoglobin and deoxygenated hemoglobin in the visible and infrared spectral regions. In some embodiments, each of the light sources of the plurality of light sources 404 may be configured with distinct optics to emit one or more wavelengths associated with particular tissue parameters. For example, light sources 404a and 404b may be configured to emit wavelength(s) associated with a PPG signal (e.g., $SpO_2$), while light source 404c may be configured to emit wavelength(s) associated with a NIRS signal.

As particularly shown in FIG. 6, the first component 402 may include a top portion 402a and a bottom portion 402b that may be integrated together to form the first component 402 and to house at least some of the electronics of the NIRS system 400, as discussed herein.

In some embodiments, the first component 402 may have a length L1 of about 20 mm to about 50 mm (e.g., about 20.0 mm, about 20.2 mm, about 20.4 mm, about 20.6 mm, about 20.8 mm, about 21.0 mm, about 21.2 mm, about 21.4 mm, about 21.6 mm, about 21.8 mm, about 22.0 mm, about 22.2 mm, about 22.4 mm, about 22.6 mm, about 22.8 mm, about 23.0 mm, about 23.2 mm, about 23.4 mm, about 23.6 mm, about 23.8 mm, about 24.0 mm, about 24.2 mm, about 24.4 mm, about 24.6 mm, about 24.8 mm, about 25.0 mm, about 25.2 mm, about 25.4 mm, about 25.6 mm, about 25.8 mm, about 26.0 mm, about 26.2 mm, about 26.4 mm, about 26.6 mm, about 26.8 mm, about 27.0 mm, about 27.2 mm, about 27.4 mm, about 27.6 mm, about 27.8 mm, about 28.0 mm, about 28.2 mm, about 28.4 mm, about 28.6 mm, about 28.8 mm, about 29.0 mm, about 29.2 mm, about 29.4 mm, about 29.6 mm, about 29.8 mm, about 30.0 mm, about 30.2 mm, about 30.4 mm, about 30.6 mm, about 30.8 mm, about 31.0 mm, about 31.2 mm, about 31.4 mm, about 31.6 mm, about 31.8 mm, about 32.0 mm, about 32.2 mm, about 32.4 mm, about 32.6 mm, about 32.8 mm, about 33.0 mm, about 33.2 mm, about 33.4 mm, about 33.6 mm, about 33.7 mm, about 33.8 mm, about 34.0 mm, about 34.2 mm, about 34.4 mm, about 34.6 mm, about 34.8 mm, about 35.0 mm, about 35.2 mm, about 35.4 mm, about 35.6 mm, about 35.8 mm, about 36.0 mm, about 36.2 mm, about 36.4 mm, about 36.6 mm, about 36.8 mm, about 37.0 mm, about 37.2 mm, about 37.4 mm, about 37.6 mm, about 37.8 mm, about 38.0 mm, about 38.2 mm, about 38.4 mm, about 38.6 mm, about 38.8 mm, about 39.0 mm, about 39.2 mm, about 39.4 mm, about 39.6 mm, about 39.8 mm, about 40.0 mm, about 40.2 mm, about 40.4 mm, about 40.6 mm, about 40.8 mm, about 41.0 mm, about 41.2 mm, about 41.4 mm, about 41.6 mm, about 41.8 mm, about 42.0 mm, about 42.2 mm, about 42.4 mm, about 42.6 mm, about 42.8 mm, about 43.0 mm, about 43.2 mm, about 43.4 mm, about 43.6 mm, about 43.8 mm, about 44.0 mm, about 44.2 mm, about 44.4 mm, about 44.6 mm, about 44.8 mm, about 45.0 mm, about 45.2 mm, about 45.4 mm, about 45.6 mm, about 45.8 mm, about 46.0 mm, about 46.2 mm, about 46.4 mm, about 46.6 mm, about 46.8 mm, about 47.0 mm, about 47.2 mm, about 47.4 mm, about 47.6 mm, about 47.8 mm, about 48.0 mm, about 48.2 mm, about 48.4 mm, about 48.6 mm, about 48.8 mm, about 49.0 mm, about 49.2 mm, about 49.4 mm, about 49.6 mm, about 49.8 mm, about 50.0 mm).

As particularly shown in FIGS. 5, 7, and 8, in some embodiments, in addition to housing the plurality of light sources, the first component 402 may also house a first detector 408a configured to detect a first plurality of signals from the plurality of light sources. The first detector may be disposed at a source-detector distance of approximately 6 to 10 mm (e.g., 6 mm, 6.5 mm, 7.0 mm, 7.5 mm, 8.0 mm, 8.5 mm, 9.0 mm, 9.5 mm, 10.0 mm). While the first detector 408a is shown integrated into the first component 402, in some embodiments, the first detector 408a may instead be integrated into the second component 406, as further discussed below.

The first component 402 may comprise integrated optical isolation barriers 440 (e.g., 440a, 440b, FIG. 8) positioned between adjacent light sources to minimize optical crosstalk between the plurality of light sources. The integrated optical isolation barriers may prevent light emitted from one LED from directly reaching adjacent LEDs or from creating interference patterns that could degrade measurement accuracy. In some cases, the optical isolation barriers may be formed as part of the component structure or may be incorporated as separate barrier elements positioned between the light sources.

In some embodiments, the NIRS system 400 (e.g., on the first component 402) may have glass disposed on one or more surfaces of the plurality of light sources 404 to reduce optical crosstalk between the plurality of light sources. The glass may have one or more anti-reflective coatings with wavelength-specific filtering. Optical crosstalk may occur when light emitted from one light source reaches adjacent light sources or detectors through unintended optical paths, such as reflections from the sensor housing, the skin surface, or internal optical components. The glass disposed on the surfaces of the light sources may serve as an optical barrier that blocks or attenuates such stray light while allowing the intended measurement light to pass through to the tissue being measured.

The anti-reflective coatings on the glass may reduce surface reflections that could contribute to optical crosstalk or signal degradation. Anti-reflective coatings may comprise thin film layers deposited on the glass surfaces that are designed to minimize reflections at the measurement wavelengths used by the NIRS system. The wavelength-specific filtering properties of the glass may allow selective transmission of light at the measurement wavelengths while blocking or attenuating light at other wavelengths that could interfere with the measurements. In some cases, the wavelength-specific filtering may be achieved through the use of optical filter coatings, doped glass materials, or other filtering mechanisms integrated into the glass structure.

The glass may comprise a silkscreen with precision-etched optical barriers and wavelength-selective transmission properties. The silkscreen may be applied to the glass surface using screen printing or similar deposition techniques to create patterned optical barriers that define the optical apertures for each light source. The precision-etched optical barriers may be formed through photolithographic etching, laser etching, or other precision manufacturing processes that create well-defined barrier structures with controlled dimensions and optical properties. The wavelength-selective transmission properties of the silkscreen may allow the glass to transmit light at the measurement wavelengths while blocking ambient light or stray light at other wavelengths.

The glass may be about 90 percent opaque with controlled light transmission characteristics for desired signal-to-noise ratio. The 90 percent opacity may refer to the overall light blocking capability of the glass in regions outside the optical apertures, where the silkscreen or other opaque materials prevent light transmission. The controlled light transmission characteristics may allow precise control over the amount and spectral distribution of light that passes through the optical apertures to illuminate the tissue. The signal-to-noise ratio of the NIRS measurements may be improved by reducing stray light that could reach the detectors through unintended optical paths while maintaining adequate illumination intensity at the measurement wavelengths.

The combination of glass with anti-reflective coatings, silkscreen with precision-etched optical barriers, optical isolation structures with wavelength-selective barriers, and opaque barriers with micro-structured geometries may provide multiple layers of optical crosstalk reduction in the NIRS monitoring wearable system. Each of these optical isolation mechanisms may address different sources of optical crosstalk, including direct light leakage between adjacent light sources, reflections from internal surfaces, and ambient light interference. The multi-layered approach to optical isolation may allow the NIRS monitoring wearable system to achieve high signal-to-noise ratios and accurate tissue oxygenation measurements across a range of operating conditions and tissue types.

The plurality of LEDs may be arranged in a spatially distributed pattern with integrated thermal management and wavelength-specific power control. The spatially distributed pattern may comprise a hexagonal array pattern or other geometric arrangement that provides uniform illumination of the tissue volume being measured. In some cases, the plurality of LEDs may comprise at least six LEDs (e.g., at least seven LEDs, or at least eight LEDs) configured to emit light at wavelengths optimized for hemoglobin absorption characteristics and arranged in a hexagonal array pattern with individual current control for each LED. The individual current control may allow independent adjustment of the optical output power of each LED to compensate for variations in LED efficiency, tissue optical properties, and/or measurement requirements.

As particularly shown in FIG. 8, the spatially distributed pattern may have an overall length L6 of about 12 mm to about 30 mm (e.g., about 12.0 mm, about 12.2 mm, about 12.4 mm, about 12.6 mm, about 12.8 mm, about 13.0 mm, about 13.2 mm, about 13.4 mm, about 13.6 mm, about 13.8 mm, about 14.0 mm, about 14.2 mm, about 14.4 mm, about 14.6 mm, about 14.8 mm, about 15.0 mm, about 15.2 mm, about 15.4 mm, about 15.6 mm, about 15.8 mm, about 16.0 mm, about 16.2 mm, about 16.4 mm, about 16.6 mm, about 16.8 mm, about 17.0 mm, about 17.2 mm, about 17.4 mm, about 17.6 mm, about 17.8 mm, about 18.0 mm, about 18.2 mm, about 18.4 mm, about 18.6 mm, about 18.8 mm, about 19.0 mm, about 19.2 mm, about 19.4 mm, about 19.6 mm, about 19.8 mm, about 20.0 mm, about 20.2 mm, about 20.4 mm, about 20.6 mm, about 20.8 mm, about 21.0 mm, about 21.2 mm, about 21.4 mm, about 21.6 mm, about 21.8 mm, about 22.0 mm, about 22.2 mm, about 22.4 mm, about 22.6 mm, about 22.8 mm, about 23.0 mm, about 23.2 mm, about 23.4 mm, about 23.6 mm, about 23.8 mm, about 24.0 mm, about 24.2 mm, about 24.4 mm, about 24.6 mm, about 24.8 mm, about 25.0 mm, about 25.2 mm, about 25.4 mm, about 25.6 mm, about 25.8 mm, about 26.0 mm, about 26.2 mm, about 26.4 mm, about 26.6 mm, about 26.8 mm, about 27.0 mm, about 27.2 mm, about 27.4 mm, about 27.6 mm, about 27.8 mm, about 28.0 mm, about 28.2 mm, about 28.4 mm, about 28.6 mm, about 28.8 mm, about 29.0 mm, about 29.2 mm, about 29.4 mm, about 29.6 mm, about 29.8 mm, about 30.0 mm), and an overall width W2 of about 8 mm to about 24 mm (e.g., about 8.0 mm, about 8.2 mm, about 8.4 mm, about 8.6 mm, about 8.8 mm, about 9.0 mm, about 9.2 mm, about 9.4 mm, about 9.6 mm, about 9.8 mm, about 10.0 mm, about 10.2 mm, about 10.4 mm, about 10.6 mm, about 10.8 mm, about 11.0 mm, about 11.2 mm, about 11.4 mm, about 11.6 mm, about 11.8 mm, about 12.0 mm, about 12.2 mm, about 12.4 mm, about 12.6 mm, about 12.8 mm, about 13.0 mm, about 13.2 mm, about 13.4 mm, about 13.6 mm, about 13.8 mm, about 14.0 mm, about 14.2 mm, about 14.4 mm, about 14.6 mm, about 14.8 mm, about 15.0 mm, about 15.2 mm, about 15.4 mm, about 15.6 mm, about 15.8 mm, about 16.0 mm, about 16.2 mm, about 16.4 mm, about 16.6 mm, about 16.8 mm, about 17.0 mm, about 17.2 mm, about 17.4 mm, about 17.6 mm, about 17.8 mm, about 18.0 mm, about 18.2 mm, about 18.4 mm, about 18.6 mm, about 18.8 mm, about 19.0 mm, about 19.2 mm, about 19.4 mm, about 19.6 mm, about 19.8 mm, about 20.0 mm, about 20.2 mm, about 20.4 mm, about 20.6 mm, about 20.8 mm, about 21.0 mm, about 21.2 mm, about 21.4 mm, about 21.6 mm, about 21.8 mm, about 22.0 mm, about 22.2 mm, about 22.4 mm, about 22.6 mm, about 22.8 mm, about 23.0 mm, about 23.2 mm, about 23.4 mm, about 23.6 mm, about 23.8 mm, about 24.0 mm).

The integrated thermal management of the plurality of LEDs may comprise heat dissipation structures, thermal interface materials, or active cooling elements configured to maintain the LEDs within specified operating temperature ranges. Thermal management may help maintain wavelength stability of the emitted light, as LED emission wavelengths may shift with temperature changes. The wavelength-specific power control may allow the NIRS system to adjust the optical power at each wavelength independently to adjust signal-to-noise ratio and measurement accuracy across different tissue types and skin tones.

The NIRS monitoring wearable system 400 may be configured to automatically adapt optical system parameters for various skin tones and densities. The automatic adaptation may involve adjusting LED drive currents, detector gain settings, or signal processing parameters based on detected optical characteristics of the tissue being measured. In some cases, the system may perform an initial calibration sequence upon placement on the user's body to determine appropriate optical parameters for the specific tissue site and skin characteristics.

The NIRS monitoring wearable system 400 may use a self-referencing measurement scheme via a symmetrical arrangement of light-emitting sources and detectors to simultaneously measure two tissue sites. The symmetrical arrangement may comprise light sources and detectors positioned such that optical measurements can be made in two directions from a central reference point, allowing differential measurements that may reduce sensitivity to motion artifacts and common-mode noise sources. The self-referencing measurement scheme may improve measurement accuracy by providing internal reference signals that can be used to compensate for variations in optical coupling between the sensor and the tissue surface.

The NIRS system 400 may include a second component 406 (e.g., made of a rigid, semi rigid, or flexible material) that may house a plurality of detectors 408 (e.g., 408b, 408c, 408d) configured to detect a second plurality of signals from the plurality of light sources 404 at multiple source-detector distances ranging from about 25 mm to about 45 mm (e.g., from about 25.0 mm, 25.2 mm, 25.4 mm, 25.6 mm, 25.8 mm, 26.0 mm, 26.2 mm, 26.4 mm, 26.6 mm, 26.8 mm, 27.0 mm, 27.2 mm, 27.4 mm, 27.6 mm, 27.8 mm, 28.0 mm, 28.2 mm, 28.4 mm, 28.6 mm, 28.8 mm, 29.0 mm, 29.2 mm, 29.4 mm, 29.6 mm, 29.8 mm, 30.0 mm, 30.2 mm, 30.4 mm, 30.6 mm, 30.8 mm, 31.0 mm, 31.2 mm, 31.4 mm, 31.6 mm, 31.8 mm, 32.0 mm, 32.2 mm, 32.4 mm, 32.6 mm, 32.8 mm, 33.0 mm, 33.2 mm, 33.4 mm, 33.6 mm, 33.8 mm, 34.0 mm, 34.2 mm, 34.4 mm, 34.6 mm, 34.8 mm, 35.0 mm, 35.2 mm, 35.4 mm, 35.6 mm, 35.8 mm, 36.0 mm, 36.2 mm, 36.4 mm, 36.6 mm, 36.8 mm, 37.0 mm, 37.2 mm, 37.4 mm, 37.6 mm, 37.8 mm, 38.0 mm, 38.2 mm, 38.4 mm, 38.6 mm, 38.8 mm, 39.0 mm, 39.2 mm, 39.4 mm, 39.6 mm, 39.8 mm, 40.0 mm, 40.2 mm, 40.4 mm, 40.6 mm, 40.8 mm, 41.0 mm, 41.2 mm, 41.4 mm, 41.6 mm, 41.8 mm, 42.0 mm, 42.2 mm, 42.4 mm, 42.6 mm, 42.8 mm, 43.0 mm, 43.2 mm, 43.4 mm, 43.6 mm, 43.8 mm, 44.0 mm, 44.2 mm, 44.4 mm, 44.6 mm, 44.8 mm, 45.0 mm). In some embodiments, the second component 406 may also house the first detector 408a. In such embodiments, the plurality of detectors 408 may detect a plurality of signals from the plurality of light sources at multiple source-detector distances ranging from about 5 mm to about 45 mm (e.g., from about 5.0 mm, 5.2 mm, 5.4 mm, 5.6 mm, 5.8 mm, 6.0 mm, 6.2 mm, 6.4 mm, 6.6 mm, 6.8 mm, 7.0 mm, 7.2 mm, 7.4 mm, 7.6 mm, 7.8 mm, 8.0 mm, 8.2 mm, 8.4 mm, 8.6 mm, 8.8 mm, 9.0 mm, 9.2 mm, 9.4 mm, 9.6 mm, 9.8 mm, 10.0 mm, 10.2 mm, 10.4 mm, 10.6 mm, 10.8 mm, 11.0 mm, 11.2 mm, 11.4 mm, 11.6 mm, 11.8 mm, 12.0 mm, 12.2 mm, 12.4 mm, 12.6 mm, 12.8 mm, 13.0 mm, 13.2 mm, 13.4 mm, 13.6 mm, 13.8 mm, 14.0 mm, 14.2 mm, 14.4 mm, 14.6 mm, 14.8 mm, 15.0 mm, 15.2 mm, 15.4 mm, 15.6 mm, 15.8 mm, 16.0 mm, 16.2 mm, 16.4 mm, 16.6 mm, 16.8 mm, 17.0 mm, 17.2 mm, 17.4 mm, 17.6 mm, 17.8 mm, 18.0 mm, 18.2 mm, 18.4 mm, 18.6 mm, 18.8 mm, 19.0 mm, 19.2 mm, 19.4 mm, 19.6 mm, 19.8 mm, 20.0 mm, 20.2 mm, 20.4 mm, 20.6 mm, 20.8 mm, 21.0 mm, 21.2 mm, 21.4 mm, 21.6 mm, 21.8 mm, 22.0 mm, 22.2 mm, 22.4 mm, 22.6 mm, 22.8 mm, 23.0 mm, 23.2 mm, 23.4 mm, 23.6 mm, 23.8 mm, 24.0 mm, 24.2 mm, 24.4 mm, 24.6 mm, 24.8 mm, 25.0 mm, 25.2 mm, 25.4 mm, 25.6 mm, 25.8 mm, 26.0 mm, 26.2 mm, 26.4 mm, 26.6 mm, 26.8 mm, 27.0 mm, 27.2 mm, 27.4 mm, 27.6 mm, 27.8 mm, 28.0 mm, 28.2 mm, 28.4 mm, 28.6 mm, 28.8 mm, 29.0 mm, 29.2 mm, 29.4 mm, 29.6 mm, 29.8 mm, 30.0 mm, 30.2 mm, 30.4 mm, 30.6 mm, 30.8 mm, 31.0 mm, 31.2 mm, 31.4 mm, 31.6 mm, 31.8 mm, 32.0 mm, 32.2 mm, 32.4 mm, 32.6 mm, 32.8 mm, 33.0 mm, 33.2 mm, 33.4 mm, 33.6 mm, 33.8 mm, 34.0 mm, 34.2 mm, 34.4 mm, 34.6 mm, 34.8 mm, 35.0 mm, 35.2 mm, 35.4 mm, 35.6 mm, 35.8 mm, 36.0 mm, 36.2 mm, 36.4 mm, 36.6 mm, 36.8 mm, 37.0 mm, 37.2 mm, 37.4 mm, 37.6 mm, 37.8 mm, 38.0 mm, 38.2 mm, 38.4 mm, 38.6 mm, 38.8 mm, 39.0 mm, 39.2 mm, 39.4 mm, 39.6 mm, 39.8 mm, 40.0 mm, 40.2 mm, 40.4 mm, 40.6 mm, 40.8 mm, 41.0 mm, 41.2 mm, 41.4 mm, 41.6 mm, 41.8 mm, 42.0 mm, 42.2 mm, 42.4 mm, 42.6 mm, 42.8 mm, 43.0 mm, 43.2 mm, 43.4 mm, 43.6 mm, 43.8 mm, 44.0 mm, 44.2 mm, 44.4 mm, 44.6 mm, 44.8 mm, 45.0 mm). As further discussed herein, the plurality of detectors may be positioned at varying distances from the plurality of LEDs to enable multi-depth tissue analysis, with the different source-detector distances corresponding to different optical path lengths through the tissue and therefore different tissue sampling depths.

As particularly shown in FIG. 6, the second component 406 may include a top portion 406a and a bottom portion 406b that may be integrated together to form the second component 406 and to house at least some of the electronics of the NIRS system 400, as discussed herein.

In some embodiments, the second component 406 may have a length L2 of about 20 mm to about 50 mm (e.g., about 20.0 mm, about 20.2 mm, about 20.4 mm, about 20.6 mm, about 20.8 mm, about 21.0 mm, about 21.2 mm, about 21.4 mm, about 21.6 mm, about 21.8 mm, about 22.0 mm, about 22.2 mm, about 22.4 mm, about 22.6 mm, about 22.8 mm, about 23.0 mm, about 23.2 mm, about 23.4 mm, about 23.6 mm, about 23.8 mm, about 24.0 mm, about 24.2 mm, about 24.4 mm, about 24.6 mm, about 24.8 mm, about 25.0 mm, about 25.2 mm, about 25.4 mm, about 25.6 mm, about 25.8 mm, about 26.0 mm, about 26.2 mm, about 26.4 mm, about 26.6 mm, about 26.8 mm, about 27.0 mm, about 27.2 mm, about 27.4 mm, about 27.6 mm, about 27.8 mm, about 28.0 mm, about 28.2 mm, about 28.4 mm, about 28.6 mm, about 28.8 mm, about 29.0 mm, about 29.2 mm, about 29.4 mm, about 29.6 mm, about 29.8 mm, about 30.0 mm, about 30.2 mm, about 30.4 mm, about 30.6 mm, about 30.8 mm, about 31.0 mm, about 31.2 mm, about 31.4 mm, about 31.6 mm, about 31.8 mm, about 32.0 mm, about 32.2 mm, about 32.4 mm, about 32.6 mm, about 32.8 mm, about 33.0 mm, about 33.2 mm, about 33.4 mm, about 33.6 mm, about 33.7 mm, about 33.8 mm, about 34.0 mm, about 34.2 mm, about 34.4 mm, about 34.6 mm, about 34.8 mm, about 35.0 mm, about 35.2 mm, about 35.4 mm, about 35.6 mm, about 35.8 mm, about 36.0 mm, about 36.2 mm, about 36.4 mm, about 36.6 mm, about 36.8 mm, about 37.0 mm, about 37.2 mm, about 37.4 mm, about 37.6 mm, about 37.8 mm, about 38.0 mm, about 38.2 mm, about 38.4 mm, about 38.6 mm, about 38.8 mm, about 39.0 mm, about 39.2 mm, about 39.4 mm, about 39.6 mm, about 39.8 mm, about 40.0 mm, about 40.2 mm, about 40.4 mm, about 40.6 mm, about 40.8 mm, about 41.0 mm, about 41.2 mm, about 41.4 mm, about 41.6 mm, about 41.8 mm, about 42.0 mm, about 42.2 mm, about 42.4 mm, about 42.6 mm, about 42.8 mm, about 43.0 mm, about 43.2 mm, about 43.4 mm, about 43.6 mm, about 43.8 mm, about 44.0 mm, about 44.2 mm, about 44.4 mm, about 44.6 mm, about 44.8 mm, about 45.0 mm, about 45.2 mm, about 45.4 mm, about 45.6 mm, about 45.8 mm, about 46.0 mm, about 46.2 mm, about 46.4 mm, about 46.6 mm, about 46.8 mm, about 47.0 mm, about 47.2 mm, about 47.4 mm, about 47.6 mm, about 47.8 mm, about 48.0 mm, about 48.2 mm, about 48.4 mm, about 48.6 mm, about 48.8 mm, about 49.0 mm, about 49.2 mm, about 49.4 mm, about 49.6 mm, about 49.8 mm, about 50.0 mm). In some embodiments, the dimensions of the first and second components 402, 406 may be the same, while in other embodiments, the dimensions of the first and second components 402, 406 may differ. The sizing selected for the first and/or second components 402, 406 may depend on the electronics being housed in each component. For example, the first component 402 may house the electronics associated with the first detector, the plurality of light sources, and/or the user interface, while the second component 406 may house the remaining detector electronics, and signal conditioning circuitry. It should be understood that the various electronics may be modified in terms of which are housed in the first versus second component 402, 406 such that the NIRS system 400 may be in a compact and wearable form factor.

In some embodiments, the plurality of detectors may comprise a plurality of photodetectors positioned at varying distances from the plurality of LEDs to enable multi-depth tissue analysis. In some cases, the plurality of photodetectors may comprise photodiodes with dynamic gain adjustment. The dynamic gain adjustment may allow the system to adapt to different signal levels encountered across various tissue types, skin tones, and measurement conditions. The dynamic gain adjustment may also allow the NIRS system 400 to maintain signal levels across different measurement conditions. For example, when measuring tissue with high optical absorption or scattering, the gain may be increased to maintain adequate signal levels, while when measuring tissue with lower optical attenuation, the gain may be decreased to prevent signal saturation. The dynamic gain adjustment may be performed automatically by the processor based on detected signal levels, or may be configured during an initial calibration sequence when the sensor is first applied to the user's body.

The plurality of photodetectors may comprise at least four photodetectors positioned at distances ranging from about 5 mm to about 45 mm (e.g., from about 5.0 mm, 5.2 mm, 5.4 mm, 5.6 mm, 5.8 mm, 6.0 mm, 6.2 mm, 6.4 mm, 6.6 mm, 6.8 mm, 7.0 mm, 7.2 mm, 7.4 mm, 7.6 mm, 7.8 mm, 8.0 mm, 8.2 mm, 8.4 mm, 8.6 mm, 8.8 mm, 9.0 mm, 9.2 mm, 9.4 mm, 9.6 mm, 9.8 mm, 10.0 mm, 10.2 mm, 10.4 mm, 10.6 mm, 10.8 mm, 11.0 mm, 11.2 mm, 11.4 mm, 11.6 mm, 11.8 mm, 12.0 mm, 12.2 mm, 12.4 mm, 12.6 mm, 12.8 mm, 13.0 mm, 13.2 mm, 13.4 mm, 13.6 mm, 13.8 mm, 14.0 mm, 14.2 mm, 14.4 mm, 14.6 mm, 14.8 mm, 15.0 mm, 15.2 mm, 15.4 mm, 15.6 mm, 15.8 mm, 16.0 mm, 16.2 mm, 16.4 mm, 16.6 mm, 16.8 mm, 17.0 mm, 17.2 mm, 17.4 mm, 17.6 mm, 17.8 mm, 18.0 mm, 18.2 mm, 18.4 mm, 18.6 mm, 18.8 mm, 19.0 mm, 19.2 mm, 19.4 mm, 19.6 mm, 19.8 mm, 20.0 mm, 20.2 mm, 20.4 mm, 20.6 mm, 20.8 mm, 21.0 mm, 21.2 mm, 21.4 mm, 21.6 mm, 21.8 mm, 22.0 mm, 22.2 mm, 22.4 mm, 22.6 mm, 22.8 mm, 23.0 mm, 23.2 mm, 23.4 mm, 23.6 mm, 23.8 mm, 24.0 mm, 24.2 mm, 24.4 mm, 24.6 mm, 24.8 mm, 25.0 mm, 25.2 mm, 25.4 mm, 25.6 mm, 25.8 mm, 26.0 mm, 26.2 mm, 26.4 mm, 26.6 mm, 26.8 mm, 27.0 mm, 27.2 mm, 27.4 mm, 27.6 mm, 27.8 mm, 28.0 mm, 28.2 mm, 28.4 mm, 28.6 mm, 28.8 mm, 29.0 mm, 29.2 mm, 29.4 mm, 29.6 mm, 29.8 mm, 30.0 mm, 30.2 mm, 30.4 mm, 30.6 mm, 30.8 mm, 31.0 mm, 31.2 mm, 31.4 mm, 31.6 mm, 31.8 mm, 32.0 mm, 32.2 mm, 32.4 mm, 32.6 mm, 32.8 mm, 33.0 mm, 33.2 mm, 33.4 mm, 33.6 mm, 33.8 mm, 34.0 mm, 34.2 mm, 34.4 mm, 34.6 mm, 34.8 mm, 35.0 mm, 35.2 mm, 35.4 mm, 35.6 mm, 35.8 mm, 36.0 mm, 36.2 mm, 36.4 mm, 36.6 mm, 36.8 mm, 37.0 mm, 37.2 mm, 37.4 mm, 37.6 mm, 37.8 mm, 38.0 mm, 38.2 mm, 38.4 mm, 38.6 mm, 38.8 mm, 39.0 mm, 39.2 mm, 39.4 mm, 39.6 mm, 39.8 mm, 40.0 mm, 40.2 mm, 40.4 mm, 40.6 mm, 40.8 mm, 41.0 mm, 41.2 mm, 41.4 mm, 41.6 mm, 41.8 mm, 42.0 mm, 42.2 mm, 42.4 mm, 42.6 mm, 42.8 mm, 43.0 mm, 43.2 mm, 43.4 mm, 43.6 mm, 43.8 mm, 44.0 mm, 44.2 mm, 44.4 mm, 44.6 mm, 44.8 mm, 45.0 mm) from the plurality of LEDs with angular positioning configured for tissue penetration depth analysis. The angular positioning of the photodetectors may be configured to maximize collection of backscattered light from the tissue volume of interest while minimizing collection of surface-reflected light that does not carry information about deep tissue oxygenation.

In some embodiments, the NIRS system 400 may include one or more optical sensor arrays that house the multiple light sources and/or multiple photodetectors arranged in a predetermined pattern. The predetermined pattern of the optical sensor array may be configured to provide measurements at multiple source-detector distances, enabling multi-depth tissue analysis. The optical sensor array(s) may comprise at least six light sources emitting at wavelengths from about 500 nm and 950 nm (e.g., 500 nm, 510 nm, 520 nm, 530 nm, 540 nm, 550 nm, 560 nm, 570 nm, 580 nm, 590 nm, 600 nm, 610 nm, 620 nm, 630 nm, 640 nm, 650 nm, 660 nm, 670 nm, 680 nm, 690 nm, 700 nm, 710 nm, 720 nm, 730 nm, 740 nm, 750 nm, 760 nm, 770 nm, 780 nm, 790 nm, 800 nm, 810 nm, 820 nm, 830 nm, 840 nm, 850 nm, 860 nm, 870 nm, 880 nm, 890 nm, 900 nm, 910 nm, 920 nm, 930 nm, 940 nm, 950 nm).

As particularly shown in FIG. 8, the predetermined pattern of the sensor array may have an overall length L7 of about 10 mm to about 28 mm (e.g., about 10.0 mm, about 10.2 mm, about 10.4 mm, about 10.6 mm, about 10.8 mm, about 11.0 mm, about 11.2 mm, about 11.4 mm, about 11.6 mm, about 11.8 mm, about 12.0 mm, about 12.2 mm, about 12.4 mm, about 12.6 mm, about 12.8 mm, about 13.0 mm, about 13.2 mm, about 13.4 mm, about 13.6 mm, about 13.8 mm, about 14.0 mm, about 14.2 mm, about 14.4 mm, about 14.6 mm, about 14.8 mm, about 15.0 mm, about 15.2 mm, about 15.4 mm, about 15.6 mm, about 15.8 mm, about 16.0 mm, about 16.2 mm, about 16.4 mm, about 16.6 mm, about 16.8 mm, about 17.0 mm, about 17.2 mm, about 17.4 mm, about 17.6 mm, about 17.8 mm, about 18.0 mm, about 18.2 mm, about 18.4 mm, about 18.6 mm, about 18.8 mm, about 19.0 mm, about 19.2 mm, about 19.4 mm, about 19.6 mm, about 19.8 mm, about 20.0 mm, about 20.2 mm, about 20.4 mm, about 20.6 mm, about 20.8 mm, about 21.0 mm, about 21.2 mm, about 21.4 mm, about 21.6 mm, about 21.8 mm, about 22.0 mm, about 22.2 mm, about 22.4 mm, about 22.6 mm, about 22.8 mm, about 23.0 mm, about 23.2 mm, about 23.4 mm, about 23.6 mm, about 23.8 mm, about 24.0 mm, about 24.2 mm, about 24.4 mm, about 24.6 mm, about 24.8 mm, about 25.0 mm, about 25.2 mm, about 25.4 mm, about 25.6 mm, about 25.8 mm, about 26.0 mm, about 26.2 mm, about 26.4 mm, about 26.6 mm, about 26.8 mm, about 27.0 mm, about 27.2 mm, about 27.4 mm, about 27.6 mm, about 27.8 mm, about 28.0 mm), and an overall width W3 of about 6 mm to about 22 mm (e.g., about 6.0 mm, about 6.2 mm, about 6.4 mm, about 6.6 mm, about 6.8 mm, about 7.0 mm, about 7.2 mm, about 7.4 mm, about 7.6 mm, about 7.8 mm, about 8.0 mm, about 8.2 mm, about 8.4 mm, about 8.6 mm, about 8.8 mm, about 9.0 mm, about 9.2 mm, about 9.4 mm, about 9.6 mm, about 9.8 mm, about 10.0 mm, about 10.2 mm, about 10.4 mm, about 10.6 mm, about 10.8 mm, about 11.0 mm, about 11.2 mm, about 11.4 mm, about 11.6 mm, about 11.8 mm, about 12.0 mm, about 12.2 mm, about 12.4 mm, about 12.6 mm, about 12.8 mm, about 13.0 mm, about 13.2 mm, about 13.4 mm, about 13.6 mm, about 13.8 mm, about 14.0 mm, about 14.2 mm, about 14.4 mm, about 14.6 mm, about 14.8 mm, about 15.0 mm, about 15.2 mm, about 15.4 mm, about 15.6 mm, about 15.8 mm, about 16.0 mm, about 16.2 mm, about 16.4 mm, about 16.6 mm, about 16.8 mm, about 17.0 mm, about 17.2 mm, about 17.4 mm, about 17.6 mm, about 17.8 mm, about 18.0 mm, about 18.2 mm, about 18.4 mm, about 18.6 mm, about 18.8 mm, about 19.0 mm, about 19.2 mm, about 19.4 mm, about 19.6 mm, about 19.8 mm, about 20.0 mm, about 20.2 mm, about 20.4 mm, about 20.6 mm, about 20.8 mm, about 21.0 mm, about 21.2 mm, about 21.4 mm, about 21.6 mm, about 21.8 mm, about 22.0 mm).

In some embodiments, the NIRS system 400 may include a memory (e.g., memory 324) with dedicated buffering for continuous data streaming. The memory may be in electronic communication with the processor and may be configured as a non-transient memory device for storing program instructions, calibration data, and/or recorded physiological measurements. The dedicated buffering capability may enable the NIRS system to maintain continuous data acquisition and streaming operations without data loss during periods of high processing demand or wireless transmission delays. In some embodiments, the NIRS system utilizes static allocation buffers to reduce or avoid memory overload.

In some embodiments, the memory may utilize a low-priority thread processing approach, such as multi-threading to reduce or avoid conflicts between one or more other components of the NIRS system (e.g., the processor, detectors, emitters, etc.). In some embodiments, the memory may be internal to a microcontroller (in some embodiments, the processor). The microcontroller may be a NRF5340 system including a dual core processor having, for example, an Application Processor (AP) for higher performance functions, and a Network Processor (NP) for lower-power functions. The AP may be configured to handle user applications, complex algorithms, and demanding features, running at up to about 128 MHz for high performance. The AP can achieve 510 CoreMark at about 128 MHz, while the NP can deliver 238 CoreMark at about 64 MHz, providing significant computational ability. The AP includes a Floating Point Unit (FPU), Digital Signal Processing (DSP) instructions, and an 8KB cache, boosting performance for intensive math and signal processing. In some embodiments, as further discussed herein, in addition to the internal memory of the microcontroller, the NIRS system 400 may also include a separate flash memory external to the microcontroller that allows for long term storage of data.

As particularly shown in FIGS. 4, 6, and 8, in some embodiments, the NIRS system 400 may include a flexible hinge 410 connecting the first and second components 402, 406 thereby enabling the NIRS system 400 to conform to the shape of the body part of the user while maintaining optical alignment. As shown, the hinge may include a top portion 410a, connecting the respective top portions 402a, 406a of the first and second components 402, 406, and/or a bottom portion 410b, connecting the respective bottom portions 402b, 406b of the first and second components 402, 406. The flexible hinge 410 may allow for the conforming of the NIRS system 400 to the shape of the user's forehead. In some embodiments, the top portion 410a may have a length L4 (e.g., in its unflexed state, FIG. 6) of about 2 mm to about 8 mm (e.g., about 2.0 mm, about 2.2 mm, about 2.4 mm, about 2.6 mm, about 2.8 mm, about 3.0 mm, about 3.2 mm, about 3.4 mm, about 3.6 mm, about 3.8 mm, about 4.0 mm, about 4.2 mm, about 4.4 mm, about 4.6 mm, about 4.8 mm, about 5.0 mm, about 5.2 mm, about 5.4 mm, about 5.6 mm, about 5.8 mm, about 6.0 mm, about 6.2 mm, about 6.4 mm, about 6.6 mm, about 6.8 mm, about 7.0 mm, about 7.2 mm, about 7.4 mm, about 7.6 mm, about 7.8 mm, about 8.0 mm). In some embodiments, the bottom portion 410b may have a length L5 (FIG. 6) of about 2 mm to about 8 mm (e.g., about 2.0 mm, about 2.2 mm, about 2.4 mm, about 2.6 mm, about 2.8 mm, about 3.0 mm, about 3.2 mm, about 3.4 mm, about 3.6 mm, about 3.8 mm, about 4.0 mm, about 4.2 mm, about 4.4 mm, about 4.6 mm, about 4.8 mm, about 5.0 mm, about 5.2 mm, about 5.4 mm, about 5.6 mm, about 5.8 mm, about 6.0 mm, about 6.2 mm, about 6.4 mm, about 6.6 mm, about 6.8 mm, about 7.0 mm, about 7.2 mm, about 7.4 mm, about 7.6 mm, about 7.8 mm, about 8.0 mm). In some embodiments, the bottom portion 410b may have a height H2 of about 0.4 mm to about 3 mm (e.g., about 0.4 mm, about 0.6 mm, about 0.8 mm, about 1.0 mm, about 1.2 mm, about 1.4 mm, about 1.6 mm, about 1.8 mm, about 2.0 mm, about 2.2 mm, about 2.4 mm, about 2.6 mm, about 2.8 mm, about 3.0 mm).

In such embodiments, the system 400 may be configured as a sensor patch comprising two enclosures surrounding rigid circuit boards connected by a bendable hinge of a flexible circuit board. The two enclosures (e.g., first and second components 402, 406) may house the electronic components, optical elements, and associated circuitry in a compact and protected configuration. The rigid circuit boards within each enclosure may provide mechanical support for the light sources, detectors, processor, memory, and other electronic components, while the flexible circuit board forming the bendable hinge may provide electrical interconnections between the two rigid circuit boards. The dual-enclosure design may allow the NIRS system to maintain structural integrity and component protection while providing the flexibility to conform to various body contours.

In some embodiments, the bendable hinge of the flexible circuit board may be configured to allow a range of bending angles while maintaining reliable electrical connections between the two rigid circuit boards. The flexible circuit board material may be selected to provide adequate flexibility for conforming to anatomical contours while maintaining sufficient mechanical strength to withstand repeated bending cycles during normal use. In some cases, the flexible circuit board may comprise multiple conductive layers separated by flexible dielectric materials, with the conductive traces routed to minimize stress concentrations at the bend regions. The bendable hinge may be designed with a specific bend radius that balances flexibility with durability, allowing the sensor to conform to body surfaces with curvatures typically encountered at the intended measurement locations.

The dual-enclosure design with rigid circuit boards connected by the bendable hinge may provide a balance between component protection and sensor flexibility. The rigid enclosures may protect the sensitive electronic and optical components from mechanical damage, moisture ingress, and environmental contamination, while the flexible hinge region may allow the overall sensor assembly to adapt to the user's anatomy. The rigid circuit boards may provide stable mounting platforms for the light sources, detectors, and other components that require precise positioning for accurate optical measurements. The separation of the light sources and detectors into different rigid enclosures connected by the flexible hinge may allow the source-detector distances to remain relatively stable even as the overall sensor conforms to curved surfaces.

The mechanical design of the NIRS system with the flexible hinge connecting rigid components may allow the sensor to be applied to various anatomical locations with different surface curvatures. The sensor may be placed on the dominant arm's forearm wrist extensor group, oriented vertically on the sternum, or oriented vertically on the dominant thigh depending on the intended monitoring application. The flexible hinge may allow the sensor to conform to the relatively flat surface of the sternum, the curved surface of the forearm, or the larger curved surface of the thigh while maintaining optical alignment for accurate tissue oxygenation measurements at each location. The conformability of the sensor may also accommodate variations in body size and shape among different users.

In some embodiments where the NIRS system includes the flexible hinge, the NIRS system may be configured to account for variation in distances between the light sources and the detectors, as discussed herein. For example, rather than the respective distances between light sources and detectors of the NIRS system be fixed distances, as may be the case if the NIRS system was entirely rigid, the inclusion of the flexible hinge, and thereby the ability for the NIRS system to conform to a shape of a body part to which it is adhered, allows for variation in those distances. To account for these variations, the NIRS system may be configured to smooth signal data received by the detectors. In some embodiments, the light sources and detectors might be positioned within the NIRS system such that a slight variation in distance due to the bending of the flexible hinge does not impact the NIRS system's ability to accurately process the received signal data, as discussed herein. In some embodiments, the NIRS system may be configured to automatically detect the variation in distances due to the bending of the hinge and account for such detected variation in its processing of the received signals (e.g., via processing algorithms), as discussed herein.

As particularly shown in FIG. 4, in some embodiments, the NIRS system 400 may include an integrated user interface 420 that may include any of the user interface features described herein, such as buttons, alerts, displays, etc. For example, the user interface 420 may include a first user object 422 configured to display a battery charge of the system 400, second and/or third user objects 424, 426 that may, for example, include respective buttons (e.g., a power button or button associated with any other function of the system 400), and/or fourth and/or fifth user objects 428, 430 that may, for example, display respective lights or alerts (e.g., indicating an enabled communication protocol or other function of the system 400). It should be understood that the user interface 420 may be configured in a variety of ways, for example, including any number and/or functions of user objects. In some embodiments, as further discussed herein, the NIRS system 400 may be configured to connect to a remote user interface configured to display data and/or provide any of the functions discussed herein.

In some embodiments, the NIRS system 400 may be configured to display (e.g., via user interface 420 and/or a remote user interface) raw optical data for near distance, such as about 8 mm (e.g., 4.0 mm, 4.2 mm, 4.4 mm, 4.6 mm, 4.8 mm, 5.0 mm, 5.2 mm, 5.4 mm, 5.6 mm, 5.8 mm, 6.0 mm, 6.2 mm, 6.4 mm, 6.6 mm, 6.8 mm, 7.0 mm, 7.2 mm, 7.4 mm, 7.6 mm, 7.8 mm, 8.0 mm, 8.2 mm, 8.4 mm, 8.6 mm, 8.8 mm, 9.0 mm, 9.2 mm, 9.4 mm, 9.6 mm, 9.8 mm, 10.0 mm, 10.2 mm, 10.4 mm, 10.6 mm, 10.8 mm, 11.0 mm, 11.2 mm, 11.4 mm, 11.6 mm, 11.8 mm, 12.0 mm), mid distance, such as about 30 mm or 35 mm (e.g., 25.0 mm, 25.2 mm, 25.4 mm, 25.6 mm, 25.8 mm, 26.0 mm, 26.2 mm, 26.4 mm, 26.6 mm, 26.8 mm, 27.0 mm, 27.2 mm, 27.4 mm, 27.6 mm, 27.8 mm, 28.0 mm, 28.2 mm, 28.4 mm, 28.6 mm, 28.8 mm, 29.0 mm, 29.2 mm, 29.4 mm, 29.6 mm, 29.8 mm, 30.0 mm, 30.2 mm, 30.4 mm, 30.6 mm, 30.8 mm, 31.0 mm, 31.2 mm, 31.4 mm, 31.6 mm, 31.8 mm, 32.0 mm, 32.2 mm, 32.4 mm, 32.6 mm, 32.8 mm, 33.0 mm, 33.2 mm, 33.4 mm, 33.6 mm, 33.8 mm, 34.0 mm, 34.2 mm, 34.4 mm, 34.6 mm, 34.8 mm, 35.0 mm, 35.2 mm, 35.4 mm, 35.6 mm, 35.8 mm, 36.0 mm, 36.2 mm, 36.4 mm, 36.6 mm, 36.8 mm, 37.0 mm), and far distance, such as 40 mm (e.g., 37.2, 37.5 mm, 37.7 mm, 37.9 mm, 38.1 mm, 38.3 mm, 38.5 mm, 38.7 mm, 38.9 mm, 39.1 mm, 39.3 mm, 39.5 mm, 39.7 mm, 39.9 mm, 40.1 mm, 40.3 mm, 40.5 mm, 40.7 mm, 40.9 mm, 41.1 mm, 41.3 mm, 41.5 mm, 41.7 mm, 41.9 mm, 42.1 mm, 42.3 mm, 42.5 mm, 42.7 mm, 42.9 mm, 43.1 mm, 43.3 mm, 43.5 mm, 43.7 mm, 43.9 mm, 44.1 mm, 44.3 mm, 44.5 mm, 44.7 mm, 44.9 mm) source-detector separations. The near distance measurements (e.g., at approximately 8 mm source-detector separation) may provide information about superficial tissue layers and may be used for pulse oximetry ($SpO_2$) measurements that rely on pulsatile arterial blood signals. The mid distance measurements (e.g., at approximately 30 mm or 35 mm source-detector separation) and far distance measurements (e.g., at approximately 40 mm source-detector separation) may provide information about deeper tissue layers and may be used for tissue oxygenation ($StO_2$) measurements that include contributions from arterial, venous, and capillary blood.

The user interface 420 (or other remote user interface) may be configured to display real-time physiological status information with customizable visualization modes and alerts. The user interface may provide visual, auditory, and tactile feedback mechanisms that communicate physiological measurements, system status, and alert conditions to users and clinicians. The real-time physiological status information displayed by the user interface may include tissue oxygenation values, pulse oximetry values, pulse rate, respiratory rate, and other biometric properties calculated by the NIRS monitoring wearable system. The user interface may update the displayed information continuously at the sampling rate of the system to provide immediate feedback about the user's physiological state.

The customizable visualization modes of the user interface may allow users to select different display formats and data presentations based on their preferences and the requirements of specific monitoring applications. The visualization modes may include numerical displays that present physiological values as digits, graphical displays that present physiological trends as waveforms or charts, and combined displays that present both numerical values and graphical trends simultaneously. The customizable visualization modes may allow users to select which physiological parameters are displayed, the time scale of trend displays, the color schemes used for different parameters, and the layout of information on the display screen. The customization options may be stored as user profiles that can be recalled for subsequent monitoring sessions.

The user interface may comprise a display screen and tactile feedback elements for user interaction with voice command recognition and gesture-based control capabilities. The display screen may be provided on a connected mobile device, tablet, or dedicated display unit that communicates with the NIRS monitoring wearable system via the wireless communication module. The display screen may present the real-time physiological status information, system configuration options, and data management functions through a graphical user interface. The display screen may support touch input for direct interaction with displayed elements, allowing users to select visualization modes, adjust parameters, and navigate between different display screens.

The tactile feedback elements of the user interface may include buttons, switches, or touch-sensitive surfaces on the NIRS monitoring wearable system or connected devices that provide physical feedback when activated. The tactile feedback elements may confirm user inputs through mechanical click sensations, vibration patterns, or other tactile responses that indicate successful activation of controls. The tactile feedback elements may allow users to interact with the system without requiring visual attention to the display screen, which may be beneficial during physical activity or in situations where visual attention is directed to other tasks.

The voice command recognition capability of the user interface may allow users to control system functions and request information through spoken commands. The voice command recognition may process spoken input to identify command keywords and execute corresponding system functions such as starting or stopping data acquisition, requesting current physiological values, or activating specific visualization modes. The voice command recognition may be implemented on a connected mobile device that processes audio input from a microphone and translates recognized commands into control signals transmitted to the NIRS monitoring wearable system. The voice command recognition may provide hands-free control of system functions, which may be beneficial during physical activity or clinical procedures where manual interaction with the device is inconvenient.

The gesture-based control capabilities of the user interface may allow users to control system functions through physical gestures detected by sensors on the NIRS monitoring wearable system or connected devices. The gesture-based control may use accelerometer data from the NIRS monitoring wearable system to detect specific motion patterns that correspond to control commands. The gesture-based control may also use touch gestures on the display screen of a connected mobile device, such as swipe gestures to navigate between display screens or pinch gestures to adjust the time scale of trend displays. The gesture-based control may provide intuitive interaction methods that reduce the need for precise button presses or menu navigation.

The user interface may display multi-distance raw data for near, mid, and far source-detector distances to provide visibility into the optical signals acquired at different tissue depths. The near distance raw data display may present optical signals from detectors positioned at, for example, approximately 8 mm from the light sources, corresponding to shallow tissue monitoring. The mid distance raw data display may present optical signals from detectors positioned at, for example, approximately 35 mm from the light sources, corresponding to intermediate tissue depth monitoring. The far distance raw data display may present optical signals from detectors positioned at, for example, approximately 40 mm from the light sources, corresponding to deep tissue monitoring. The multi-distance raw data display may present signals at multiple wavelengths for each source-detector distance, with different wavelengths displayed in different colors to allow visual differentiation.

The user interface may include accelerometer data display for X, Y, and Z axes to monitor device orientation and motion. The accelerometer data display may present three-axis acceleration measurements as numerical values, waveforms, or graphical indicators that show the current orientation and motion state of the NIRS monitoring wearable system. The X-axis accelerometer data may correspond to acceleration along a first axis of the device, the Y-axis accelerometer data may correspond to acceleration along a second axis perpendicular to the first axis, and the Z-axis accelerometer data may correspond to acceleration along a third axis perpendicular to both the first and second axes. The accelerometer data display may allow users and clinicians to monitor device orientation during placement and to identify motion events that may affect physiological measurements.

The accelerometer data display may be presented in a dedicated panel or graph within the user interface alongside the optical signal displays and processed biometric displays. The accelerometer waveforms may be displayed with different colors for each axis to allow visual differentiation of motion components in different directions. The accelerometer data may be used by the signal processing algorithms for motion artifact rejection, and the display of accelerometer data may allow users to correlate observed motion events with variations in the optical signals or processed physiological measurements. The accelerometer data display may also indicate when the device orientation has changed, which may be relevant for interpreting physiological measurements that are affected by body position.

The user interface may include processed biometric displays that present calculated physiological parameters in formats suitable for clinical monitoring and patient assessment. For example, the processed biometric displays may include StO2 displayed as a percentage, oxy/deoxy hemoglobin concentrations displayed in micromoles per liter, pulse rate displayed in beats per minute, and PPG waveform displayed as a continuous trace. The processed biometric displays may present current values, trend graphs showing changes over time, and statistical summaries calculated over specified time windows. The processed biometric displays may be configured to highlight values that fall outside normal ranges or that exceed configured threshold values.

In some embodiments, the NIRS system 400 may include an event marker button to flag events or transitions during data collection for post-processing analysis. The event marker button may be activated through the user interface on a connected mobile device or through a physical button on the NIRS monitoring wearable system. When the event marker button is activated, a marker may be inserted into the data stream at the current timestamp, creating a reference point that can be identified during subsequent data analysis. The event markers may be used to annotate the beginning or end of clinical assessments, the occurrence of physiological events, changes in patient activity or position, or other events that may be relevant to interpretation of the physiological measurements.

The event marker button may add a flag to the saved data file that appears as a vertical line on the displayed data graphs at the time the marker was activated. The event markers may be visible on all data display panels including the raw optical data displays, accelerometer data display, and processed biometric displays, allowing correlation of marked events with signals from all sensor channels. The event markers may be stored with the physiological data and may be preserved when data is transferred to external storage or cloud services. During post-processing analysis, the event markers may allow rapid navigation to specific time points in the data and may facilitate segmentation of data into periods corresponding to different phases of clinical assessments or monitoring protocols.

The user interface may provide timer functionality that displays elapsed time from the start of data recording. The timer may start when data acquisition is initiated and may stop when data acquisition is paused or terminated. The timer display may allow users to track the duration of monitoring sessions and to coordinate data collection with timed clinical assessments such as the six-minute walk test. The timer may be reset when a new data session is started, allowing accurate timing of subsequent monitoring periods. The timer display may be positioned prominently within the user interface to provide easy visibility of elapsed time during monitoring sessions.

The user interface may provide pause and resume functionality that allows temporary suspension of data display updates without stopping data acquisition. When the pause function is activated, the display may freeze at the current state while the NIRS monitoring wearable system continues to acquire and store physiological data. The pause functionality may allow users to examine specific features in the displayed data without the display continuously updating with new data. When the resume function is activated, the display may resume updating with current data, and the data acquired during the pause period may be concatenated with previously recorded data to maintain a continuous data record.

The user interface may provide session management functions including starting new sessions, stopping active sessions, and clearing session data. The start session function may initiate data acquisition and enable the optical systems on the NIRS monitoring wearable system. The stop session function may terminate data acquisition and disable the optical systems to conserve battery power. The clear session function may erase the current session data from the display and memory buffers, allowing a fresh data stream to be started without residual data from previous sessions. The session management functions may be accessible through buttons or menu options in the user interface, and may require confirmation before executing operations that could result in data loss.

The customizable visualization modes may include options for adjusting the time scale of trend displays, allowing users to view physiological trends over different time periods. Shorter time scales may provide higher temporal resolution for observing rapid physiological changes such as cardiac pulsations or acute responses to interventions. Longer time scales may provide visibility of gradual trends and patterns that develop over extended monitoring periods. The time scale adjustment may be performed through gesture-based controls such as pinch-to-zoom on touch screens, through menu selections, or through voice commands that specify the desired time window.

The customizable visualization modes may include options for selecting which physiological parameters are displayed and the arrangement of display elements on the screen. Users may configure the display to show a subset of available parameters that are most relevant to the current monitoring application, reducing visual clutter and focusing attention on parameters of interest. The arrangement of display elements may be customized to place frequently viewed parameters in prominent positions and to group related parameters together. The display configurations may be saved as presets that can be recalled for different monitoring scenarios or user preferences.

The system may measure tissue oxygenation at depths up to approximately 20 mm below the skin surface for monitoring skeletal muscle oximetry ($SmO_2$) or deep tissue oximetry ($StO_2$). The longer source-detector distances of 35 mm and 40 mm may enable optical interrogation of tissue volumes at these deeper depths, as the optical path length through tissue increases with source-detector separation. The multi-depth sensing capability may allow the NIRS monitoring wearable system to simultaneously assess oxygenation in both superficial and deep tissue compartments, providing a more comprehensive picture of tissue oxygenation status than single-depth measurements alone.

In some embodiments, at least two (or at least three detectors) of the NIRS system 400 (e.g., detector(s) 408b, 408b, 408d) may be configured to detect a first signal associated with $StO_2$ of the body part of the user. The detectors configured for $StO_2$ detection may be positioned at the longer source-detector distances (such as 35 mm and 40 mm) where the optical signals carry information about deeper tissue oxygenation levels. Multiple detectors for $StO_2$ measurement may provide redundancy and may allow for spatial averaging or differential measurements that improve measurement accuracy.

In some embodiments, at least one detector of the NIRS system 400 (e.g., detector 408a) may be configured to detect a second signal associated with SpO$_2$ of the body part of the user. The detector configured for SpO$_2$ detection may be positioned at a shorter source-detector distance (such as 8 mm) where the optical signals are dominated by contributions from superficial tissue layers containing pulsatile arterial blood. The SpO$_2$ measurement may rely on detecting periodic oscillations in the optical signal that correspond to the cardiac cycle, and the shorter source-detector distance may provide stronger pulsatile signal components for accurate SpO$_2$ estimation.

In some embodiments, at least two light sources of the NIRS system 400 may be configured to emit wavelengths associated with StO$_2$ measurement. The wavelengths associated with StO$_2$ measurement may include wavelengths in the near-infrared range where oxygenated hemoglobin and deoxygenated hemoglobin exhibit different absorption characteristics. In some cases, at least six light sources of the plurality of light sources may be configured to emit wavelengths associated with SpO$_2$ measurement. The wavelengths associated with SpO$_2$ measurement may include both red wavelengths (such as 660 nm) and near-infrared wavelengths (such as 940 nm) that are used in pulse oximetry algorithms to calculate arterial oxygen saturation from the ratio of pulsatile signal amplitudes at the two wavelengths.

As discussed herein, the relationship between source-detector distance and tissue sampling depth may be governed by the optical properties of biological tissue, including absorption and scattering coefficients at the measurement wavelengths. Near-infrared light traveling through tissue follows a curved path due to multiple scattering events, with the average depth of penetration increasing as the source-detector separation increases. As such, the NIRS system 400 may use the signals from detectors at different source-detector distances to calculate oxygenation values at corresponding tissue depths, for example, with the 8 mm distance providing shallow tissue information, the 30 mm and 35 mm distances providing mid-depth tissue information, and the 40 mm distance providing deep tissue information up to approximately 20 mm below the skin surface.

The multi-depth tissue analysis enabled by the range of source-detector distances from 8 mm to 40 mm may allow the NIRS monitoring wearable system to differentiate between oxygenation changes occurring in different tissue compartments. In some cases, superficial tissue oxygenation may change independently of deep tissue oxygenation, and the multi-depth measurement capability may allow detection of such differential changes. The processor of the NIRS system (e.g., processor 310) may be configured to apply depth-specific algorithms, such as any of those discussed herein, to the signals from each source-detector distance to calculate oxygenation values at the corresponding tissue depths, and may combine the multi-depth measurements to provide a comprehensive assessment of tissue oxygenation status across the full range of accessible tissue depths.

A first distance between a first light source of the NIRS system 400 (e.g., 404a, 404b, 404c) and a first detector of the NIRS system 400 (e.g., 408a) may be about 8 mm (e.g., 4.0 mm, 4.2 mm, 4.4 mm, 4.6 mm, 4.8 mm, 5.0 mm, 5.2 mm, 5.4 mm, 5.6 mm, 5.8 mm, 6.0 mm, 6.2 mm, 6.4 mm, 6.6 mm, 6.8 mm, 7.0 mm, 7.2 mm, 7.4 mm, 7.6 mm, 7.8 mm, 8.0 mm, 8.2 mm, 8.4 mm, 8.6 mm, 8.8 mm, 9.0 mm, 9.2 mm, 9.4 mm, 9.6 mm, 9.8 mm, 10.0 mm, 10.2 mm, 10.4 mm, 10.6 mm, 10.8 mm, 11.0 mm, 11.2 mm, 11.4 mm, 11.6 mm, 11.8 mm, 12.0 mm). The 8 mm source-detector distance may correspond to shallow tissue monitoring, where the optical path through tissue samples primarily superficial tissue layers including the epidermis, dermis, and subcutaneous tissue. At the 8 mm source-detector separation, the optical signals may be dominated by contributions from tissue depths of approximately 2 mm to 4 mm below the skin surface. The shallow tissue measurements obtained at the 8 mm source-detector distance may be used for PPG based measurements including SpO$_2$, pulse rate, and respiratory rate, as the pulsatile arterial blood signals may be more prominent in the superficial tissue layers.

A second distance between a second light source of the NIRS system 400 (e.g., 404a, 404b, 404c) and a second detector of the NIRS system 400 (e.g., 408d) may be about 30 mm (e.g., 25.0 mm, 25.2 mm, 25.4 mm, 25.6 mm, 25.8 mm, 26.0 mm, 26.2 mm, 26.4 mm, 26.6 mm, 26.8 mm, 27.0 mm, 27.2 mm, 27.4 mm, 27.6 mm, 27.8 mm, 28.0 mm, 28.2 mm, 28.4 mm, 28.6 mm, 28.8 mm, 29.0 mm, 29.2 mm, 29.4 mm, 29.6 mm, 29.8 mm, 30.0 mm, 30.2 mm, 30.4 mm, 30.6 mm, 30.8 mm, 31.0 mm, 31.2 mm, 31.4 mm, 31.6 mm, 31.8 mm, 32.0 mm, 32.2 mm, 32.4 mm). The 30 mm source-detector distance may correspond to mid-distance measurements that sample tissue at intermediate depths between the shallow and deep tissue compartments. At the 30 mm source-detector separation, the optical path through tissue may extend to depths of approximately 10 mm to 15 mm below the skin surface, depending on the optical properties of the tissue being measured. The mid-distance measurements obtained at the 30 mm source-detector distance may provide information about tissue oxygenation in muscle tissue, adipose tissue, or other structures located beneath the superficial skin layers.

A third distance between a third light source of the NIRS system 400 (e.g., 404a, 404b, 404c) and a third detector of the NIRS system 400 (e.g., 408c) may be about 35 mm (e.g., 32.6 mm, 32.8 mm, 33.0 mm, 33.2 mm, 33.4 mm, 33.6 mm, 33.8 mm, 34.0 mm, 34.2 mm, 34.4 mm, 34.6 mm, 34.8 mm, 35.0 mm, 35.2 mm, 35.4 mm, 35.6 mm, 35.8 mm, 36.0 mm, 36.2 mm, 36.4 mm, 36.6 mm, 36.8 mm, 37.0 mm). The 35 mm source-detector distance may also correspond to mid-distance measurements and may provide tissue sampling at depths of approximately 12 mm to 17 mm below the skin surface. The 35 mm source-detector separation may be used in combination with the 30 mm source-detector separation to provide multiple mid-depth measurements that can be averaged or compared to improve measurement accuracy and reduce sensitivity to local tissue heterogeneities. In some cases, the 35 mm source-detector distance may be used as the primary mid-distance measurement when a single mid-depth measurement is sufficient for the intended application.

A fourth distance between a fourth light source of the NIRS system 400 (e.g., 404a, 404b, 404c) and a fourth detector of the NIRS system 400 (e.g., 408b) may be about 40 mm (e.g., 37.2, 37.5 mm, 37.7 mm, 37.9 mm, 38.1 mm, 38.3 mm, 38.5 mm, 38.7 mm, 38.9 mm, 39.1 mm, 39.3 mm, 39.5 mm, 39.7 mm, 39.9 mm, 40.1 mm, 40.3 mm, 40.5 mm, 40.7 mm, 40.9 mm, 41.1 mm, 41.3 mm, 41.5 mm, 41.7 mm, 41.9 mm, 42.1 mm, 42.3 mm, 42.5 mm, 42.7 mm, 42.9 mm, 43.1 mm, 43.3 mm, 43.5 mm, 43.7 mm, 43.9 mm, 44.1 mm, 44.3 mm, 44.5 mm, 44.7 mm, 44.9 mm). The 40 mm source-detector distance may correspond to deep tissue penetration measurements that sample tissue at the greatest depths accessible by the NIRS monitoring wearable system. At the 40 mm source-detector separation, the optical path through tissue may extend to depths of approximately 15 mm to 20 mm below the skin surface, enabling measurement of tissue oxygenation in deep muscle tissue, bone marrow, or other deep tissue structures depending on the anatomical location of sensor placement. The deep tissue measurements obtained at the 40 mm source-detector distance may be used for $StO_2$ measurements that reflect the oxygenation status of the microvascular blood pool including arterial, venous, and capillary blood compartments.

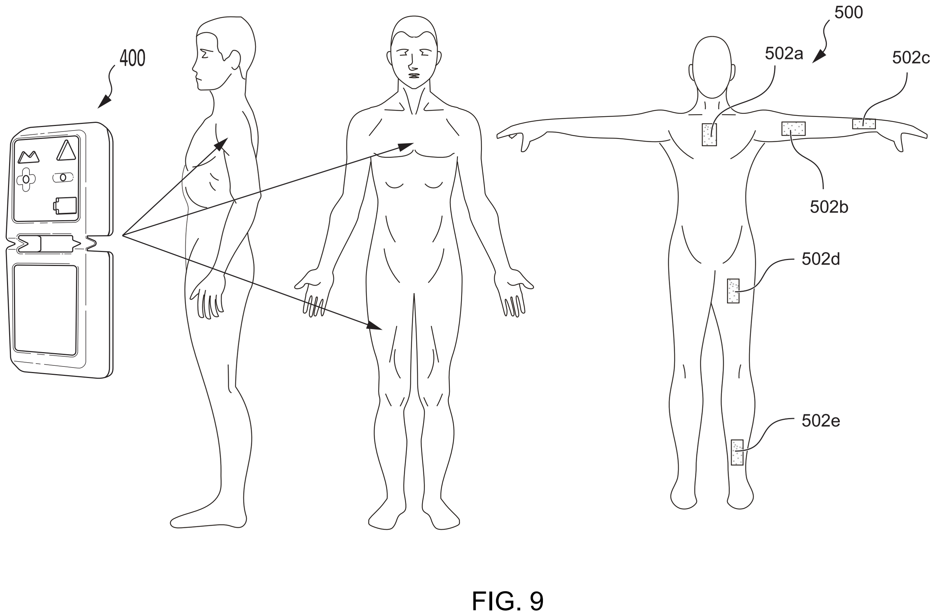

FIG. 9 provides examples of parts of the body on which the NIRS system 400 may be adhered for biometric property monitoring, as further discussed herein. For example, the NIRS system 400 may be adhered to the sternum around position 502a, the upper arm around position 502b, the lower arm or wrist around position 502c, the thigh around position 502d, and/or the lower leg or ankle around position 502e. The NIRS system 400 may be adhered to the surface of a user's skin through the use of a biocompatible adhesive. The adhesive may comprise a biocompatible 3M combination adhesive configured for robust device attachment to the subject in various circumstances. The combination adhesive may provide both initial tack for easy application and sustained adhesion for extended monitoring periods. The biocompatible adhesive may be configured to maintain secure attachment during physical activity, perspiration, and other conditions that could compromise adhesion while allowing removal without causing skin damage or excessive discomfort.

In some embodiments, the adhesive may include one or more optical windows with cutouts configured to ensure proper alignment of the system on the skin surface while preventing blocking of optical data collection. The optical windows may be positioned over the light sources and detectors to allow light transmission between the sensor components and the tissue being measured. The cutouts in the adhesive may be aligned with the optical windows to prevent the adhesive material from covering or partially obstructing the optical apertures. Proper alignment of the optical windows with the adhesive cutouts may help maintain consistent optical coupling between the sensor and the tissue surface, which may improve measurement accuracy and repeatability. In some cases, the adhesive may include alignment features or markings that facilitate correct positioning of the adhesive relative to the optical windows during sensor application.

In some embodiments, the NIRS system 400 may comprise hermetically sealed optical windows that provide protection for the optical components while allowing light transmission for tissue oxygenation measurements. The hermetically sealed optical windows may prevent moisture, dust, and other contaminants from entering the sensor housing through the optical apertures. The hermetic sealing of the optical windows may be achieved through the use of adhesive bonding, welding, or other sealing techniques that create a continuous barrier between the optical window material and the sensor housing. The hermetically sealed optical windows may maintain the optical clarity and transmission characteristics of the windows over extended use periods by preventing contamination of the optical surfaces.

In some embodiments, the NIRS system 400 may include a rechargeable battery (e.g., battery 322) having a lifespan of at least two days with power management circuitry for adaptive power consumption. In some cases, the system may provide 12 to 96 hours (e.g., 12 hours, 14 hours, 16 hours, 18 hours, 20 hours, 22 hours, 24 hours, 26 hours, 28 hours, 30 hours, 32 hours, 34 hours, 36 hours, 38 hours, 40 hours, 42 hours, 44 hours, 46 hours, 48 hours, 50 hours, 52 hours, 54 hours, 56 hours, 58 hours, 60 hours, 62 hours, 64 hours, 66 hours, 68 hours, 70 hours, 72 hours, 74 hours, 76 hours, 78 hours, 80 hours, 82 hours, 84 hours, 86 hours, 88 hours, 90 hours, 92 hours, 94 hours, 96 hours) of continuous monitoring battery life depending on operational mode. The power management circuitry may be configured to automatically and/or dynamically adjust power consumption based on measurement requirements, duty cycle settings, and wireless communication activity to extend battery life during extended monitoring periods. In some embodiments, the rechargeable battery may have fast-charging capability with thermal protection. The extended battery lifespan may enable the NIRS system to support continuous physiological monitoring applications without requiring frequent recharging, which may be beneficial for remote patient monitoring, ambulatory monitoring during daily activities, and clinical research applications that require uninterrupted data collection over multiple days.

In some embodiments, the rechargeable battery may be configured to extend its lifespan on a single charge by duty cycling the emitters. In some embodiments, a portable battery charger can be used to recharge the battery while the NIRS system is in use to extend the usable time and avoid having to discontinue use of the NIRS system while being used to monitor a patient.

In some embodiments, the rechargeable battery may comprise a rechargeable lithium-ion battery that provides high energy density in a compact form factor suitable for wearable applications. Lithium-ion battery chemistry may offer advantages including high specific energy, low self-discharge rates, and the ability to be recharged many times without significant capacity degradation. The lithium-ion battery may be configured with a capacity selected to provide the target battery lifespan of at least two to three days while maintaining the overall size and weight of the NIRS system within acceptable limits for comfortable extended wear.

In some embodiments, the NIRS system 400 may comprise power management circuitry for adaptive power consumption. The power management circuitry may be configured to monitor the power requirements of the various system components and adjust power delivery to optimize battery life while maintaining measurement performance. The adaptive power consumption capability may allow the NIRS system to reduce power consumption during periods of lower activity or when full measurement capability is not required, thereby extending the effective battery lifespan beyond what would be achieved with constant power consumption.

The power management circuitry may implement intelligent power management that dynamically adjusts power consumption based on measurement requirements, wireless communication activity, and user-defined settings. The intelligent power management may include features such as automatic power-down of unused subsystems, voltage scaling to reduce power consumption when full performance is not required, and coordination of power-intensive operations to minimize peak power demand.

The NIRS system 400 may be configured to operate in a duty cycle sampling mode to increase battery life. In duty cycle sampling mode, the system may sample at, for example, 38 Hz (points per second) during active sampling periods and reduce power consumption during inactive periods between sampling bursts. The duty cycle represents the percentage of time the system is in an active sampling state during each measurement cycle. By reducing the duty cycle, the NIRS system may extend battery life while still collecting sufficient data for accurate physiological measurements. The duty cycle sampling mode may allow data collection for about 24 to 96 hours (e.g., 25 hours, 40 hours, 55 hours, 70 hours, 85 hours, 95 hours) depending on the selected duty cycle percentage and other operational parameters.

The dynamic power scaling based on measurement requirements may allow the NIRS system to adjust power consumption across a range of operating levels. The NIRS system may have a power usage of about 45 to about 85 milliwatts (mW) with dynamic power scaling based on measurement requirements. In some cases, the NIRS system may have a power usage of about 50 to about 80 mW with dynamic power scaling based on measurement requirements. In some cases, the NIRS system may have a power usage of about 55 to about 75 mW with dynamic power scaling based on measurement requirements. In some cases, the NIRS system may have a power usage of about 60 to about 70 mW with dynamic power scaling based on measurement requirements.

The range of power usage levels may correspond to different operational modes and measurement configurations. Lower power consumption levels may be achieved when the system operates in reduced duty cycle modes, when fewer wavelengths are being measured, or when wireless communication is inactive. Higher power consumption levels may occur during continuous sampling at full duty cycle, when all wavelengths are being measured simultaneously, or when wireless data transmission is active. The dynamic power scaling may allow the NIRS system to automatically adjust between these power levels based on the current measurement requirements and operational state.

The rechargeable battery may comprise fast-charging capability with thermal protection. The fast-charging capability may allow the battery to be recharged more quickly than conventional charging methods, reducing the time the NIRS system is unavailable for monitoring. The thermal protection may monitor battery temperature during charging and adjust the charging rate to prevent overheating that could damage the battery or reduce battery lifespan. The thermal protection circuitry may reduce the charging current or pause charging if the battery temperature exceeds safe operating limits, and may resume normal charging when the temperature returns to acceptable levels.

The NIRS system 400 may require approximately 2.5 hours (e.g., approximately 3.0 hours, 3.5 hours, 4.0 hours) to recharge a fully depleted battery. The recharging may be performed using a USB-C charging connection that provides standardized charging capability compatible with widely available USB-C chargers and cables. The USB-C charging interface may support the power delivery requirements for fast charging while providing a compact and durable connector suitable for repeated connection and disconnection cycles. The recharge time may allow the NIRS system to be fully recharged overnight or during periods when monitoring is not required, enabling continuous monitoring capability with minimal interruption.

The rechargeable lithium-ion battery may comprise wireless charging capability in addition to the USB-C wired charging capability. Wireless charging may allow the battery to be recharged without physical connection to a charging cable, which may be convenient for users and may reduce wear on the charging connector. The wireless charging capability may be implemented using inductive charging technology that transfers power from a charging pad to a receiving coil in the NIRS system. In some cases, the wireless charging may provide a slower charging rate compared to wired USB-C charging, but may offer convenience advantages for certain use scenarios.

The rechargeable lithium-ion battery may comprise an integrated fuel gauge for accurate battery life prediction. The integrated fuel gauge may monitor battery voltage, current, and temperature to calculate the remaining battery capacity and estimate the remaining operating time. The fuel gauge may use coulomb counting, voltage-based estimation, or a combination of techniques to provide accurate battery state-of-charge information. The accurate battery life prediction provided by the integrated fuel gauge may allow users and clinicians to plan monitoring sessions and recharging schedules to avoid unexpected battery depletion during monitoring.

The NIRS system 400 may comprise a battery indicator (e.g., FIG. 4, 422) configured to display the current battery charge level. The battery indicator may comprise four LED segments where each segment represents 25 percent of battery capacity. When the battery is fully charged, all four LED segments may be illuminated, and as the battery discharges, the number of illuminated segments may decrease to indicate the remaining capacity. For example, three of four LEDs illuminated may indicate 51 to 75 percent charge capacity remaining, two LEDs may indicate 26 to 50 percent remaining, and one LED may indicate 1 to 25 percent remaining. The battery indicator may be activated by a short press of a button on the NIRS system, allowing users to check the battery status without requiring connection to an external display device.

The battery charge level may also be displayed on a connected mobile device or tablet running the NIRS monitoring application software. The battery gauge displayed in the software may provide a numerical or graphical indication of the remaining battery capacity, and may update when the NIRS system is connected to the mobile device via Bluetooth or other wireless communication. The combination of the on-device LED battery indicator and the software-based battery gauge may provide multiple ways for users to monitor battery status and plan recharging as needed to maintain continuous monitoring capability.

A power source of the wearable NIRS device may be configured to provide continuous power to the NIRS device for extended monitoring periods with intelligent power management. The power source may comprise the rechargeable lithium-ion battery in combination with the power management circuitry that implements the intelligent power management functions.

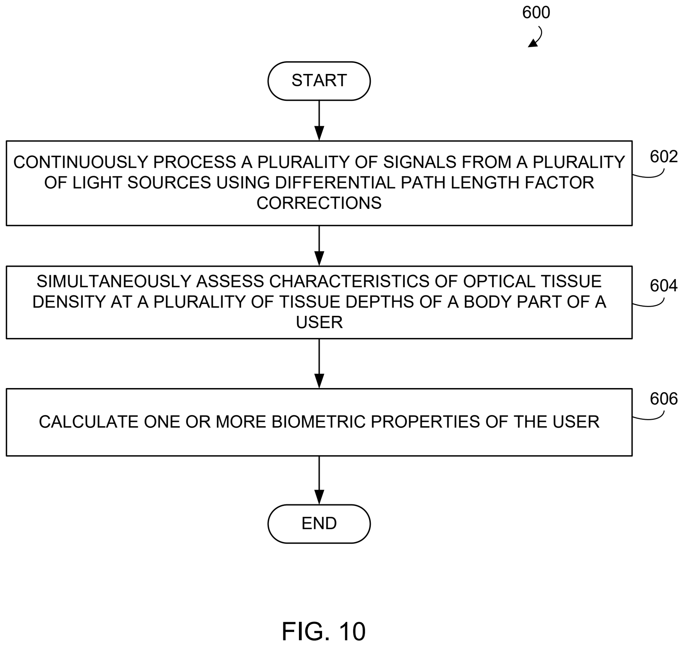

FIGS. 10-12 provide flowcharts of example methods (e.g., method 600, 700, 800) of how the NIRS system of FIGS. 4-8 can be used. It should be understood that the disclosed methods can include a variety of processing steps and as such, are discussed simultaneously throughout the disclosure.

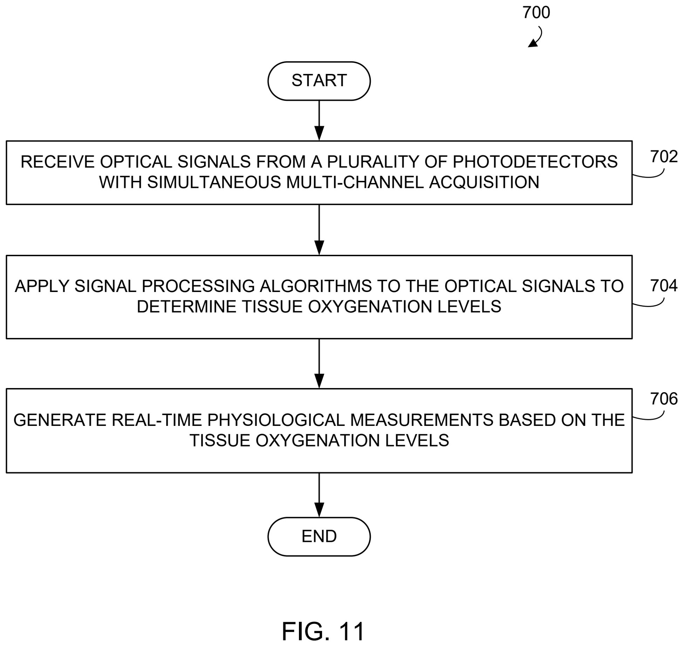

The NIRS system 400 may include one or more processors (e.g., 310) configured to receive optical signals from the plurality of photodetectors with simultaneous multi-channel acquisition, as particularly shown in block 702 of method 700 (FIG. 11). The simultaneous multi-channel acquisition may allow the processing unit to capture optical signals from all photodetectors at the same time or in rapid succession, enabling synchronized measurement of tissue oxygenation at multiple depths and wavelengths. The processor(s) may include analog-to-digital converters configured to digitize the analog signals from each photodetector channel, with the digitized signals stored in memory buffers for subsequent signal processing. The simultaneous acquisition of signals from multiple channels may reduce timing errors that could arise from sequential acquisition and may improve the accuracy of multi-depth tissue oxygenation calculations that rely on comparing signals from different source-detector distances.

The processor(s) may be configured to apply signal processing algorithms, as discussed herein, to the optical signals to determine tissue oxygenation levels, as particularly shown in block 704 of method 700 (FIG. 11). The signal processing algorithms may include preprocessing steps such as filtering, baseline correction, and normalization, followed by oxygenation calculation algorithms that convert the optical signals into tissue oxygenation values. The machine learning-based artifact rejection may employ trained models that identify and remove or attenuate signal components that do not represent true physiological changes, such as motion artifacts, ambient light interference, or sensor coupling variations. The machine learning models may be trained on datasets containing labeled examples of artifact-contaminated signals and clean signals, allowing the models to learn patterns that distinguish artifacts from physiological signals.

The adaptive calibration implemented by the processor(s) may adjust the signal processing parameters based on characteristics of the tissue being measured and the current operating conditions. The adaptive calibration may account for variations in skin tone, tissue optical properties, sensor-tissue coupling, and other factors that can affect the relationship between the measured optical signals and the underlying tissue oxygenation levels. In some cases, the adaptive calibration may be performed during an initial calibration period when the sensor is first applied to the user's body, with the calibration parameters stored and applied to subsequent measurements. The adaptive calibration may also be updated continuously during monitoring to track changes in tissue characteristics or sensor coupling that occur over time.

The processor(s) may be further configured to compensate for motion artifacts using adaptive filtering algorithms combined with inertial measurement unit data for enhanced motion artifact rejection. The NIRS system may include an accelerometer or other inertial measurement unit that detects motion of the sensor and the user's body. The inertial measurement unit may provide acceleration data for three axes (X, Y, and Z) that can be correlated with motion-induced variations in the optical signals. The adaptive filtering algorithms may use the inertial measurement unit data as a reference signal to identify and remove motion artifact components from the optical signals while preserving the physiological signal components.

The adaptive filtering algorithms for motion artifact rejection may include techniques such as adaptive noise cancellation, independent component analysis, or regression-based artifact removal. The adaptive noise cancellation approach may use the inertial measurement unit signals as a noise reference and adaptively filter the optical signals to remove components that are correlated with the motion signals. The independent component analysis approach may separate the optical signals into independent components and identify components that correspond to motion artifacts based on their correlation with the inertial measurement unit data. The regression-based approach may model the relationship between the inertial measurement unit signals and the motion artifact components in the optical signals, and subtract the estimated artifact components from the measured signals.

The combination of machine learning-based artifact rejection and adaptive filtering with inertial measurement unit data may provide enhanced motion artifact rejection compared to either approach alone. The machine learning-based artifact rejection may identify and remove artifacts that have characteristic patterns in the optical signals, while the adaptive filtering with inertial measurement unit data may remove artifacts that are correlated with detected motion events. The dual approach may allow the NIRS monitoring wearable system to maintain accurate tissue oxygenation measurements during physical activity, postural changes, and other conditions that produce motion artifacts in the optical signals.

The NIRS system 400 may be configured to continuously process the plurality of signals using differential path length factor corrections, as particularly shown in block 602 of method 600 (FIG. 10). The differential path length factor represents the ratio of the actual optical path length through tissue to the geometric source-detector distance, accounting for the increased path length that results from multiple scattering of light in biological tissue. The differential path length factor may vary with wavelength, tissue type, and source-detector distance, and accurate tissue oxygenation calculations may require applying appropriate differential path length factor corrections to the measured optical signals. The processing unit may store differential path length factor values for each wavelength and source-detector distance combination, and may apply these corrections during the signal processing to convert the measured optical attenuation values into tissue oxygenation estimates.

The differential path length factor corrections may be applied as part of the modified Beer-Lambert law calculations used to determine tissue oxygenation from the measured optical signals. The modified Beer-Lambert law relates the optical attenuation at each wavelength to the concentrations of oxygenated hemoglobin and deoxygenated hemoglobin in the tissue, with the differential path length factor accounting for the scattering-induced increase in optical path length. The processor(s) may calculate the concentrations of oxygenated hemoglobin and deoxygenated hemoglobin from the multi-wavelength optical measurements, and may then calculate the tissue oxygen saturation as the ratio of oxygenated hemoglobin to total hemoglobin concentration.

The processor(s) may be configured to execute one or more multi-depth tissue analysis algorithms that process signals from detectors at different source-detector distances to determine oxygenation levels and/or simultaneously assess characteristics of optical tissue density at corresponding tissue depths, as particularly shown in block 604 of method 600 (FIG. 10). The multi-depth tissue analysis algorithms may apply depth-specific differential path length factor corrections to the signals from each source-detector distance, as the differential path length factor may vary with the tissue depth being sampled. The multi-depth tissue analysis algorithms may also account for the different tissue volumes sampled at each source-detector distance, with shorter distances sampling primarily superficial tissue and longer distances sampling deeper tissue compartments.

The multi-depth tissue analysis algorithms may include spatially resolved spectroscopy techniques that use the relationship between optical attenuation and source-detector distance to calculate tissue optical properties and oxygenation levels. The spatially resolved spectroscopy approach may analyze the slope of optical attenuation versus source-detector distance to determine the absorption and scattering coefficients of the tissue, which may then be used to calculate tissue oxygenation. In some cases, the multi-depth tissue analysis algorithms may combine measurements from multiple source-detector distances using weighted averaging or other combination techniques to improve measurement accuracy and reduce sensitivity to local tissue heterogeneities.

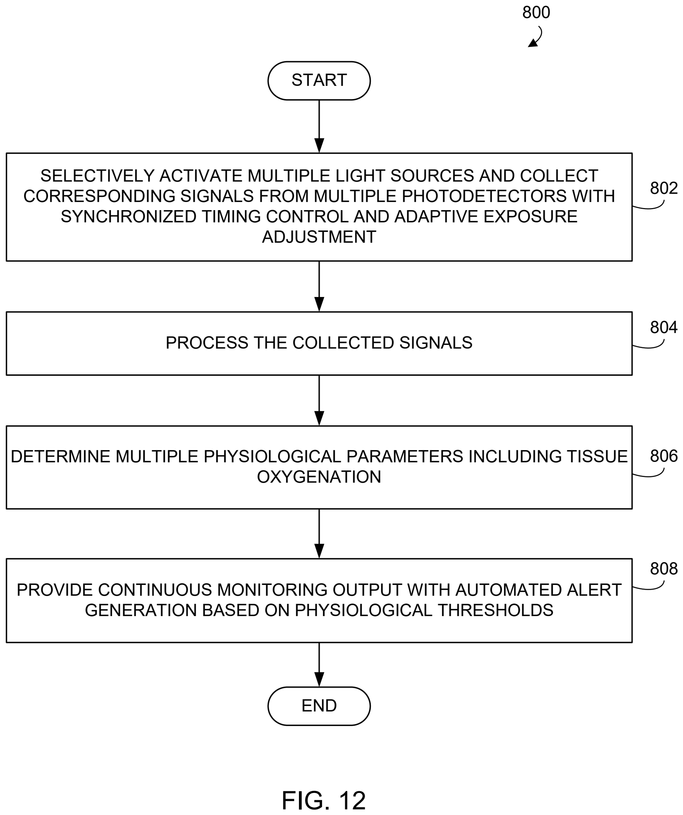

The processor(s) may be further configured to selectively activate the multiple light sources and collect corresponding signals from the multiple photodetectors with synchronized timing control and adaptive exposure adjustment, as particularly shown in block 802 of method 800 (FIG. 12). The control circuit may generate timing signals that control when each light source is activated and when the corresponding detector signals are sampled. The synchronized timing control may ensure that the detector signals are sampled at the appropriate times relative to the light source activation to capture the optical signals while minimizing interference from ambient light or signals from other light sources.

The processor(s) may be configured to operate the multiple light sources in a time-multiplexed manner to avoid interference between measurements with adaptive timing sequences based on signal quality metrics. In time-multiplexed operation, each light source may be activated sequentially rather than simultaneously, with the detector signals sampled during each light source activation period. The time-multiplexed operation may prevent optical crosstalk between different wavelengths and may allow the use of common detectors for measuring signals from multiple light sources. The sequential activation of light sources may follow a predetermined sequence that cycles through all wavelengths and source-detector combinations to collect a complete set of multi-wavelength, multi-distance measurements.

The adaptive timing sequences implemented by the processor(s) may adjust the timing parameters based on signal quality metrics measured during operation. The signal quality metrics may include signal amplitude, signal-to-noise ratio, signal stability, and the presence of artifacts or interference. When the signal quality metrics indicate degraded signal quality, the control circuit may adjust the timing parameters to improve signal acquisition. The timing adjustments may include changes to the light source activation duration, the detector sampling timing, the interval between sequential measurements, or the overall measurement cycle rate.

The adaptive exposure adjustment implemented by the processor(s) may adjust the optical exposure parameters to maintain optimal signal levels across different tissue types, skin tones, and measurement conditions. The exposure adjustment may include changes to the light source drive current, which affects the optical power emitted by each light source, and changes to the detector integration time, which affects the amount of signal accumulated during each measurement. When the detected signal levels are too low, the processor(s) may increase the light source drive current or extend the detector integration time to improve signal strength. When the detected signal levels are too high and approaching saturation, the processor(s) may decrease the light source drive current or reduce the detector integration time to prevent signal clipping.

The system may sample data at, for example, 38 Hz (points per second) for continuous physiological monitoring. The 38 Hz sampling rate may provide sufficient temporal resolution to capture physiological variations including cardiac pulsations, respiratory modulations, and slower trends in tissue oxygenation. The sampling rate may be maintained during continuous monitoring to provide uninterrupted data streams for real-time display and analysis. In some cases, the sampling rate may be adjusted based on the measurement requirements, with higher sampling rates used when finer temporal resolution is needed and lower sampling rates used when extended battery life is prioritized over temporal resolution.

The processor(s) may coordinate the activation of light sources and the collection of detector signals to achieve the target sampling rate while completing measurements at all wavelengths and source-detector distances within each sampling period. The time-multiplexed operation may divide each sampling period into multiple time slots, with each time slot allocated to a specific light source activation and detector signal collection. The duration of each time slot and the number of time slots per sampling period may be configured to balance the requirements for complete multi-wavelength, multi-distance measurements with the target sampling rate and the available processing time for signal processing algorithms.

The processor(s) may generate real-time physiological measurements based on the tissue oxygenation levels, as particularly shown in block 706 of method 700 (FIG. 11). In some embodiments, this may be configured using predictive analytics for trend analysis. The real-time physiological measurements may include StO2, SpO2, pulse rate, respiratory rate, heart rate, perfusion, blood pressure, blood volume, total hemoglobin, macrovascular and microvascular hemodynamic coherence, and other biometric properties calculated from the processed optical signals. The predictive analytics for trend analysis may analyze the time series of physiological measurements to identify trends, detect changes from baseline values, and predict future values based on observed patterns. The predictive analytics may employ statistical methods, machine learning models, or rule-based algorithms to analyze the physiological data and generate alerts or predictions that may inform clinical decision-making.

An analysis engine (e.g., part of the processor(s)) of the NIRS system 400 may be configured to process the collected signals, as particularly shown in block 804 of method 800 (FIG. 12). In some embodiments, this processing may be done using one or more of the algorithms discussed herein. In some embodiments, this processing may be done using spectroscopic analysis techniques that may include multi-wavelength analysis methods that separate the contributions of different chromophores (such as oxygenated hemoglobin, deoxygenated hemoglobin, and other tissue components) to the measured optical signals. The real-time spectral deconvolution may decompose the multi-wavelength optical measurements into the individual chromophore contributions, allowing calculation of the concentrations of each chromophore and the resulting tissue oxygenation values.

The analysis engine may be configured to determine multiple physiological parameters including tissue oxygenation based on the processed signals, as particularly shown in block 806 of method 800 (FIG. 12). The multi-modal sensor fusion may combine information from the NIRS optical measurements, the PPG signals, the inertial measurement unit data, and any other sensor modalities included in the NIRS monitoring wearable system. The sensor fusion approach may improve the accuracy and reliability of the physiological parameter estimates by combining complementary information from multiple sensing modalities and by using redundant measurements to detect and correct errors or artifacts in individual sensor channels.

The analysis engine may be configured to apply machine learning algorithms trained on physiological data to enhance measurement accuracy with continuous learning capabilities for personalized calibration. The machine learning algorithms may be trained on datasets containing physiological measurements from multiple subjects under various conditions, allowing the algorithms to learn patterns and relationships that improve measurement accuracy across diverse populations and use scenarios. The continuous learning capabilities may allow the machine learning algorithms to adapt to individual user characteristics over time, providing personalized calibration that improves measurement accuracy for each specific user based on their physiological data history. In some embodiments, the system may also include a lookup table such that the analysis engine may utilize the lookup table to select and/or customize operation performance for individual users.

The analysis engine may extract deoxygenation and reoxygenation slopes from tissue oximetry signals to characterize cardiovascular function and disease severity. The deoxygenation slope may represent the rate at which tissue oxygen saturation decreases during periods of reduced blood flow or increased oxygen consumption, such as during vascular occlusion testing or physical activity. The reoxygenation slope may represent the rate at which tissue oxygen saturation recovers following release of vascular occlusion or cessation of activity. These slope parameters may provide quantitative measures of microvascular function that correlate with cardiovascular health status and disease severity.

The extraction of deoxygenation and reoxygenation slopes may involve identifying time periods in the tissue oximetry signal that correspond to deoxygenation events and reoxygenation events, and calculating the rate of change of tissue oxygen saturation during those periods. The analysis engine may apply linear regression or other curve-fitting techniques to the tissue oximetry data during the identified time periods to calculate the slope values. In some cases, the analysis engine may also extract additional parameters such as the minimum tissue oxygen saturation reached during deoxygenation, the maximum tissue oxygen saturation reached during reoxygenation, and the time required to reach baseline oxygen saturation following a perturbation.

The deoxygenation and reoxygenation slope parameters may be used to differentiate patients based on cardiovascular disease severity. Patients with more severe cardiovascular disease may exhibit slower reoxygenation slopes following vascular occlusion, reflecting impaired microvascular function and reduced capacity for reactive hyperemia. The analysis engine may compare measured slope parameters to reference values or threshold criteria to classify cardiovascular function status. In some cases, the slope parameters may be combined with other physiological measurements using multi-modal sensor fusion to provide a more comprehensive assessment of cardiovascular health.

The analysis engine may be configured to provide continuous monitoring output (e.g., for clinical or research applications) with automated alert generation based on physiological thresholds, as particularly shown in block 808 of method 800 (FIG. 12). The continuous monitoring output may include real-time display of tissue oxygenation values, trend graphs showing changes over time, and summary statistics calculated over specified time windows. The continuous monitoring output may be transmitted to external display devices, clinical monitoring systems, or data storage systems for review by clinicians, researchers, or patients. The format and content of the continuous monitoring output may be configurable to meet the requirements of different clinical or research applications.

The automated alert generation may monitor the physiological parameters calculated by the analysis engine and generate alerts when parameter values exceed or fall below predetermined thresholds. The physiological thresholds may be configured based on clinical guidelines, research protocols, or individual patient characteristics. In some cases, the thresholds may be set to detect clinically significant changes in tissue oxygenation, such as decreases below a minimum acceptable level or rapid changes that may indicate acute physiological events. The automated alerts may be presented as visual indicators, audible alarms, haptic feedback, or notifications transmitted to external devices.

The automated alert generation may implement multiple threshold levels corresponding to different levels of clinical concern. A first threshold level may generate an informational alert indicating that a physiological parameter has moved outside a normal range, while a second threshold level may generate a warning alert indicating a more significant deviation that may require attention. A third threshold level may generate a critical alert indicating a potentially dangerous condition that requires immediate intervention. The multi-level threshold approach may allow clinicians to prioritize their attention based on the severity of the detected condition.

The analysis engine may be configured to apply machine learning algorithms trained on physiological data to enhance measurement accuracy with continuous learning capabilities for personalized calibration. The machine learning algorithms may be trained on datasets containing physiological measurements from multiple subjects under various conditions, allowing the algorithms to learn patterns and relationships that improve measurement accuracy across diverse populations and use scenarios. The training datasets may include labeled examples of physiological signals with corresponding reference measurements obtained from clinical standard devices, allowing the machine learning algorithms to learn mappings between the NIRS measurements and accurate physiological parameter values.

The machine learning algorithms applied by the analysis engine may include neural networks, support vector machines, random forests, or other supervised learning methods configured to predict physiological parameters from the multi-wavelength optical measurements. The machine learning algorithms may also include unsupervised learning methods configured to identify patterns in the physiological data, detect anomalies, or cluster similar physiological states. In some cases, the machine learning algorithms may include deep learning architectures such as convolutional neural networks or recurrent neural networks that can learn complex temporal and spectral patterns in the physiological signals.

The continuous learning capabilities of the analysis engine may allow the machine learning algorithms to adapt to individual user characteristics over time, providing personalized calibration that improves measurement accuracy for each specific user. The continuous learning may involve updating the machine learning model parameters based on new data collected from the user during ongoing monitoring. The personalized calibration may account for individual variations in tissue optical properties, skin characteristics, and physiological responses that affect the relationship between the measured optical signals and the underlying physiological parameters.

The continuous learning capabilities may implement online learning algorithms that update model parameters incrementally as new data becomes available, without requiring complete retraining of the machine learning models. The online learning approach may allow the analysis engine to adapt to gradual changes in user characteristics over time, such as changes in tissue composition, hydration status, or cardiovascular function. In some cases, the continuous learning may be constrained to prevent excessive adaptation that could degrade measurement accuracy, with safeguards that limit the rate or magnitude of model parameter updates.

The personalized calibration provided by the continuous learning capabilities may improve measurement accuracy by accounting for systematic differences between individual users that are not captured by population-level calibration models. The personalized calibration may learn user-specific correction factors that adjust the physiological parameter estimates based on the individual's baseline characteristics and response patterns. Over time, the personalized calibration may converge to a user-specific model that provides more accurate physiological measurements than a generic population-level model.

The analysis engine may combine the spectroscopic analysis techniques, multi-modal sensor fusion, automated alert generation, and machine learning algorithms into an integrated processing pipeline that provides comprehensive physiological assessment. The integrated processing pipeline may execute in real-time to provide continuous monitoring output with minimal latency between signal acquisition and physiological parameter display. The processing pipeline may be implemented on the processor of the NIRS monitoring device, on an external computing device connected via wireless communication, or distributed across both the wearable device and external computing resources depending on the computational requirements and power constraints of the specific implementation.

The NIRS system 400 may be configured to calculate the one or more biometric properties using one or more algorithms trained on multi-modal physiological data, as particularly shown in block 606 of method 600 (FIG. 10). The multi-modal physiological data used for algorithm training may include NIRS optical measurements, PPG signals, ECG waveforms, impedance cardiography (ICG) signals, accelerometer data, and reference measurements from clinical standard devices. The algorithms trained on multi-modal physiological data may learn relationships between the sensor measurements and the target biometric properties, enabling accurate estimation of physiological parameters from the wearable sensor data. The training process may involve supervised learning techniques where the algorithms learn to map input sensor data to output physiological parameter values based on labeled training examples.

The system may combine impedance cardiography (ICG) with NIRS and PPG to measure stroke volume (SV) and cardiac output (CO) in addition to tissue oxygenation. Impedance cardiography may measure changes in thoracic impedance across the chest that result from blood volume changes during the cardiac cycle. The changes in thoracic impedance may be used to calculate stroke volume, which represents the volume of blood ejected by the heart with each beat. Cardiac output may be calculated as the product of stroke volume and heart rate, providing a measure of the total volume of blood pumped by the heart per unit time. The combination of ICG with NIRS and PPG may enable simultaneous assessment of cardiac pumping function and tissue oxygen delivery, providing complementary information about cardiovascular performance.

The system may use electrocardiography (ECG) combined with impedance cardiography (ICG) to provide simultaneous measurement of cardiac electrical and mechanical function. Electrocardiography may measure the electrical activity of the heart through electrodes placed on the skin surface, providing information about heart rhythm, conduction abnormalities, and cardiac timing. Impedance cardiography may measure the mechanical pumping action of the heart through changes in thoracic impedance. The combination of ECG and ICG may allow correlation of cardiac electrical events with mechanical events, enabling assessment of electromechanical coupling and detection of conditions where electrical and mechanical function are dissociated.

The system may display processed biometric data including $StO_2$ percentage, oxy/deoxy hemoglobin in micromoles per liter, pulse rate in BPM, and PPG waveform. The $StO_2$ percentage may represent the tissue oxygen saturation calculated from the NIRS optical measurements, expressed as a percentage of total hemoglobin that is oxygenated. The oxy/deoxy hemoglobin values in micromoles per liter may represent the concentrations of oxygenated hemoglobin and deoxygenated hemoglobin in the measured tissue volume, providing information about both oxygen saturation and total hemoglobin content. The pulse rate in beats per minute (BPM) may be derived from the PPG waveform by detecting the periodic oscillations that correspond to cardiac pulsations. The PPG waveform display may show the time-varying optical signal that reflects pulsatile blood volume changes in the superficial tissue layers.

Tissue oxygenation ($StO_2$) measurements may provide information about the balance between oxygen delivery and oxygen consumption in the measured tissue volume. The $StO_2$ value may reflect the oxygenation status of the microvascular blood pool, including contributions from arterial, venous, and capillary blood. Changes in $StO_2$ may indicate alterations in blood flow, changes in metabolic oxygen demand, or shifts in the distribution of blood between vascular compartments. The continuous monitoring of $StO_2$ may allow detection of trends and acute changes in tissue oxygenation that may be clinically significant.

Pulse oximetry ($SpO_2$) measurements may provide information about arterial blood oxygen saturation, reflecting the ability of the lungs to oxygenate blood and the adequacy of oxygen transport in the arterial circulation. The $SpO_2$ value may be calculated from the ratio of pulsatile signal amplitudes at red and near-infrared wavelengths, using the different absorption characteristics of oxygenated and deoxygenated hemoglobin at these wavelengths. The $SpO_2$ measurement may serve as a systemic indicator of respiratory function and arterial oxygenation status.

Pulse rate (PR) and heart rate measurements may be derived from the periodic oscillations in the optical signals that correspond to cardiac pulsations. The pulse rate may be calculated by detecting the peaks or other characteristic features in the PPG waveform and measuring the time intervals between successive cardiac cycles. The heart rate may also be derived from ECG signals when ECG sensing is available, providing a direct measurement of cardiac electrical activity. The pulse rate and heart rate measurements may be used to assess cardiovascular function, detect arrhythmias, and monitor responses to physical activity or physiological stressors.

Respiratory rate (RR) measurements may be derived from respiratory modulations in the optical signals or from other physiological signals that vary with breathing. The respiratory modulations may appear as low-frequency variations in the PPG waveform amplitude or baseline that correspond to the respiratory cycle. The respiratory rate may be calculated by analyzing the frequency content of these respiratory modulations or by detecting individual respiratory cycles in the signal. The respiratory rate measurement may provide information about pulmonary function and may be used to detect respiratory abnormalities or changes in breathing patterns.

Perfusion measurements may characterize the blood flow to the tissue being monitored, reflecting the adequacy of microvascular circulation. Perfusion may be assessed through analysis of the PPG signal amplitude, which may vary with changes in blood volume and blood flow in the superficial tissue layers. The NIRS measurements may also provide information about perfusion through changes in tissue hemoglobin content and oxygenation that reflect blood flow dynamics. The perfusion measurements may be used to assess microvascular function and detect conditions associated with impaired tissue blood flow.

Blood pressure measurements may be derived from analysis of the PPG waveform characteristics or from dedicated blood pressure sensing modalities. The PPG waveform may contain information related to arterial compliance and vascular tone that correlates with blood pressure. In some cases, the NIRS monitoring wearable system may incorporate algorithms that estimate blood pressure from PPG waveform features such as pulse transit time, pulse wave velocity, or waveform morphology parameters. The blood pressure estimates may be calibrated against reference measurements from standard blood pressure devices.

Blood volume measurements may be derived from the NIRS and PPG optical signals. Blood volume may be estimated from the total hemoglobin content measured by NIRS, which reflects the amount of blood present in the measured tissue volume. Changes in blood volume may indicate alterations in vascular tone, fluid status, or blood redistribution between tissue compartments.

Macrovascular and microvascular hemodynamic coherence measurements may characterize the relationship between blood flow in large vessels and perfusion in the microvascular tissue bed. Hemodynamic coherence may be assessed by comparing cardiac output or other macrovascular flow parameters with tissue oxygenation or perfusion measurements that reflect microvascular function. In healthy cardiovascular systems, increases in cardiac output may be accompanied by proportional increases in tissue perfusion, while in disease states, the coupling between macrovascular and microvascular hemodynamics may be impaired. The hemodynamic coherence measurements may provide information about the integrity of cardiovascular regulation and the effectiveness of blood flow distribution to tissues.

The NIRS system 400 may generate real-time physiological measurements based on the tissue oxygenation levels with predictive analytics for trend analysis. The real-time physiological measurements may be updated continuously at the sampling rate of the system, providing immediate feedback about the user's physiological state. The predictive analytics for trend analysis may analyze the time series of physiological measurements to identify patterns, detect deviations from baseline values, and forecast future values based on observed trends. The predictive analytics may employ statistical methods, time series analysis techniques, or machine learning models to characterize physiological trends and generate predictions.

The predictive analytics for trend analysis may detect gradual changes in physiological parameters that may indicate developing clinical conditions before acute symptoms appear. The trend analysis may calculate rates of change, identify inflection points, and compare current values to historical baselines for the individual user. In some cases, the predictive analytics may generate alerts when trend analysis indicates that physiological parameters are likely to exceed threshold values within a specified time horizon, enabling proactive clinical intervention. The predictive analytics may also identify periodic patterns in physiological data, such as circadian variations or activity-related changes, and may distinguish normal physiological variations from abnormal trends that may require clinical attention.

The algorithms trained on multi-modal physiological data may incorporate data from multiple sensing modalities to improve the accuracy and robustness of biometric property calculations. The multi-modal approach may allow the algorithms to leverage complementary information from different sensors, such as using ECG timing information to improve the accuracy of ICG-based stroke volume calculations, or using accelerometer data to distinguish motion-related signal variations from physiological changes. The algorithms may be trained using machine learning techniques that learn optimal combinations of multi-modal sensor inputs for predicting each biometric property.

The training of algorithms on multi-modal physiological data may involve collection of datasets containing synchronized measurements from the NIRS system and reference clinical devices. The reference measurements may include gold-standard assessments of the target biometric properties, such as echocardiography for cardiac function parameters, arterial blood gas analysis for oxygen saturation, or invasive hemodynamic monitoring for cardiac output. The algorithms may be trained to minimize the difference between the predicted biometric property values and the reference measurements, resulting in calibrated algorithms that provide accurate estimates from the wearable sensor data.

The NIRS system 400 may comprise a data storage component (e.g., database 330) configured to store physiological measurements and analysis results with compression algorithms for extended data retention. The data storage component may comprise non-volatile memory such as flash memory that retains stored data when power is removed from the NIRS system. The data storage component may store raw optical signals from the photodetectors, processed tissue oxygenation values, calculated biometric properties, and metadata including timestamps, sensor configuration parameters, and event markers. The storage of both raw data and processed results may allow retrospective analysis and reprocessing of the physiological measurements using different algorithms or parameters.

The compression algorithms implemented by the data storage component may reduce the storage space required for physiological measurements and analysis results, enabling extended data retention within the available memory capacity. The compression algorithms may employ lossless compression techniques that preserve all information in the original data, allowing exact reconstruction of the stored measurements when the data is retrieved. In some cases, the compression algorithms may employ lossy compression techniques that achieve higher compression ratios by discarding information that is not required for the intended analysis applications. The selection of compression algorithm and compression parameters may be configurable based on the requirements of the specific monitoring application and the available storage capacity.

The compression algorithms for extended data retention may be optimized for the characteristics of physiological data, which may exhibit temporal correlations, periodic patterns, and limited dynamic range that can be exploited for efficient compression. The compression algorithms may employ differential encoding that stores differences between successive samples rather than absolute values, reducing the number of bits required to represent slowly varying signals. The compression algorithms may also employ predictive coding that uses models of expected signal behavior to encode deviations from predicted values, achieving efficient compression for signals with predictable patterns such as cardiac pulsations or respiratory modulations.

The data storage component may implement data management functions including file system organization, data indexing, and storage allocation that facilitate efficient storage and retrieval of physiological measurements. The file system organization may structure stored data into sessions, segments, or other logical units that correspond to monitoring periods, clinical assessments, or user-defined events. The data indexing may maintain metadata that allows rapid location of specific data segments based on timestamps, event markers, or other search criteria. The storage allocation may manage the available memory capacity to accommodate new data while preserving previously stored data according to configured retention policies.

The NIRS system 400 may store data locally on internal flash memory when operating in save mode disconnected from a mobile device. The save mode may be activated through the user interface of the NIRS monitoring wearable system or through commands received from a connected mobile device prior to disconnection. When save mode is active, the system may record all acquired sensor data and calculated physiological measurements to the internal flash memory rather than streaming the data to an external device. The local storage capability may allow the NIRS system to operate autonomously for extended monitoring periods without requiring continuous connection to a mobile device or other external data collection system.

The internal flash memory may provide sufficient storage capacity to accommodate the data generated during extended monitoring sessions. The system may collect data for, for example, 24 to 72 hours continuously depending on duty cycle settings and battery capacity. The storage capacity required for extended monitoring may depend on the sampling rate, the number of sensor channels being recorded, the data resolution, and the effectiveness of the compression algorithms applied to the stored data. The internal flash memory capacity may be selected to accommodate the maximum expected monitoring duration at the highest data rate configuration, with the compression algorithms providing additional margin for extended retention.

When operating in save mode, the NIRS system 400 may continue to perform real-time signal processing and physiological parameter calculations using the onboard processor. The processed physiological measurements may be stored along with the raw sensor data, allowing immediate access to calculated values when the data is retrieved without requiring reprocessing. In some cases, the system may store only processed measurements when storage capacity is limited, with the option to store raw data when sufficient capacity is available. The storage configuration may be selectable based on the requirements of the specific monitoring application and the available storage capacity.

The data stored on the internal flash memory may be retrieved by reconnecting the NIRS system 400 to a mobile device or other external data collection system. The data transfer may be performed over the wireless communication interface, with the stored data transmitted from the internal flash memory to the external device for analysis, archival, or further processing. The data transfer process may include verification steps that confirm successful transfer of all stored data before clearing the internal flash memory to make capacity available for subsequent monitoring sessions. The data transfer progress may be indicated on the mobile device display, and the system may prevent interruption of the transfer process to avoid data loss.

The NIRS system 400 may use a Data Acquisition System (DAS) for collecting raw data along with clinical notes and subject demographic information and/or phenotypic data. The DAS may comprise software running on a mobile device, tablet, or computer that interfaces with the NIRS system to receive, display, and store physiological data. The DAS may provide a user interface for entering clinical notes, subject demographic information, and other metadata that is associated with the collected physiological measurements. The integration of clinical notes and demographic information with the physiological data may facilitate subsequent analysis by providing context for the measured values and enabling correlation of physiological parameters with clinical observations and subject characteristics.

The DAS may receive raw optical data from the NIRS monitoring wearable system 400 including measurements at multiple wavelengths and multiple source-detector distances. The raw optical data may be stored by the DAS along with timestamps that allow synchronization with other data sources and correlation with clinical events. The DAS may also receive processed physiological measurements calculated by the NIRS monitoring wearable system, including tissue oxygenation values, pulse oximetry values, pulse rate, respiratory rate, and other biometric properties. The storage of both raw data and processed measurements by the DAS may allow retrospective analysis using different processing algorithms or parameters.

The clinical notes collected by the DAS may include observations made by clinical staff during monitoring sessions, descriptions of clinical interventions or assessments performed, and annotations of events that may affect the physiological measurements. The clinical notes may be entered through text input, voice recording, or selection from predefined options depending on the user interface capabilities of the DAS. The clinical notes may be timestamped and associated with the corresponding physiological data to allow correlation of clinical observations with measured physiological changes.

The subject demographic information collected by the DAS may include age, gender, height, weight, medical history, current medications, and other characteristics that may be relevant to interpretation of the physiological measurements. The demographic information may be entered at the beginning of a monitoring session and stored with the session data for subsequent analysis. The demographic information may be used to stratify analysis results, to apply population-specific calibration parameters, or to identify correlations between subject characteristics and physiological responses.

The DAS may implement data organization structures that separate collected data by monitoring protocols, clinical assessments, or subject groups. The data organization may facilitate analysis of specific subsets of the collected data, such as comparing physiological responses during different assessment protocols or comparing measurements between subjects with different clinical characteristics. The DAS may provide data export functions that allow transfer of collected data to external analysis systems, clinical databases, or research data repositories in standard file formats.

The combination of local storage on the NIRS monitoring wearable system 400 and external storage through the DAS may provide flexibility in data collection configurations for different monitoring applications. For ambulatory monitoring during daily activities, the local storage capability may allow autonomous data collection without requiring the subject to carry a mobile device. For clinical assessments with direct supervision, the DAS may provide real-time data display and immediate access to collected measurements. For extended monitoring with periodic data retrieval, the local storage may accumulate data between retrieval sessions while the DAS provides long-term archival and analysis capabilities.

The NIRS monitoring wearable system 400 may comprise a wireless communication module configured to transmit the real-time physiological measurements to an external device using encrypted data transmission with adaptive bandwidth management. The wireless communication module may provide bidirectional communication between the NIRS monitoring wearable system and external devices such as mobile phones, tablets, computers, or clinical monitoring systems. The wireless communication module may transmit physiological measurements including tissue oxygenation values, pulse oximetry values, pulse rate, respiratory rate, and other calculated biometric properties in real-time as the measurements are acquired and processed by the NIRS monitoring wearable system.

The encrypted data transmission implemented by the wireless communication module may protect the confidentiality and integrity of physiological data during wireless transmission. The encryption may employ cryptographic algorithms that encode the transmitted data such that the data can be decoded by authorized receiving devices possessing the appropriate decryption keys. The encryption may protect against unauthorized interception of physiological data during transmission, which may be relevant for applications involving protected health information or other sensitive physiological measurements. The encryption protocols may comply with applicable security standards for medical device communications and health data protection.

The adaptive bandwidth management implemented by the wireless communication module may adjust data transmission parameters based on available communication bandwidth and data transmission requirements. When communication bandwidth is limited, the adaptive bandwidth management may reduce the data transmission rate by decreasing the sampling rate of transmitted data, applying data compression, or prioritizing transmission of processed physiological measurements over raw sensor data. When communication bandwidth is abundant, the adaptive bandwidth management may increase the data transmission rate to provide higher temporal resolution or to transmit additional data channels. The adaptive bandwidth management may monitor communication link quality and automatically adjust transmission parameters to maintain reliable data delivery while maximizing the information content of transmitted data.

The wireless communication module may support one or more of Bluetooth, Wi-Fi, and cellular communication protocols. Bluetooth communication may provide short-range wireless connectivity suitable for communication with nearby mobile devices or tablets. Wi-Fi communication may provide medium-range wireless connectivity with higher bandwidth suitable for communication with local network infrastructure. Cellular communication may provide wide-area wireless connectivity suitable for communication when Bluetooth and Wi-Fi networks are unavailable. The support for multiple communication protocols may allow the NIRS monitoring wearable system to maintain connectivity across different environments and use scenarios.

The system may connect to an Android application on a phone or tablet for real-time physiological data display and control. The Android application may provide a user interface for viewing real-time physiological measurements, configuring system parameters, and controlling data acquisition operations. The Android application may display tissue oxygenation values, pulse oximetry values, pulse rate, respiratory rate, and other biometric properties calculated by the NIRS monitoring wearable system. The Android application may also display raw optical data from the photodetectors, accelerometer data, and other sensor signals for diagnostic or research purposes.

The Android application may provide control functions for starting and stopping data acquisition sessions, configuring sampling parameters, and activating save mode for local data storage on the NIRS monitoring wearable system. The Android application may allow users to enter session metadata including subject identifiers, clinical notes, and event markers that are associated with the collected physiological data. The Android application may provide feedback about system status including battery level, connection status, and data acquisition state. The bidirectional communication between the NIRS monitoring wearable system and the Android application may allow real-time control of system operation while simultaneously receiving and displaying physiological measurements.

The system may transfer stored data to cloud storage via services such as Google Drive for remote data access and analysis. The cloud data transfer capability may allow physiological data collected by the NIRS monitoring wearable system to be uploaded to cloud storage services where the data can be accessed by clinicians, researchers, or other authorized users from remote locations. The cloud data transfer may be initiated through the Android application, which may provide functions for selecting data sessions to upload, specifying the destination cloud storage location, and monitoring the progress of data transfer operations. The cloud storage may provide long-term archival of physiological data and may facilitate sharing of data between multiple users or institutions.

The system may enable remote patient monitoring (RPM) and telemedicine integration for patients with low healthcare accessibility. Remote patient monitoring may allow clinicians to monitor physiological measurements from patients located at home or in other remote settings without requiring in-person clinical visits. The wireless communication capabilities of the NIRS monitoring wearable system may transmit physiological data to clinical monitoring systems or telemedicine platforms where clinicians can review the data and make clinical decisions. The remote patient monitoring capability may improve healthcare accessibility for patients in rural areas, patients with mobility limitations, or patients who face other barriers to accessing in-person healthcare services.

The telemedicine integration may allow real-time physician-supported medical decision making for patients who otherwise have low accessibility to healthcare. The physiological measurements transmitted by the NIRS monitoring wearable system may be incorporated into telemedicine consultations where clinicians can review tissue oxygenation trends, pulse oximetry values, and other biometric properties while communicating with patients via video or audio conferencing. The implementation of advanced medical monitoring capabilities into existing remote patient monitoring and telemedicine networks may improve physician confidence and patient outcomes by providing a higher volume of quality medical data available at the network edge. The remote patient monitoring with advanced medical wearables may also increase patient throughput, allowing physicians to manage a larger group of patients in need.

The wireless communication module may implement connection management functions that establish, maintain, and restore communication links with external devices. The connection management may include device pairing procedures that authenticate and authorize communication between the NIRS monitoring wearable system and specific external devices. The connection management may monitor link quality and automatically attempt to restore communication when connections are interrupted due to range limitations, interference, or other factors. The connection management may maintain connection state information that allows rapid reconnection when communication is temporarily interrupted, minimizing data loss and user intervention required to restore connectivity.

The Bluetooth communication supported by the wireless communication module may provide low-power wireless connectivity suitable for continuous communication with nearby mobile devices during extended monitoring periods. The Bluetooth communication may operate in the 2.4 GHz frequency band and may support data rates sufficient for real-time transmission of physiological measurements. The Bluetooth range may extend to approximately 10 meters under typical conditions, allowing users to move within a room or nearby area while maintaining connectivity with a stationary mobile device. The Bluetooth communication may implement power-saving features that reduce power consumption during periods of low data transmission activity.

The Wi-Fi communication supported by the wireless communication module may provide higher-bandwidth wireless connectivity suitable for transmission of larger data volumes such as raw sensor data or extended data sessions stored on the NIRS monitoring wearable system. The Wi-Fi communication may connect to local wireless network infrastructure, allowing the NIRS monitoring wearable system to communicate with devices on the local network or to access internet-connected services for cloud data transfer. The Wi-Fi communication may support data rates substantially higher than Bluetooth, enabling faster transfer of stored data sessions and higher-resolution real-time data streaming when bandwidth requirements exceed Bluetooth capabilities.

The cellular communication supported by the wireless communication module may provide wide-area wireless connectivity that does not depend on local network infrastructure. The cellular communication may allow the NIRS monitoring wearable system to transmit physiological data directly to cloud services or clinical monitoring systems from any location with cellular network coverage. The cellular communication may be particularly relevant for remote patient monitoring applications where patients may not have access to Wi-Fi networks or may be mobile during monitoring. The cellular communication may support data transmission over 4G LTE, 5G, or other cellular network technologies depending on the specific implementation and available network infrastructure.

The adaptive bandwidth management may implement data prioritization schemes that ensure transmission of high-priority physiological measurements when bandwidth is constrained. Processed physiological measurements such as tissue oxygenation values and pulse rate may be assigned higher priority than raw sensor data, ensuring that clinically relevant information is transmitted even when bandwidth limitations prevent transmission of complete data streams. Alert conditions or threshold violations may be assigned highest priority to ensure rapid notification of clinically significant events. The data prioritization may be configurable based on the requirements of specific monitoring applications and clinical protocols.

The encrypted data transmission may implement end-to-end encryption that protects physiological data from the point of transmission by the NIRS monitoring wearable system to the point of reception by the authorized receiving device or service. The encryption keys may be established during device pairing or authentication procedures and may be periodically refreshed to maintain security. The encryption may be transparent to the user and may not require manual key management or configuration. The encrypted data transmission may comply with healthcare data security requirements and may support audit logging of data access for regulatory compliance purposes.

*Algorithms*

In some embodiments, the disclosed NIRS systems may rely on any of the following algorithms to perform multi-depth tissue analysis.

**PPG Algorithms**

A NIRS monitoring wearable system as disclosed herein may implement signal processing algorithms for PPG, pulse rate, and $SpO_2$ calculations. The signal processing algorithms may be executed by a processor and may be supported by software applications running on connected devices such as Android applications or Python-based applications. The algorithms may process optical signals from multiple emitters and detectors to calculate physiological parameters including pulse rate, respiratory rate, $SpO_2$, and perfusion index.

In some embodiments, the PPG algorithm may calculate PPG signals using data from one or more emitters (for example, two green emitters) operating at a wavelength of approximately 500 to 600 nanometers (nm) (e.g., approximately 505 nm, 506 nm, 507 nm, 508 nm, 509 nm, 510 nm, 511 nm, 512 nm, 513 nm, 514 nm, 515 nm, 516 nm, 517 nm, 518 nm, 519 nm, 520 nm, 521 nm, 522 nm, 523 nm, 524 nm, 525 nm, 526 nm, 527 nm, 528 nm, 529 nm, 530 nm, 531 nm, 532 nm, 533 nm, 534 nm, 535 nm, 536 nm, 537 nm, 538 nm, 539 nm, 540 nm, 541 nm, 542 nm, 543 nm, 544 nm, 545 nm, 546 nm, 547 nm, 548 nm, 549 nm, 550 nm, 551 nm, 552 nm, 553 nm, 554 nm, 555 nm, 556 nm, 557 nm, 558 nm, 559 nm, 560 nm, 561 nm, 562 nm, 563 nm, 564 nm, 565 nm, 566 nm, 567 nm, 568 nm, 569 nm, 570 nm, 571 nm, 572 nm, 573 nm, 574 nm, 575 nm, 576 nm, 577 nm, 578 nm, 579 nm, 580 nm, 581 nm, 582 nm, 583 nm, 584 nm, 585 nm, 586 nm, 587 nm, 588 nm, 589 nm, 590 nm, 591 nm, 592 nm, 593 nm, 594 nm, 595 nm, 596 nm, 597 nm, 598 nm, 599 nm, and 600 nm) received by a detector positioned at a short source-detector distance of approximately 4 to 12 millimeters (mm) (e.g., approximately 4.0, 4.5, 5.0, 5.5, 6.0, 6.5, 7.0, 7.5, 8.0, 8.5, 9.0, 9.5, 10.0, 10.5, 11.0, 11.5, 12.0) mm. The signal may be processed one block at a time, where each block corresponds to one second of data at the sampling rate. Each block may be checked against low signal, saturated signal, and high ambient light rejection criteria before processing. In some cases, if both emitters have valid analog-to-digital converter (ADC) values, the data from the two emitters may be fused by taking the square root of the element-wise product of the two signals. If only one emitter has valid data that is not saturated or exhibiting low signal, the data from that single emitter may be used. If neither emitter has valid ADC values or if there is excessive ambient light, the block may be rejected and zeros may be reported for that block of data.

The PPG algorithm may implement two different second-order bandpass filters running in parallel. A first bandpass filter with a passband of approximately 1 to 4 Hz (e.g., 1.0, 1.5, 2.0, 2.5, 3.0, 3.5, 4.0) may be used for the reported PPG waveform, while a second bandpass filter with a passband of approximately 0.5 to 4 Hz (e.g., 0.5, 1.0, 1.5, 2.0, 2.5, 3.0, 3.5, 4.0) may be used for internal algorithm processing. The ADC data may be converted from ADC counts to current values in nanoamps before filtering. The filter may comprise a biquad filter structure with multiple stages.

The pulse rate algorithm may receive a filtered epoch as input and may apply a Hanning window to the data to reduce spectral leakage during frequency analysis. The Hanning-processed epoch may be resampled to a specified number of samples to be compatible with Fast Fourier Transform (FFT) processing. In some implementations, the epoch may be resampled to many samples (e.g., 100, 150, 200, 256, 300) samples for FFT compatibility. The epoch may be normalized using a rolling mean and rolling standard deviation to enhance the signal characteristics. The algorithm may then perform FFT on the normalized epoch and use the resulting frequency spectrum to identify peaks in the power spectrum across the frequency band corresponding to physiological pulse rates.

Peak weights may be assigned in the pulse rate algorithm using the current peak magnitude along with the percent change from the previous pulse rate estimate. The pulse rate may be determined by a weighted average of the current peak and two neighboring peaks, with the power spectrum magnitudes serving as weights. This weighted averaging approach may improve the resolution of pulse rate estimates beyond the inherent frequency resolution of the FFT. The calculated pulse rate value may be added to a rolling median buffer containing previous reported pulse rate values. Before reporting, the pulse rate may be clipped to a physiologically plausible range such as 30 to 240 beats per minute (e.g., 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240) to prevent reporting of impossible values.

The $SpO_2$ algorithm may calculate oxygen saturation, PPG ratio, and perfusion index using data from multiple emitters including red emitters operating at approximately 600 to 700 nm (e.g., 600 nm, 601 nm, 602 nm, 603 nm, 604 nm, 605 nm, 606 nm, 607 nm, 608 nm, 609 nm, 610 nm, 611 nm, 612 nm, 613 nm, 614 nm, 615 nm, 616 nm, 617 nm, 618 nm, 619 nm, 620 nm, 621 nm, 622 nm, 623 nm, 624 nm, 625 nm, 626 nm, 627 nm, 628 nm, 629 nm, 630 nm, 631 nm, 632 nm, 633 nm, 634 nm, 635 nm, 636 nm, 637 nm, 638 nm, 639 nm, 640 nm, 641 nm, 642 nm, 643 nm, 644 nm, 645 nm, 646 nm, 647 nm, 648 nm, 649 nm, 650 nm, 651 nm, 652 nm, 653 nm, 654 nm, 655 nm, 656 nm, 657 nm, 658 nm, 659 nm, 660 nm, 661 nm, 662 nm, 663 nm, 664 nm, 665 nm, 666 nm, 667 nm, 668 nm, 669 nm, 670 nm, 671 nm, 672 nm, 673 nm, 674 nm, 675 nm, 676 nm, 677 nm, 678 nm, 679 nm, 680 nm, 681 nm, 682 nm, 683 nm, 684 nm, 685 nm, 686 nm, 687 nm, 688 nm, 689 nm, 690 nm, 691 nm, 692 nm, 693 nm, 694 nm, 695 nm, 696 nm, 697 nm, 698 nm, 699 nm, 700 nm) and infrared emitters operating at approximately 900 to 1,000 nm (e.g., 900 nm, 901 nm, 902 nm, 903 nm, 904 nm, 905 nm, 906 nm, 907 nm, 908 nm, 909 nm, 910 nm, 911 nm, 912 nm, 913 nm, 914 nm, 915 nm, 916 nm, 917 nm, 918 nm, 919 nm, 920 nm, 921 nm, 922 nm, 923 nm, 924 nm, 925 nm, 926 nm, 927 nm, 928 nm, 929 nm, 930 nm, 931 nm, 932 nm, 933 nm, 934 nm, 935 nm, 936 nm, 937 nm, 938 nm, 939 nm, 940 nm, 941 nm, 942 nm, 943 nm, 944 nm, 945 nm, 946 nm, 947 nm, 948 nm, 949 nm, 950 nm, 951 nm, 952 nm, 953 nm, 954 nm, 955 nm, 956 nm, 957 nm, 958 nm, 959 nm, 960 nm, 961 nm, 962 nm, 963 nm, 964 nm, 965 nm, 966 nm, 967 nm, 968 nm, 969 nm, 970 nm, 971 nm, 972 nm, 973 nm, 974 nm, 975 nm, 976 nm, 977 nm, 978 nm, 979 nm, 980 nm, 981 nm, 982 nm, 983 nm, 984 nm, 985 nm, 986 nm, 987 nm, 988 nm, 989 nm, 990 nm, 991 nm, 992 nm, 993 nm, 994 nm, 995 nm, 996 nm, 997 nm, 998 nm, 999 nm, 1,000 nm). The signals may be received by a detector positioned at a short source-detector distance and may be processed with the same filtering and sensor fusion techniques used for the green signal. The $SpO_2$ algorithm may use information from the pulse rate algorithm to select corresponding FFT components from the alternating current (AC) component of the red and infrared signals.

The $SpO_2$ calculation may involve upsampling the filtered red and infrared data to a specified number of samples, calculating the FFT, and computing the magnitudes of the transformed data. Values from the processed data may be selected based on whether they fall within a specified bandwidth of the current pulse rate and the first harmonic frequency. In some implementations, a bandwidth of approximately plus or minus 7 beats per minute around the pulse rate may be used for selecting relevant frequency components. The resulting average magnitudes from this data may be used for calculating a signal quality index (SQI). PPG SQI is defined by the relative power of the FFT in the selected PR range.

A surrogate melanin index may be calculated from the direct current (DC) components of the red and infrared signals and may be used for $SpO_2$ correction based on skin tones. The melanin index may be calculated as the ratio of the red DC component to the infrared DC component. The $SpO_2$ value may be calculated from the PPG ratio and melanin index using a linear model with calibration coefficients. The linear model may take the form: $SpO_2$ = (slope coefficient 1 $\times$ PPG ratio) + (slope coefficient 2 $\times$ melanin index) + intercept. The calibration coefficients may be derived from controlled hypoxia studies comparing device measurements against reference arterial blood oxygen saturation measurements.

If the SQI falls below a threshold value, previously calculated $SpO_2$, PPG ratio, and perfusion index values may be reported again rather than calculating new values from potentially unreliable data. The SQI may be calculated based on the average energy in the red and infrared signals within the pulse rate frequency band. The SQI value may be scaled and clipped before reporting to provide a standardized quality metric.

The perfusion index may be calculated as the ratio of the AC component to the DC component of the infrared signal, multiplied by a scaling factor such as 100 to express the value as a percentage. The PPG ratio may be calculated as the ratio of the red AC/DC ratio to the infrared AC/DC ratio, which forms the basis for the SpO$_2$ calculation.

The algorithms may implement various threshold parameters and timing constants to control signal processing behavior. A sampling rate of approximately 38 Hz (e.g., between 30 to 45 Hz) may be used for waveform acquisition. Each processing block may comprise samples corresponding to one second of data at the sampling rate. An epoch for processing may comprise multiple blocks, such as 10 blocks corresponding to 10 seconds of data. The FFT processing may upsample the epoch data to a predetermined number of samples (e.g., 100, 150, 200, 256, 300 samples) to provide adequate frequency resolution.

Signal quality thresholds may be implemented to reject data that does not meet minimum quality criteria. A low signal threshold may be set at approximately 5 percent of the maximum ADC resolution, while a saturated signal threshold may be set at approximately 99 percent of the maximum ADC resolution. An ambient light saturation threshold may be implemented to reject data when ambient light levels exceed acceptable limits. When bad data is encountered, the algorithm may implement a delay period such as 10 seconds before allowing new samples into the FFT processing to avoid contamination from transient artifacts.

The algorithms may implement rolling median buffers for smoothing reported values. The pulse rate and perfusion index may use rolling median buffers with a size of 5 samples. The PPG ratio may use a rolling median buffer with a larger size such as 15 samples. The use of rolling medians may reduce the impact of outlier values on reported measurements while maintaining responsiveness to genuine physiological changes.

Filter convergence delay may be implemented to allow the bandpass filters sufficient time to reach steady-state operation before beginning epoch processing. A delay period such as 5 seconds may be used at the start of sampling to allow filter transients to settle. During this convergence period, epochs may not be processed for physiological parameter calculation.

The algorithms may implement reset logic to handle extended periods of poor signal quality. Counter variables may track consecutive epochs with low or critical SQI values. If the count of epochs with critical SQI exceeds a threshold such as 10 epochs, the rolling median structures may be reset. If the count of epochs with low SQI exceeds a threshold such as 30 epochs, the rolling median structures may also be reset. These reset mechanisms may help the algorithm recover from extended periods of motion artifact or poor sensor coupling.

In some embodiments, the detailed step-by-step algorithm processing may proceed as follows. When the device starts sampling, an initialization function may be called which initializes multiple dataset structures for storing optical signals from different wavelength emitters. Separate dataset structures may be initialized for, for example, 535 nm, 660 nm, and 940 nm signals from multiple emitters, as well as an ambient dataset structure for storing ambient light data used to determine if the current block of data is saturated. Filter instances may be initialized and stored as fields within each dataset structure, with filter coefficients generated using parameters including a lower threshold of 0.5 Hz, an upper threshold of 4 Hz, an order of 2, and a sampling frequency of 38 Hz. An output structure may be initialized to store all output data including calculated physiological parameters.

After initialization, the device may enter a main processing loop where samples are collected and processed one at a time. When a sample is collected, a vector generation function may be called which initializes static variables including a sample count that stores how many samples have been collected for the current epoch, and a filter count that tracks how many blocks have been filtered. Processing of epochs may not begin until a specified number of blocks have been filtered to allow the filter sufficient time to converge and avoid transients in the output data. Each time the vector generation function is called, the raw dataset lists stored in the dataset structures may be updated to add the new sample.

The sample count may be incremented and checked to determine if a full block of raw samples is ready for filtering. If a full raw block is available, the algorithm may loop through each non-ambient signal structure's raw data and check if it contains low signal or saturated signal, updating a flag indicating bad data if needed. The algorithm may also loop through the ambient signal data and check if any samples exceed an ambient saturation threshold. If any pair of emitter datasets or the ambient dataset contains bad data, rejection flags may be set to true and a counter may be set to delay reporting of PPG data. If the pulse rate rejection flag is true, a count of consecutive rejections may be maintained, and if this count exceeds a threshold, hyperparameters of peak detection may be reset.

If the SpO$_2$ rejection flag is true, a count of consecutive rejections may be maintained, and if this count exceeds a threshold, data reporting rolling structures may be reset. If the SpO$_2$ rejection flag is false, the 660 nm and 940 nm signals may be processed using the same fusing and filtering logic as for the 535 nm signals. If rejection flags were not triggered but a delay counter is greater than zero, the delay counter may be decremented and rejection flags may be set to true. If the pulse rate rejection flag is false, both 535 nm datasets may be converted to current values, the 535 nm signals may be fused by taking the square root of the element-wise product if both signals are good or by selecting whichever signal is good if only one is valid, and the fused signal may be filtered.

If the sample count is greater than or equal to the epoch size, the algorithm may check whether the filter count is less than or equal to a filter convergence delay threshold. If so, the filter has not yet converged, the filter count may be incremented, and the epoch may not be processed. Otherwise, the filter has converged and the epoch may be processed using an epoch processing function. Regardless of whether the epoch is processed, the buffers in each dataset structure may be shifted by one block and the block size may be subtracted from the sample count.

In some embodiments, the pulse rate epoch processing may proceed as follows. If the pulse rate rejection flag is false, a normalization window size may be calculated based on the FFT upsampling factor and block count, multiplied by a factor such as 0.7. A Hanning filter may be applied to the waveform using a Hanning window buffer, and the post-processed waveform may be stored. The Hanning-processed data may then be resampled to the FFT upsampling number of samples to be compatible with FFT processing. Once the epoch has the required number of samples, the epoch may be normalized using an enhancement function that normalizes the filtered data by subtracting the mean and dividing by the standard deviation of the data, implementing rolling z-score normalization. The mean and standard deviation calculations may exclude samples from the beginning and end of the epoch based on the normalization window size. If the rolling standard deviation is less than a threshold value, only the rolling mean may be subtracted from the signal.

The normalized epoch may be sent to an FFT function to calculate the frequency spectrum. The output FFT may be processed by a function that separates the real and imaginary components of the frequency spectrum along with DC and Nyquist frequency components. The averaged FFT data may be sent to a peak detection function that calculates peaks in the FFT using a slope change algorithm. If the number of detected peaks is greater than zero, weights for each peak may be calculated using a peak weight function that takes the average FFT data, detected peaks, and block count as inputs. The peak weights may be calculated using the peak magnitude of each peak and the corresponding percent change in pulse rate assuming the corresponding peak is the actual pulse rate. The percent change may be calculated from the previous epoch's pulse rate and may be saturated to avoid getting stuck at the previous pulse rate by assuming a minimum percent change value when the calculated change is below a threshold.

The pulse rate may be calculated from the maximum peak weight location in the frequency domain. Since the resolution of pulse rate is limited if only the location of maximum peak weight is used, a weighted average method may be employed. Two neighboring peaks, one to the left and one to the right of the maximum peak, along with the frequency at the peak and their corresponding peak weights may be used to calculate pulse rate according to the formula: pulse_rate = 60.0 × ((avg_magnitude[max_peak_1dx-1] × freq_1) + (avg_magnitude[max_peak_idx] × freq_2) + (avg_magnitude [max_peak_idx+1] × freq_3)) / (avg_magnitude[max_peak_idx-1] + avg_magnitude[max_peak_idx] + avg_magnitude [max_peak_idx+1]), where freq_1, freq_2, and freq_3 are the frequencies corresponding to the left neighbor, peak, and right neighbor respectively. The calculated pulse rate value may be added to a rolling median containing previous reported pulse rate values. Before reporting, the pulse rate may be clipped to a range such as 30 to 240 beats per minute to prevent impossibly low or high values.

After calculating pulse rate, if the SpO$_2$ rejection flag is false, the algorithm may resample the filtered red and infrared data to the FFT upsampling number of samples via linear interpolation. The resampled red and infrared data may then be transformed via FFT. The magnitudes of the transformed data may be calculated and stored in processed FFT fields of FFT data structures. The algorithm may loop through the processed FFT data and calculate the energy in the red and infrared signals within the pulse rate band, which includes magnitudes within a specified bandwidth of the current pulse rate and magnitudes within the first harmonic band. The pulse rate band may include frequency values satisfying: freq[i] > (pulse_rate - PR_BAND_HALF_WIDTH)/60.0 and freq[i] < (pulse rate + PR_BAND _HALF_WIDTH)/60.0. The first harmonic band may include frequency values satisfying: freq[i] > (2 × pulse rate - PR_BAND _HALF_WIDTH)/60.0 and freq[i] < (2 × pulse rate + PR_BAND_HALF_WIDTH)/60.0. If the most recently calculated pulse rate value is outside a valid range, a value of zero may be used for these bounds instead.

The average of the energy values may be stored in average magnitude fields of the FFT data structures. These values may also be used to calculate SpO$_2$ SQI using the formula: SpO2_SQI = min(avg_red_energy × 20, avg_ired_energy × 20), where the averages are zero if the total energy is zero. When SpO$_2$ SQI is reported to the output packet, it may be clipped and rounded using: SpO2_SQI_reported = ceil(clip(SpO2_SQI, 0, 10)) / 2.

The red signal, infrared signal, and SpO$_2$ SQI value may be sent to an SpO$_2$ calculation function to calculate SpO$_2$, PPG ratio, and perfusion index. The SpO$_2$ algorithm may use the same logic as the pulse rate algorithm regarding resetting rolling median structures and hyperparameters. The function may use global counter variables to track whether recent SQI values have been too low. If the combined SQI is less than or equal to a critical threshold, both low and critical SQI counters may be incremented. If the critical SQI counter exceeds a threshold, the rolling median structures may be reset. If either counter exceeds a parameter reset threshold, the pulse rate hyperparameters may be reset. If the combined SQI is less than or equal to a low threshold but above the critical threshold, the low SQI counter may be incremented and the critical SQI counter may be reset. If the low SQI counter exceeds a rolling reset threshold, the rolling median structures may be reset. If the low SQI counter exceeds a parameter reset threshold, the SpO$_2$ hyperparameters may be reset.

If all quality checks are passed, SpO$_2$, PPG ratio, and perfusion index may be calculated as follows. Red DC RMS and infrared DC RMS may be calculated by taking the average of the raw red and infrared data respectively. Red AC RMS and infrared AC RMS may be set to the average magnitudes of the processed FFT data. PPG ratio may be calculated as: PPG_ratio = (red_ac_rms / red_dc_rms)/ (ir_ac_rms/ ir_dc_rms). Perfusion index may be calculated as: PI = (ir_ac_rms / ir_dc_rms) × 100. A correction factor for melanin may be calculated using a surrogate melanin index: MI = (red_dc_rms / ir_dc_rms). SpO$_2$ may then be calculated as: SpO2 = SPO2_SLOPE_1 × PPG_ratio + SPO2_SLOPE_2 × MI + SPO2_INTERCEPT. The PPG ratio, perfusion index, and $SpO_2$ values may be added to their respective rolling average or median structures or buffers. PPG ratio and perfusion index may use rolling medians to calculate reported values and may report every second. $SpO_2$ may not use a rolling average or median and may also report every second. The final step of the vector generation function may be to send the enhanced green signal to the output PPG buffer if there is data in the current epoch to send.

The algorithms may utilize various macro definitions to control processing parameters. The $SpO_2$ linear model coefficients may include SPO2_SLOPE_1 with a value of approximately -23.2, SPO2_SLOPE_2 with a value of approximately 10.97, and SPO2_INTERCEPT with a value of approximately 117.58, which may be calibrated using arterial blood data from controlled hypoxia studies. The target sampling rate may be defined as 38 Hz, with the actual sampling rate also set to 38 Hz. The block size may be defined as 38 samples, corresponding to one second of data at the sampling rate. The number of filter stages may be defined as 2 for the biquad filter structure. The number of blocks per epoch may be defined as 10, resulting in an epoch size of 380 samples corresponding to 10 seconds of data.

The FFT upsampling factor may be defined as 256 samples, which determines the number of samples to which the epoch is resampled for FFT processing. The processed FFT length may be defined as 129, which includes the DC and Nyquist frequencies and corresponds to FFT_UPSAMPLING/2 + 1. The maximum number of peaks may be defined as 128, corresponding to FFT_UPSAMPLING/2. The pulse rate band half width may be defined as 7 beats per minute, which determines the bandwidth used for $SpO_2$ SQI calculation. The default window size for rolling standard deviation may be defined as 18, calculated as (FFT_UPSAMPLING / BLOCKS_PER_EPOCH) $\times$ 0.7.

The rolling median buffer sizes may be defined as follows: the pulse rate rolling median buffer size may be 5 samples, the perfusion index rolling median buffer size may be 5 samples, and the PPG ratio rolling median buffer size may be 15 samples. Signal quality thresholds may include a green SQI low threshold and green SQI critical threshold, which may be disabled by setting to -1. The red SQI low threshold may be defined as 6, and the red SQI critical threshold may be defined as 2. The bad epoch reset threshold may be defined as 5 epochs, which determines how long to wait before resetting rolling data and peak hyperparameters. The critical SQI rolling reset threshold may be defined as 10 epochs, and the low SQI rolling reset threshold may be defined as 30 epochs.

The number of samples before reporting may be defined as 1, which determines how many samples to add to pulse rate, $SpO_2$, and perfusion index structures before reporting values. The ADC resolution may be defined as 16384, representing the maximum ADC value. The low signal threshold may be defined as 0.05 $\times$ 16384, corresponding to 5 percent of the maximum ADC resolution. The saturated signal threshold may be defined as 0.99 $\times$ 16384, corresponding to 99 percent of the maximum ADC resolution. The ambient saturation threshold may be defined as 200 ADC counts. The PPG current scaling factor may be defined as 1000000, which converts from milliamps to nanoamps. The standard deviation threshold may be defined as 0.01, representing the minimum allowed rolling standard deviation value. The filter convergence delay may be defined as 5 seconds, which determines how long to wait at the start of sampling for the filter to converge before beginning processing. The wait period after bad data may be defined as 10 seconds, which determines how long to wait before allowing data into the FFT after encountering bad data.

### NIRS Algorithms

In some embodiments, the NIRS monitoring wearable system may implement tissue oxygenation ($StO_2$), total hemoglobin index (tHb), and Oxygen Extraction Ratio (OER) algorithms using near-infrared spectroscopy techniques to calculate oxygen saturation levels in biological tissue. The $StO_2$ algorithm may use ambient-corrected data from two transimpedance amplifier (TIA) emitters operating at wavelengths of approximately 735 nm (e.g., 700 nm, 705 nm, 710 nm, 715 nm, 720 nm, 725 nm, 730 nm, 735 nm, 735 nm, 740 nm, 745 nm, 750 nm, 755 nm, 760 nm) and approximately 850 nm (e.g., 800 nm, 805 nm, 810 nm, 815 nm, 820 nm, 825 nm, 830 nm, 835 nm, 840 nm, 845 nm, 850 nm, 855 nm, 860 nm, 865 nm, 870 nm, 875 nm), and two detectors positioned at source-detector distances of approximately 35 mm (e.g., 30 mm, 30.5 mm, 31.0 mm, 31.5 mm, 32.0 mm, 32.5 mm, 33.0 mm, 33.5 mm, 34.0 mm, 34.5 mm, 35.0 mm, 35.5 mm, 36.0 mm, 36.5 mm, 37 mm) and approximately 40 mm (e.g., 37.5 mm, 38.0 mm, 38.5 mm, 39.0 mm, 39.5 mm, 40.0 mm, 40.5 mm, 41.0 mm, 41.5 mm, 42.0 mm, 42.5 mm, 43.0 mm, 43.5 mm, 44.0 mm, 44.5 mm, 45.0 mm). The algorithm may process optical signals to determine the relative concentrations of oxygenated hemoglobin and deoxygenated hemoglobin in the measured tissue volume.

When the device starts sampling, a configuration function may be called to set which emitters and detectors to use and to initialize data structures used to contain algorithm data. Filter structures may be created for storing lowpass filter instances for each signal channel. Dataset structures may be created for storing raw programmable gain amplifier (PGA), TIA, and ambient analog-to-digital converter (ADC) data for each detector. Buffer structures may be created to avoid data from being overwritten in the main dataset structures during processing. A processed data structure may be created to store filtered TIA and PGA currents and current ratios, where the PGA currents and current ratios may be used for detecting bad data in the epoch.

After completing autocalibration, the device may call an initialization function to initialize a calibration values structure. This structure may contain TIA, feedback, voltage bias, and series values for each detector, centroid values for each wavelength and hardware revision, oxygenated hemoglobin (HbO) and deoxygenated hemoglobin (dHb) extinction coefficients for each wavelength, reference voltage values, and timestamp values. The calibration values may be obtained from non-volatile memory (NVM) storage on the device.

After the initial sampling and calibration steps, the device may enter a main processing loop where samples are collected and processed one at a time. When the device collects a sample, a vector generation function may be called that modifies multiple global variables. A sample count variable may store how many samples are in the current epoch, and after an epoch is finished processing, this counter may decrease by the block size due to the oldest block of data being removed from the epoch. Processing buffer count and block buffer count variables may be used to count how many samples are in the processing buffer and the backup buffer respectively. If the algorithm is still processing an epoch while there are new samples to add to buffers, the block buffer count may be incremented; otherwise, the processing buffer count may be incremented.

A bad signal count variable may be used for keeping track of how many of the preceding epochs were rejected, and this number may reset to zero when a good epoch is processed. When a block of data is encountered that contains bad data, a rejection flag may be set to true to signal this condition, and a blocks until report variable may be set to a wait period value to keep track of how long the algorithm should wait before calculating $StO_2$ again.

Every time the vector generation function is called, each of the appropriate raw ADC data buffers in the dataset structures may be updated with the new data sample. The following values may be collected: TIA signals at 735 nm for 35 mm and 40 mm detectors, TIA signals at 850 nm for 35 mm and 40 mm detectors, PGA signals at 735 nm for 35 mm and 40 mm detectors, PGA signals at 850 nm for 35 mm and 40 mm detectors, and ambient TIA signals for 35 mm and 40 mm detectors.

When a full block of data is collected, meaning the sample count is a multiple of the block size, that block may be checked to determine if there are any issues with the signal. The blocks may be converted from ADC values to current values. The PGA values may be converted to currents without any ambient correction. The TIA values may be ambient-corrected by subtracting the corresponding ambient ADC values before conversion. After the TIA currents are calculated, TIA current ratios may be calculated according to the formulas: wl_i_tia_ratio = w1_d2_i_tia / wl_dl_i_tia (or zero if division by zero would occur), and w2_i_tia_ratio = w2_d2_i_tia / w2_dl_i_tia (or zero if division by zero would occur).

Before the currents are filtered, several conditions may be verified. The TIA ADC values may be required to be between lower and upper bounds for each wavelength and detector combination. The ambient TIA ADC values may be required to be below upper threshold values for each detector. The TIA current ratios may be required to be between lower and upper ratio bounds, such as between 0.1 and 0.7.

If any of these conditions fails, the data for the current block may be set to zero instead of being filtered, the blocks until report variable may be set to the wait period value meaning that $StO_2$ will not be calculated for the specified number of seconds, and the bad signal count variable may be incremented. If the bad signal count exceeds a threshold such as 5 consecutive bad epochs, an $StO_2$ value of zero may be reported; otherwise, the previous $StO_2$ value may be reported. If the TIA current ratio condition failed, a sensor decoupled fault may be reported. If the ambient light condition failed, a high ambient fault may be reported.

If all of the conditions are satisfied, the bad signal count may be reset to zero, the blocks until report variable may be decremented, and the most recent block may be filtered using a lowpass filter with a cutoff frequency of approximately 0.5 Hz.

Once a full epoch of data has been collected, if the blocks until report variable equals zero (meaning it has been at least the wait period since a block containing bad data was encountered), then NIRS signal strength index (SSI) and $StO_2$ may be calculated. The epoch size may be defined as 5 blocks corresponding to 5 seconds of data, with epochs having a 4-block overlap meaning a new epoch is processed every second.

The NIRS SSI may be calculated using average ambient-corrected TIA ADC data. These averages may be calculated for each emitter-detector pair by calculating the difference between each TIA ADC and ambient TIA value in the epoch and taking the average. There may be two emitters and two detectors used for the $StO_2$ algorithm, resulting in four averages to calculate. The lowest value of these four averages may be selected and NIRS SSI may be calculated using threshold bounds: NIRS_SSI may equal 1 if the average is less than 25, may equal 2 if the average is between 25 and 40, may equal 3 if the average is between 40 and 55, may equal 4 if the average is between 55 and 80, and may equal 5 if the average is greater than 80. A NIRS SSI value of 5 may represent very good signal quality, while an SSI value of 1 may represent the lowest quality and may not be sufficient for accurate calculations.

If the resulting NIRS SSI value is below a threshold, $StO_2$ may not be calculated, the bad signal count may be incremented, a low SSI fault may be reported, and either the previous $StO_2$ may be returned or zero may be reported depending on whether the bad signal count is below the threshold.

If the resulting NIRS SSI is at least as large as the threshold, $StO_2$ may be calculated using the averages of each of the TIA current values. The change in attenuation for each wavelength ma be calculated using the formula:

$$\frac{\partial A}{\partial d}(\lambda) = \frac{I_{d_2}(\lambda) - I_{d_1}(\lambda)}{d_2 - d_1}$$ , where I represents the current at each detector and d represents the source-detector distance.

The absorption coefficient term $k\mu_a$ may be calculated for both wavelengths using the formula:

$$k\mu_a(\lambda) = \frac{1}{3(1 - 6.3 \times 10^{-6} \times w_{centroid})} \left( \log(10)\frac{\partial A}{\partial d} - \frac{4}{d_1 + d_2} \right)^2$$ , where the centroid value may be obtained from the NVM using a coefficient retrieval function. The constant $6.3 \times 10^{-6}$ may represent the slope of the scattering coefficient.

The denominator for the hemoglobin concentration calculations may be calculated using the formula:

$$denom = \frac{1}{dHb_{\lambda_2} \times HbO_{\lambda_1} - dHb_{\lambda_1} \times HbO_{\lambda_2}}$$ , where dHb and HbO represent the extinction coefficients for deoxygenated hemoglobin and oxygenated hemoglobin at each wavelength respectively. If the denominator is zero, the epoch may be rejected and the previous $StO_2$ value may be returned.

Otherwise, the scaled hemoglobin concentrations kHbO and kdHb may be calculated using the formulas: *kHbO =* *(dHb$_{\lambda_2}$ × kμ$_{a,\lambda_2}$ - dHb$_{\lambda_1}$ × kμ$_{a,\lambda_1}$)/denom* and *kdHb = (HbO$_{\lambda_1}$ × kμ$_{a,\lambda_2}$ - HbO$_{\lambda_2}$ × kμ$_{a\lambda_1}$)/denom.*

The denominator of the $StO_2$ formula may be calculated as (kHbO + kdHb). If this denominator is zero, the epoch may be rejected and the previous $StO_2$ value may be returned. Otherwise, $StO_2$ may be calculated using the formula:

$$StO_2 = 100 \times \frac{kHbO}{kHbO + kdHb}$$ .

The calculated $StO_2$ value may be scaled using a linear transformation with calibration coefficients. The linear scaling may take the form: Scaled $StO_2$ = slope × $StO_2$ + intercept, where the slope may be approximately 1.8 and the intercept may be approximately -49.8. These coefficients may be derived from calibration studies comparing device measurements against reference blood oxygen saturation values calculated from venous and arterial blood samples using a venous to arterial ratio such as 70:30.

The calculated $StO_2$ value may then be rounded and clipped to the range of 0 to 100 percent before being saved to the output data packet.

The NIRS $StO_2$ algorithm may utilize various macro definitions to control processing parameters. The target sampling rate may be defined as 38 Hz. The number of blocks per epoch may be defined as 5, corresponding to 5 seconds of data. The linear fit slope may be defined as 1.8 and the linear fit intercept may be defined as -49.8. The scattering coefficient slope constant may be defined as $6.3 \times 10^{-6}$. The ADC resolution may be defined as 16384, representing the maximum ADC value for a 14-bit converter.

Signal quality thresholds may include low signal thresholds for TIA at 35 mm and 40 mm detectors, which may be set to 5 ADC counts. Saturated signal thresholds for TIA at 35 mm and 40 mm detectors may be set to 90 percent of the maximum ADC resolution. The TIA ratio lower bound may be defined as 0.1 and the TIA ratio upper bound may be defined as 0.7. Ambient light saturation thresholds for 35 mm and 40 mm detectors may be set to 90 percent of the maximum ADC resolution. The NIRS SSI threshold may be defined as 1, representing the minimum acceptable signal quality for $StO_2$ calculation. The bad epoch threshold may be defined as 5, representing the number of consecutive bad epochs to allow before returning an $StO_2$ value of zero. The current scaling factor may be defined as 1000000, which converts from milliamps to nanoamps. The wait period after bad data may be defined as 2 epochs, which determines how long to wait before reporting $StO_2$ again after encountering bad data.

The NIRS algorithm may be validated using controlled hypoxia studies with venous and arterial blood reference measurements analyzed using co-oximetry techniques. The global field saturation (fSO$_2$) reference may be calculated using the formula: *fSO$_2$ = a × SjvO$_2$ + b × SaO$_2$,* where SjvO$_2$ represents jugular bulb venous saturation, SaO$_2$ represents arterial blood saturation, and the coefficients a and b may be approximately 0.70 and 0.30 respectively representing the venous to arterial blood ratio in the tissue. The calibration may achieve correlation coefficients of approximately 0.87 against reference blood measurements and approximately 0.89 against predicate devices, with root mean square error values of approximately 4.2 percent against blood reference and approximately 3.7 percent against predicate devices.

*Placement of the NIRS System on a User*

As particularly shown in FIG. 9, the NIRS system may be placed on multiple body locations for comprehensive physiological monitoring depending on the clinical application and the tissue compartments of interest. The body locations for sensor placement may include, for example, the forearm wrist extensor group, the sternum, and the dominant thigh, with each location providing access to different tissue types and physiological information. The selection of sensor placement location may be based on the specific monitoring objectives, the accessibility of the tissue site, and the need to minimize interference with other clinical activities or patient comfort during extended monitoring periods.

The NIRS system may be placed on the dominant arm's forearm wrist extensor group for monitoring upper limb tissue oxygenation. The forearm wrist extensor group may comprise the extensor carpi radialis muscle and surrounding tissues located on the posterior aspect of the forearm. The extensor carpi radialis muscle may provide a suitable tissue site for NIRS monitoring due to the presence of skeletal muscle tissue at accessible depths below the skin surface. Sensor placement on the forearm wrist extensor group may allow monitoring of peripheral tissue oxygenation and microvascular function in the upper extremity, which may be relevant for assessing cardiovascular responses to activity, vascular occlusion testing, and other clinical assessments.

The forearm placement location may be determined based on self-reported arm dominance by the user or patient. The dominant arm may be selected for sensor placement to provide consistency across monitoring sessions and to facilitate comparison of measurements between different subjects or time points. The forearm wrist extensor group may be located by palpating the muscle belly on the posterior aspect of the forearm, approximately one-third of the distance from the elbow to the wrist. The sensor may be positioned over the muscle belly with the optical windows aligned to illuminate the underlying muscle tissue.

The NIRS system may be placed on the sternum oriented vertically for monitoring central tissue oxygenation. The sternum may provide a sensor placement location that is relatively stable during physical activity and that provides access to tissue overlying the thoracic cavity. The vertical orientation of the sensor on the sternum may align the source-detector axis along the length of the sternum, with the sensor positioned toward the bottom of the sternum to avoid interference with the manubrium and clavicular structures. The sternal placement may allow monitoring of tissue oxygenation in the chest wall tissues, which may reflect central cardiovascular function and may be relevant for assessing responses to postural changes, physical activity, and other physiological stressors.

The sternal sensor placement may provide tissue oxygenation measurements that complement measurements obtained from peripheral sites such as the forearm or thigh. The central location of the sternum may result in tissue oxygenation measurements that are less affected by peripheral vasoconstriction or local tissue factors that may influence measurements at extremity sites. The sternal placement may be particularly relevant for monitoring applications where central tissue oxygenation is of clinical interest, such as assessment of cardiac output effects on tissue perfusion or monitoring of responses to interventions that affect central hemodynamics.

The NIRS system may be placed on the dominant thigh for monitoring lower limb tissue oxygenation. The dominant thigh placement may target the vastus lateralis muscle, which is a large skeletal muscle located on the lateral aspect of the thigh. The vastus lateralis muscle may provide a suitable tissue site for NIRS monitoring due to the substantial muscle mass at accessible depths below the skin surface. Sensor placement on the vastus lateralis may allow monitoring of lower extremity tissue oxygenation and microvascular function, which may be relevant for assessing cardiovascular responses to physical activity such as walking tests, postural changes, and other clinical assessments involving lower limb muscle activity.

The thigh sensor placement may be positioned at the center front of the thigh on the right leg, with the sensor oriented to illuminate the vastus lateralis muscle tissue. The thigh placement location may be determined based on self-reported leg dominance by the user or patient, with the dominant leg selected for sensor placement to provide consistency across monitoring sessions. The vastus lateralis muscle may be located by palpating the lateral aspect of the thigh, approximately midway between the hip and knee joints. The sensor may be positioned over the muscle belly with the optical windows aligned to illuminate the underlying muscle tissue at the target depths.

The thigh placement location may be selected for emergency room applications involving monitoring of patients with massive hemorrhage. The thigh placement may minimize interference with medical staff during resuscitation procedures by positioning the sensor on a body location that is typically accessible and does not obstruct access to the chest, abdomen, or upper extremities where life-saving interventions may be performed. The application of the NIRS monitoring wearable system to the patient's thigh upon admittance to the emergency room may provide the least delay and minimal interference to the medical staff treating and resuscitating the patient while enabling continuous monitoring of tissue oxygenation and perfusion during the resuscitation process.

The thigh placement for hemorrhage monitoring may allow collection of peripheral tissue oxygenation data that reflects the body's response to blood loss and the effectiveness of resuscitation interventions. The tissue oxygenation measurements from the thigh may trend with mean arterial pressure during hypovolemic hemorrhage, providing continuous assessment of the efficacy of medical interventions in patients for whom continuous blood pressure monitoring may be unavailable or impractical. The thigh placement may also allow monitoring of tissue perfusion recovery as blood volume is restored through fluid administration or blood transfusion, providing real-time feedback about the adequacy of resuscitation efforts.

The NIRS monitoring wearable system may be placed on the forehead for monitoring cerebral or frontal tissue oxygenation. The forehead placement may be positioned above the eyebrow rather than at the center of the forehead to optimize optical coupling and signal quality. The area above the eyebrow may provide a relatively flat surface with consistent tissue thickness that is suitable for NIRS measurements. The forehead placement may allow monitoring of tissue oxygenation in the frontal region, which may be relevant for applications involving assessment of cerebral perfusion or oxygenation status.

The forehead placement above the eyebrow may avoid the frontal sinus region located at the center of the forehead, where air-filled cavities may interfere with optical measurements. The frontal sinus may create optical heterogeneities that affect light propagation through the tissue and may reduce the accuracy of tissue oxygenation measurements. By positioning the sensor above the eyebrow and lateral to the midline, the optical path may traverse more homogeneous tissue structures that provide more reliable NIRS measurements.

The forehead sensor placement may be used with a black skull cap for ambient light rejection. The black skull cap may be worn over the sensor to block ambient light from reaching the optical detectors through gaps between the sensor and the skin surface or through the sensor housing. Ambient light interference may degrade signal quality and measurement accuracy by introducing optical signals that are not related to the tissue being measured. The black skull cap may provide a light-blocking barrier that attenuates ambient light reaching the sensor, improving the signal-to-noise ratio of the optical measurements.

The multi-site sensor placement capability of the NIRS system may allow simultaneous monitoring of tissue oxygenation at multiple body locations using multiple sensor units. In some monitoring configurations, three sensors may be adhered to each subject, with one sensor on the dominant arm's forearm wrist extensor group, one sensor oriented vertically on the sternum, and one sensor oriented vertically on the dominant thigh. The simultaneous multi-site monitoring may provide comprehensive assessment of tissue oxygenation across different body regions, allowing comparison of central and peripheral tissue oxygenation responses and detection of regional differences in tissue perfusion.

The multi-site sensor placement may be used for clinical assessments that evaluate tissue oxygenation responses at different body locations during standardized interventions. The vascular occlusion testing protocol may use the forearm sensor to monitor tissue oxygenation changes during cuff inflation and deflation on the upper arm. The six-minute walk test protocol may use the thigh and sternum sensors to monitor tissue oxygenation changes during physical activity. The postural reactivity protocol may use sensors at all three locations to monitor tissue oxygenation changes during transitions between supine and standing positions. The multi-site measurements may provide complementary information about cardiovascular function and tissue perfusion that is not available from single-site monitoring alone.

The sensor placement locations may be selected based on the anatomical characteristics of each body site and the tissue structures accessible at each location. For example, the forearm placement may provide access to skeletal muscle tissue at depths of approximately 5 mm to 15 mm below the skin surface, depending on subcutaneous fat thickness and muscle development. The sternal placement may provide access to chest wall tissues including muscle, connective tissue, and bone marrow at varying depths. The thigh placement may provide access to the large vastus lateralis muscle at depths that may extend to 20 mm or more below the skin surface in subjects with moderate subcutaneous fat thickness. The forehead placement may provide access to frontal tissues including skin, subcutaneous tissue, and underlying structures at relatively shallow depths compared to the extremity and trunk locations.

The sensor orientation at each placement location may be configured to optimize optical coupling and measurement accuracy for the specific anatomical characteristics of each body site. The vertical orientation on the sternum may align the source-detector axis along the relatively flat surface of the sternum, minimizing curvature effects on optical coupling. The orientation on the forearm and thigh may be adjusted based on the local surface curvature and muscle fiber orientation to optimize illumination of the target tissue volume. The flexible hinge connecting the rigid components of the NIRS monitoring wearable system may allow the sensor to conform to the local surface curvature at each placement location while maintaining optical alignment between the light sources and detectors.

*Biocompatible Adhesive*

The NIRS monitoring wearable system 400 may be used with a biocompatible adhesive configured for robust device attachment to the user's skin in various circumstances. The biocompatible adhesive may comprise a 3M combination adhesive that provides both initial tack for easy application and sustained adhesion for extended monitoring periods. The combination adhesive may be configured to maintain secure attachment during physical activity, perspiration, postural changes, and other conditions that could compromise adhesion while allowing removal without causing skin damage or excessive discomfort. The biocompatible adhesive may comply with relevant biocompatibility standards for materials intended for prolonged skin contact, reducing the risk of skin irritation, allergic reactions, or other adverse effects during extended wear periods.

The adhesive may include optical cutouts configured to ensure proper alignment with the optical windows of the NIRS system. The optical cutouts may be positioned to correspond with the locations of the light sources and detectors on the sensor, preventing the adhesive material from covering or partially obstructing the optical apertures during measurement. The optical cutouts may be sized and shaped to provide clearance around the optical windows while maintaining sufficient adhesive contact area for secure device attachment. Proper alignment of the optical cutouts with the optical windows may help maintain consistent optical coupling between the sensor and the tissue surface, which may improve measurement accuracy and repeatability by ensuring that light can pass freely between the sensor components and the tissue being measured.

The adhesive may comprise a patch-side surface and a skin-side surface, with each surface protected by a removable liner prior to application. The patch-side surface may be configured for attachment to the silicone surface of the NIRS system, while the skin-side surface may be configured for attachment to the user's skin at the region of interest. The patch-side liner may be identified by text labeling and may include a tab for easy removal during the application process. The skin-side liner may be transparent and may also include a tab for removal. The two-liner configuration may allow sequential application of the adhesive, first to the sensor and then to the skin, facilitating proper alignment and secure attachment.

The application of the adhesive to the NIRS system may involve removing the patch-side liner and overlaying the exposed adhesive onto the silicone side of the sensor. The optical cutouts in the adhesive may be aligned with the optical windows of the sensor to ensure that the optical apertures are not covered by adhesive material. The adhesive may be pressed firmly onto the sensor surface to ensure uniform contact and secure attachment, with pressure applied using fingers while avoiding folds or air bubbles that could compromise adhesion. The skin-side liner may remain in place during this step to protect the skin-side adhesive surface until the sensor is ready to be applied to the user's body.

The application of the sensor to the user's skin may involve removing the skin-side liner and placing the sensor on the prepared skin surface at the intended measurement location. The sensor may be pressed firmly onto the skin to ensure uniform contact between the adhesive and the skin surface.

The adhesive may be configured as a single-use component that is discarded after each monitoring session, while the NIRS system may be configured as a reusable component that can be cleaned and used with new adhesive for subsequent monitoring sessions. The single-use adhesive configuration may ensure consistent adhesion quality for each monitoring session and may support hygiene requirements by preventing reuse of adhesive that has contacted a previous user's skin. The reusable sensor configuration may reduce the cost and environmental impact of the monitoring system by allowing the electronic and optical components to be used for multiple monitoring sessions with different users.

*Applications of the NIRS System*

The NIRS monitoring wearable system 400 may be used in a variety of end use applications, including patient monitoring and diagnostic evaluation in, for example, peripheral arterial disease (PAD), acute limb ischemia (ALI) detection, reperfusion, cardiovascular issues, hemorrhage, chemotherapeutic agents, etc. It should be understood that the following portion of the description provides only examples of the types of patient monitoring that may be conducted using the NIRS systems disclosed herein (e.g., system 400), and as such, any of the disclosed functions of the NIRS system may be used across any end use application.

The NIRS monitoring wearable system 400 may be used for vascular occlusion testing (VOT) to assess microvascular function and tissue oxygenation kinetics during ischemia and reperfusion. Vascular occlusion testing may involve temporarily occluding blood flow to a limb using a pressure cuff and monitoring the resulting changes in tissue oxygenation as the tissue becomes ischemic during occlusion and then reperfuses following release of the occlusion. The tissue oxygenation kinetics observed during VOT may provide quantitative measures of microvascular function that correlate with cardiovascular health status and disease severity.

The vascular occlusion testing protocol may use a sphygmomanometer inflated to 50 mmHg above the subject's systolic pressure to achieve complete arterial occlusion of the limb being tested. The sphygmomanometer may be positioned on the upper arm proximal to the NIRS sensor placement location on the forearm wrist extensor group. The inflation pressure of 50 mmHg above systolic pressure may ensure complete cessation of arterial blood flow to the distal tissues, creating an ischemic condition that allows assessment of tissue oxygen consumption and microvascular reserve. Prior to performing the vascular occlusion test, the subject's blood pressure may be measured using the sphygmomanometer and recorded to establish the target inflation pressure for the occlusion.

The vascular occlusion test may maintain cuff inflation until $StO_2$ decreases to 40 percent or a maximum of 3 minutes, whichever occurs first. The 40 percent $StO_2$ threshold may represent a level of tissue deoxygenation that provides adequate assessment of deoxygenation kinetics while avoiding excessive ischemic stress to the tissue. The 3-minute maximum duration may serve as a safety limit that prevents prolonged ischemia in subjects whose tissue oxygenation does not decrease to the 40 percent threshold within a reasonable time period. The termination criteria of either reaching 40 percent $StO_2$ or 3 minutes of occlusion may provide standardized test conditions that allow comparison of results across different subjects and monitoring sessions.

During the vascular occlusion test, the NIRS monitoring wearable system may continuously monitor tissue oxygenation in the forearm wrist extensor muscle distal to the occlusion site. As the cuff is inflated and arterial blood flow is occluded, the tissue oxygenation may begin to decrease as the tissue continues to consume oxygen from the blood present in the microvascular bed without replenishment from arterial inflow. The rate of tissue oxygenation decrease during the occlusion period may reflect the metabolic oxygen consumption rate of the tissue and the initial oxygen reserve in the microvascular blood pool.

Following the occlusion period, the sphygmomanometer may be rapidly deflated to restore blood flow to the distal tissues. The rapid deflation may allow immediate reperfusion of the ischemic tissue, triggering a reactive hyperemia response where blood flow temporarily increases above baseline levels to restore tissue oxygen stores. The tissue oxygenation may increase rapidly following cuff release as oxygenated arterial blood flows into the previously ischemic tissue and replaces the deoxygenated blood in the microvascular bed. The rate of tissue oxygenation increase during the reperfusion period may reflect the capacity of the microvascular system to deliver oxygenated blood to the tissue and the integrity of the reactive hyperemia response.

The NIRS monitoring wearable system may extract deoxygenation and reoxygenation slopes from the tissue oximetry signals recorded during the vascular occlusion test. The deoxygenation slope may be calculated from the rate of tissue oxygenation decrease during the cuff inflation period, representing the speed at which the tissue becomes deoxygenated when arterial blood flow is occluded. The reoxygenation slope may be calculated from the rate of tissue oxygenation increase during the reperfusion period following cuff release, representing the speed at which the tissue recovers to baseline oxygenation levels when blood flow is restored.

The extraction of deoxygenation and reoxygenation slopes may involve identifying the time periods in the tissue oximetry signal that correspond to the deoxygenation phase and the reoxygenation phase of the vascular occlusion test. The deoxygenation phase may begin when the cuff is inflated and tissue oxygenation begins to decrease, and may continue until the cuff is released or the termination criteria are met. The reoxygenation phase may begin when the cuff is released and tissue oxygenation begins to increase, and may continue until tissue oxygenation returns to baseline levels or stabilizes at a new steady-state value.

The slope calculations may involve applying linear regression or other curve-fitting techniques to the tissue oximetry data during the identified deoxygenation and reoxygenation time periods. The deoxygenation slope may be expressed as the change in tissue oxygenation percentage per unit time (such as percent per second or percent per minute) during the occlusion period. The reoxygenation slope may be expressed in the same units, representing the rate of tissue oxygenation recovery during the reperfusion period. The slope values may be calculated automatically by the analysis engine of the NIRS monitoring wearable system or may be calculated during post-processing analysis of the recorded tissue oximetry data.

The vascular occlusion testing protocol may be performed twice on each subject to establish an accurate average of the measured parameters. The second vascular occlusion test may be performed after tissue oxygenation has returned to baseline for at least 3 minutes following the first test. The 3-minute recovery period may allow the tissue to return to a stable baseline state before the second test, ensuring that the second measurement is not affected by residual effects from the first occlusion. The averaging of results from two vascular occlusion tests may reduce measurement variability and improve the reliability of the extracted slope parameters.

The times of the blood pressure measurements and vascular occlusion test events may be recorded in a case report form along with the subject's recorded blood pressure values. The recorded timing information may allow synchronization of the vascular occlusion test events with the tissue oximetry data during subsequent analysis. Blood pressure measurements may be performed before each vascular occlusion test to establish the appropriate cuff inflation pressure for each test. Additional physiological parameters including pulse oximetry ($SpO_2$) and pulse rate may be recorded before and after the vascular occlusion test to provide context for the tissue oxygenation measurements.

The reperfusion slopes measured during vascular occlusion testing may correlate with heart failure severity, where lower reperfusion slopes indicate more severe heart failure classification. Subjects with more severe heart failure may exhibit slower reoxygenation following cuff pressure release, reflecting impaired microvascular function and reduced capacity for reactive hyperemia. The impaired microvascular function in heart failure may result from endothelial dysfunction, reduced capillary density, or other pathophysiological changes that limit the ability of the microvascular system to rapidly deliver oxygenated blood to ischemic tissue.

The correlation between reperfusion slopes and heart failure severity may be assessed by comparing the measured slope values across subjects classified according to the New York Heart Association (NYHA) functional classification system. The NYHA classification system categorizes heart failure patients into four classes (I through IV) based on the severity of symptoms and functional limitations. Subjects with NYHA Class I heart failure may exhibit the highest reperfusion slopes, indicating relatively preserved microvascular function. Subjects with NYHA Class II and Class III heart failure may exhibit progressively lower reperfusion slopes, indicating increasingly impaired microvascular function with more severe disease.

The mean reperfusion slopes observed during vascular occlusion testing may differ between NYHA classes, with the differences potentially reaching statistical significance when adequate sample sizes are evaluated. The reperfusion slope measurements may provide a quantitative biomarker of microvascular function that complements the subjective symptom-based NYHA classification. The tissue oximetry-based assessment of microvascular function during vascular occlusion testing may provide objective physiological data that can be used to characterize heart failure patients and potentially to monitor disease progression or response to treatment over time.

The deoxygenation slopes measured during the occlusion period may also provide information about tissue metabolic function and oxygen consumption rates. The rate of tissue deoxygenation during arterial occlusion may reflect the balance between tissue oxygen consumption and the initial oxygen reserve in the microvascular blood pool. Subjects with higher metabolic rates or lower initial tissue oxygenation may exhibit faster deoxygenation during occlusion. The deoxygenation slope measurements may complement the reperfusion slope measurements in characterizing the overall tissue oxygenation kinetics during vascular occlusion testing.

The vascular occlusion testing protocol using the NIRS monitoring wearable system may provide a standardized assessment of microvascular function that can be performed in clinical settings with minimal equipment requirements. The use of a standard aneroid sphygmomanometer for vascular occlusion may allow the test to be performed using equipment commonly available in clinical environments. The continuous tissue oxygenation monitoring provided by the NIRS monitoring wearable system may capture the complete time course of deoxygenation and reoxygenation, enabling extraction of slope parameters that characterize microvascular function. The correlation of reperfusion slopes with heart failure severity may support the use of vascular occlusion testing as a tool for assessing cardiovascular disease status and potentially for monitoring disease progression or treatment response in heart failure patients.

The NIRS monitoring wearable system may be used for six-minute walk test (6MWT) assessment to measure tissue oxygenation kinetics during mild physical activity representative of daily living. The six-minute walk test may comprise a standardized clinical assessment where subjects walk at their own pace for six minutes under clinical supervision, with the total distance traveled recorded as a measure of functional exercise capacity. The tissue oxygenation measurements obtained during the six-minute walk test may provide quantitative data about cardiovascular and pulmonary responses to physical activity that complement the distance-based assessment of functional capacity.

The six-minute walk test protocol may use the NIRS monitoring wearable system with sensors placed at multiple body locations to monitor tissue oxygenation changes during the walking activity. Sensors may be positioned on the dominant thigh to monitor lower limb muscle oxygenation during the walking activity, and sensors may be positioned on the sternum to monitor central tissue oxygenation. The multi-site monitoring during the six-minute walk test may provide information about tissue oxygenation responses in both the actively working lower limb muscles and the central thoracic tissues, allowing assessment of how different tissue compartments respond to the metabolic demands of walking activity.

The NIRS monitoring wearable system may record baseline tissue oxygenation values at the sternum and thigh sites prior to the start of the six-minute walk test for comparison during activity. The baseline tissue oxygenation value at the sternum site may be approximately 86.6 percent, representing the resting tissue oxygen saturation in the chest wall tissues overlying the sternum. The baseline tissue oxygenation value at the thigh site may be approximately 83.1 percent, representing the resting tissue oxygen saturation in the vastus lateralis muscle. The baseline values may serve as reference points for calculating changes in tissue oxygenation that occur during the walking activity.

The difference between the baseline tissue oxygenation values at the sternum and thigh sites may reflect differences in tissue composition, blood flow distribution, and metabolic activity between these anatomical locations at rest. The sternum site may exhibit higher baseline tissue oxygenation compared to the thigh site due to differences in the ratio of arterial to venous blood in the measured tissue volumes, differences in local metabolic oxygen consumption rates, or differences in tissue optical properties that affect the measured oxygenation values. The baseline values recorded at each site may vary between individual subjects based on factors including body composition, cardiovascular fitness, and underlying health status.

During the six-minute walk test, the NIRS system may continuously monitor tissue oxygenation at the sternum and thigh sites as the subject walks. The tissue oxygenation values may change from the baseline levels as the cardiovascular system responds to the metabolic demands of walking activity. The lower limb muscles may increase oxygen consumption during walking, which may result in decreased tissue oxygenation in the thigh as oxygen is extracted from the microvascular blood pool faster than it is replenished by arterial blood flow. The central tissues at the sternum site may also exhibit oxygenation changes during walking activity as cardiac output increases and blood flow is redistributed to meet the metabolic demands of the working muscles.

The NIRS system may measure percent change in tissue oxygenation over the initial 100 seconds of the six-minute walk test to differentiate between NYHA heart failure classes. The initial 100 seconds of the walk test may represent a period during which the cardiovascular system transitions from rest to steady-state exercise, with the rate and magnitude of tissue oxygenation changes during this transition period reflecting cardiovascular reserve and the ability to match oxygen delivery to metabolic demand. The percent change in tissue oxygenation may be calculated as the difference between the tissue oxygenation value at 100 seconds and the baseline value, expressed as a percentage of the baseline value or as an absolute percentage point change.

The tissue deoxygenation rate during the six-minute walk test may differentiate patients based on NYHA heart failure class, where faster decrease rates in tissue oxygenation indicate more severe heart failure. Subjects with more severe heart failure may exhibit faster decreases in tissue oxygenation during the initial phase of the walk test because their cardiovascular systems may have reduced capacity to increase cardiac output and blood flow in response to the metabolic demands of walking activity. The impaired cardiovascular response in more severe heart failure may result in a mismatch between oxygen delivery and oxygen consumption, leading to more rapid depletion of oxygen from the microvascular blood pool and faster decreases in measured tissue oxygenation.

The tissue deoxygenation rate may be calculated from the slope of the tissue oxygenation versus time curve during the initial portion of the six-minute walk test. The slope calculation may involve linear regression or other curve-fitting techniques applied to the tissue oxygenation data during the first 100 seconds or another specified time window of the walk test. The deoxygenation rate may be expressed as the change in tissue oxygenation percentage per unit time, such as percent per second or percent per minute, allowing quantitative comparison of deoxygenation kinetics between subjects with different heart failure severity.

Greater magnitude decreases in tissue oxygenation during the six-minute walk test may also indicate more severe heart failure. The magnitude of tissue oxygenation decrease may be measured as the difference between the baseline tissue oxygenation value and the minimum tissue oxygenation value reached during the walk test, or as the difference between baseline and the tissue oxygenation value at a specified time point such as 100 seconds. Subjects with more severe heart failure may exhibit larger decreases in tissue oxygenation because their cardiovascular systems may be unable to maintain adequate oxygen delivery to meet the metabolic demands of walking activity, resulting in greater depletion of oxygen from the tissue microvascular bed.

The percent change in tissue oxygenation over the initial 100 seconds of the six-minute walk test may vary between NYHA classes at both the sternum and thigh measurement sites. At the sternum site, subjects with NYHA Class I heart failure may exhibit a percent change of approximately 5.3 percent over the initial 100 seconds, while subjects with NYHA Class II heart failure may exhibit a percent change of approximately 3.35 percent, and subjects with NYHA Class III heart failure may exhibit a percent change of approximately 2.67 percent. The decreasing percent change values with increasing NYHA class severity may reflect the reduced cardiovascular reserve and impaired ability to increase cardiac output in response to exercise in subjects with more severe heart failure.

At the thigh site, the percent change in tissue oxygenation over the initial 100 seconds of the six-minute walk test may also vary between NYHA classes, though the pattern may differ from the sternum site due to the different tissue compartments being measured. The thigh measurements may reflect oxygenation changes in the actively working vastus lateralis muscle, where oxygen consumption increases during walking activity. The relationship between percent change in thigh tissue oxygenation and NYHA class may provide complementary information to the sternum measurements about how the cardiovascular system responds to the metabolic demands of physical activity in subjects with different heart failure severity.

The six-minute walk test protocol may record the test onset time, total distance traveled, distance traveled per minute during the test, and any clinical notes in a case report form. The distance measurements may provide a conventional measure of functional exercise capacity that can be correlated with the tissue oxygenation kinetics measured by the NIRS monitoring wearable system. Subject breaks or test discontinuation may be recorded in the case report form notes, including whether the subject rested standing up or sitting, as rest periods may affect the tissue oxygenation measurements and the interpretation of the oxygenation kinetics data.

Blood pressure measurements may be recorded preceding and following the six-minute walk test to provide additional cardiovascular assessment data. The blood pressure measurements may be used to assess the cardiovascular response to the walking activity and may be correlated with the tissue oxygenation changes observed during the test. The combination of tissue oxygenation kinetics, distance traveled, and blood pressure response may provide a comprehensive assessment of cardiovascular function during mild physical activity that can be used to characterize heart failure patients and potentially to monitor disease progression or treatment response over time.

The tissue oxygenation kinetics measured during the six-minute walk test may provide information about cardiovascular function that is not captured by the distance measurement alone. Two subjects may walk similar distances during the six-minute walk test but exhibit different tissue oxygenation kinetics, with one subject maintaining relatively stable tissue oxygenation and another subject exhibiting rapid decreases in tissue oxygenation during the activity. The tissue oxygenation kinetics may reflect differences in cardiovascular reserve, microvascular function, or metabolic efficiency that affect the ability to maintain tissue oxygen delivery during physical activity.

The six-minute walk test assessment using the NIRS monitoring wearable system may provide a step toward remote monitoring of heart failure patients during daily activities. The tissue oxygenation kinetics observed during the standardized six-minute walk test may establish baseline patterns for individual patients that can be compared to tissue oxygenation patterns observed during daily activities in home or ambulatory monitoring settings. Changes in tissue oxygenation kinetics during daily activities compared to baseline patterns may indicate changes in cardiovascular function that could prompt clinical evaluation or intervention.

The correlation between tissue deoxygenation rate during the six-minute walk test and NYHA heart failure class may support the use of tissue oximetry as a tool for assessing cardiovascular disease status. The NYHA classification system provides a subjective assessment of heart failure severity based on symptoms and functional limitations, while the tissue deoxygenation rate provides an objective physiological measurement that may correlate with disease severity. The combination of subjective clinical assessment and objective tissue oximetry measurements may provide a more comprehensive characterization of heart failure patients than either assessment method alone.

The multi-site tissue oxygenation monitoring during the six-minute walk test may allow assessment of regional differences in cardiovascular response to physical activity. The sternum site may reflect central cardiovascular function and the ability to increase cardiac output during activity, while the thigh site may reflect peripheral muscle perfusion and the ability to deliver oxygen to the working muscles. Differences in tissue oxygenation kinetics between the sternum and thigh sites may provide information about the distribution of blood flow during activity and the coordination between central cardiac function and peripheral vascular responses.

The six-minute walk test protocol using the NIRS system may be performed in clinical settings with minimal additional equipment requirements beyond the standard six-minute walk test setup. The NIRS sensors may be applied to the subject prior to the walk test and may continuously record tissue oxygenation data throughout the test without requiring additional intervention by clinical staff. The tissue oxygenation data may be stored on the NIRS system or transmitted wirelessly to a mobile device for real-time display and subsequent analysis. The integration of tissue oximetry monitoring with the standard six-minute walk test may enhance the clinical utility of the test by providing additional physiological data about cardiovascular function during mild physical activity.

The NIRS system may be used for postural reactivity testing to monitor tissue oxygenation changes when a subject transitions from a supine position to a standing position. Postural reactivity testing may assess cardiovascular response to postural change, specifically monitoring for potential orthostatic hypotension and tissue oxygenation hyper-reactivity to standing. Orthostatic hypotension may occur when the cardiovascular system fails to adequately compensate for the gravitational redistribution of blood volume that occurs upon standing, resulting in decreased blood pressure and potentially reduced tissue perfusion. The tissue oxygenation changes observed during the postural transition may provide quantitative measures of cardiovascular function that complement blood pressure measurements in assessing orthostatic responses.

The postural reactivity testing protocol may require the subject to recline in a supine position on a clinical exam table for a minimum of five minutes before standing. The five-minute supine period may allow the cardiovascular system to reach a stable resting state with blood volume distributed according to the supine body position. During the supine period, the subject may remain still without sitting up or getting up from the table to avoid perturbations that could affect the baseline cardiovascular state. The stable supine baseline may serve as a reference condition for assessing the magnitude and dynamics of cardiovascular changes that occur upon standing.

Blood pressure may be recorded at one minute intervals for three consecutive minutes while the subject remains in the supine position. The three consecutive blood pressure measurements may provide an assessment of baseline blood pressure stability and may allow calculation of an average supine blood pressure value for comparison with standing blood pressure measurements. The one minute intervals between blood pressure measurements may allow sufficient time for the blood pressure measurement procedure while maintaining a standardized timing protocol. The blood pressure measurements may be performed on the left upper arm using a standard sphygmomanometer or automated blood pressure monitor.

Immediately after the third supine blood pressure measurement is completed, the subject may stand up from the exam table under clinical supervision. The transition from supine to standing may be performed as quickly as is safely possible to create a standardized postural challenge that tests the cardiovascular system's ability to respond to rapid gravitational redistribution of blood volume. Clinical supervision during the standing transition may ensure subject safety and may allow observation of any symptoms of orthostatic intolerance such as dizziness, lightheadedness, or visual disturbances that may accompany orthostatic hypotension.

Following the position change to standing, blood pressure may again be recorded at one minute intervals for three consecutive minutes. The first standing blood pressure measurement may begin after the subject has stood for one minute, allowing time for the initial cardiovascular response to the postural change to occur. The three consecutive standing blood pressure measurements may capture the time course of blood pressure changes following the postural transition, including any initial decrease in blood pressure and subsequent recovery as cardiovascular compensatory mechanisms engage. The difference between supine and standing blood pressure measurements may be calculated to quantify the magnitude of orthostatic blood pressure change.

The NIRS system may continuously monitor tissue oxygenation at multiple body sites throughout the postural reactivity test, including during the supine baseline period, during the transition from supine to standing, and during the standing period. Sensors may be positioned on the forearm, sternum, and thigh to monitor tissue oxygenation changes at upper limb, central, and lower limb sites during the postural transition. The multi-site monitoring may allow assessment of regional differences in tissue oxygenation responses to the postural change, as different body regions may experience different magnitudes of blood volume redistribution and perfusion changes upon standing.

The tissue oxygenation kinetics measured during the postural reactivity test may be compared to the systolic blood pressure difference between supine and standing positions. The comparison may allow assessment of the relationship between blood pressure changes and tissue oxygenation changes during the postural transition. In subjects with adequate cardiovascular compensation, blood pressure may be maintained near baseline levels upon standing, and tissue oxygenation may remain relatively stable. In subjects with orthostatic hypotension, blood pressure may decrease upon standing, and tissue oxygenation may also decrease as reduced blood pressure results in decreased tissue perfusion.

The tissue oxygenation changes measured during the postural reactivity test may relate to heart failure severity, where faster decreases in tissue oxygenation rate upon standing and greater overall decreases in tissue oxygenation magnitude upon standing may indicate more severe heart failure. Subjects with more severe heart failure may have reduced cardiovascular reserve and impaired ability to compensate for the gravitational redistribution of blood volume that occurs upon standing. The impaired compensation may result in larger decreases in blood pressure and tissue perfusion upon standing, which may be reflected in faster and larger decreases in tissue oxygenation measured by the NIRS system.

The postural reactivity test may assess potential hypotension reactions that are common during daily routine tasks such as getting out of bed or rapid transition from supine to standing. The standardized postural reactivity testing protocol may provide a controlled assessment of cardiovascular responses to postural change that can be correlated with symptoms and functional limitations experienced by patients during daily activities. The tissue oxygenation measurements obtained during the postural reactivity test may provide objective physiological data that complements subjective symptom reports and blood pressure measurements in characterizing orthostatic responses.

The test onset time and all blood pressure measurements recorded during the postural reactivity test may be documented in a case report form for subsequent analysis. The timing information may allow synchronization of the blood pressure measurements with the continuous tissue oxygenation data recorded by the NIRS system. The synchronized data may enable correlation of blood pressure changes with tissue oxygenation changes at each measurement time point, allowing assessment of the relationship between systemic hemodynamic changes and regional tissue perfusion changes during the postural transition.

The tissue oxygenation difference between supine and standing positions may be calculated for each sensor location to quantify the magnitude of tissue oxygenation change associated with the postural transition. The tissue oxygenation difference may be calculated as the difference between the average tissue oxygenation value during the supine baseline period and the tissue oxygenation value at a specified time point following standing, or as the difference between supine baseline and the minimum tissue oxygenation value reached during the standing period. The tissue oxygenation differences at the forearm, sternum, and thigh sites may provide complementary information about regional cardiovascular responses to the postural change.

The rate of tissue oxygenation decrease upon standing may be calculated from the slope of the tissue oxygenation versus time curve during the transition from supine to standing. The deoxygenation rate may reflect the speed at which tissue perfusion decreases following the postural change, with faster deoxygenation rates indicating more rapid decreases in tissue blood flow. The deoxygenation rate may be calculated separately for each sensor location, allowing comparison of the dynamics of tissue oxygenation changes at different body sites during the postural transition.

The postural reactivity testing protocol using the NIRS system may provide a standardized assessment of cardiovascular responses to postural change that can be performed in clinical settings with minimal equipment requirements. The combination of blood pressure measurements and continuous tissue oxygenation monitoring may provide a more comprehensive assessment of orthostatic responses than blood pressure measurements alone. The tissue oxygenation measurements may detect changes in tissue perfusion that occur even when blood pressure changes are modest, potentially identifying subjects with impaired cardiovascular compensation who may be at risk for orthostatic symptoms during daily activities.

The multi-site tissue oxygenation monitoring during the postural reactivity test may allow assessment of regional differences in cardiovascular response to the postural change. The forearm site may reflect upper extremity perfusion changes, the sternum site may reflect central thoracic perfusion changes, and the thigh site may reflect lower extremity perfusion changes during the transition from supine to standing. The gravitational redistribution of blood volume upon standing may result in different patterns of perfusion change at different body sites, with lower extremity sites potentially experiencing blood pooling and upper body sites potentially experiencing reduced perfusion as blood shifts toward the lower body.

The correlation between tissue oxygenation changes during postural reactivity testing and heart failure severity may support the use of tissue oximetry as a tool for assessing cardiovascular disease status. The NYHA classification system provides a subjective assessment of heart failure severity based on symptoms and functional limitations, while the tissue oxygenation kinetics during postural reactivity testing provide objective physiological measurements that may correlate with disease severity. The combination of subjective clinical assessment and objective tissue oximetry measurements during standardized postural challenges may provide a more comprehensive characterization of cardiovascular function in heart failure patients than either assessment method alone.

The NIRS system may classify patients according to New York Heart Association (NYHA) heart failure classes I through III based on tissue oxygenation kinetics measurements. The NYHA functional classification system categorizes heart failure patients into four classes based on the severity of symptoms and functional limitations experienced during physical activity. Class I patients may have heart disease but experience no symptoms and no limitation in ordinary physical activity. Class II patients may experience slight limitation of physical activity, with ordinary activity resulting in fatigue, palpitation, dyspnea, or anginal pain. Class III patients may experience marked limitation of physical activity, with less than ordinary activity causing fatigue, palpitation, dyspnea, or anginal pain. The tissue oxygenation kinetics measurements obtained during standardized cardiovascular stressors may provide objective physiological data that correlates with the NYHA classification and may support differentiation between patients with different disease severity levels.

The classification of patients according to NYHA heart failure classes based on tissue oxygenation kinetics may involve analysis of multiple parameters extracted from the tissue oximetry signals during cardiovascular stressor protocols. The parameters may include deoxygenation slopes during vascular occlusion or physical activity, reoxygenation slopes during reperfusion following vascular occlusion release, baseline tissue oxygenation values at rest, minimum tissue oxygenation values reached during stressor protocols, and the time required for tissue oxygenation to return to baseline following perturbation. The combination of these parameters may provide a multi-dimensional characterization of microvascular function that correlates with heart failure severity and supports classification of patients into NYHA classes.

The tissue oxygenation kinetics measurements may differentiate between NYHA Class I, Class II, and Class III heart failure patients based on quantitative differences in the measured parameters. Patients with NYHA Class I heart failure may exhibit tissue oxygenation kinetics that are relatively preserved compared to healthy individuals, with reperfusion slopes following vascular occlusion that approach normal values and tissue deoxygenation rates during physical activity that remain within acceptable ranges. Patients with NYHA Class II heart failure may exhibit intermediate tissue oxygenation kinetics, with reperfusion slopes that are reduced compared to Class I patients and tissue deoxygenation rates during activity that are moderately elevated. Patients with NYHA Class III heart failure may exhibit the most impaired tissue oxygenation kinetics, with the lowest reperfusion slopes and the fastest tissue deoxygenation rates during physical activity.

The microvascular responses measured during cardiovascular stressors may characterize heart failure patients based on disease stage by reflecting the underlying pathophysiological changes that occur as heart failure progresses. In early-stage heart failure corresponding to NYHA Class I, the microvascular system may retain substantial functional reserve and may be able to respond adequately to cardiovascular stressors such as vascular occlusion or physical activity. As heart failure progresses to NYHA Class II and Class III, the microvascular system may become increasingly impaired due to endothelial dysfunction, reduced capillary density, increased vascular stiffness, or other pathophysiological changes that limit the ability of the microvascular system to respond to increased metabolic demands or to recover from ischemic challenges.

The characterization of heart failure patients based on disease stage using microvascular responses may involve assessment of reactive hyperemia capacity during vascular occlusion testing. Reactive hyperemia refers to the transient increase in blood flow that occurs following release of arterial occlusion, representing the microvascular system's response to the ischemic challenge. In healthy individuals and patients with early-stage heart failure, the reactive hyperemia response may be robust, resulting in rapid reoxygenation of tissue following occlusion release. In patients with more advanced heart failure, the reactive hyperemia response may be blunted due to impaired endothelial function and reduced vasodilatory capacity, resulting in slower reoxygenation and lower reperfusion slopes measured by the NIRS monitoring wearable system.

The microvascular responses during cardiovascular stressors may also reflect the ability of the cardiovascular system to increase cardiac output and redistribute blood flow in response to metabolic demands. During physical activity such as the six-minute walk test, the cardiovascular system may increase cardiac output to meet the increased oxygen demands of working muscles. In patients with heart failure, the ability to increase cardiac output may be limited by reduced cardiac contractility, impaired ventricular filling, or other cardiac dysfunction. The limited cardiac output response may result in inadequate oxygen delivery to working muscles, leading to faster tissue deoxygenation rates and larger decreases in tissue oxygenation during activity compared to patients with less severe heart failure or healthy individuals.

The tissue oxygenation kinetics measurements may provide a quantitative basis for classifying heart failure patients that complements the subjective symptom-based NYHA classification. The NYHA classification relies on patient-reported symptoms and clinician assessment of functional limitations, which may be subject to variability based on patient perception, communication, and clinician interpretation. The tissue oxygenation kinetics measurements may provide objective physiological data that is not dependent on patient reporting and may be reproducible across different measurement sessions and clinical settings. The combination of subjective NYHA classification and objective tissue oxygenation kinetics measurements may provide a more comprehensive and reliable characterization of heart failure severity than either approach alone.

The NIRS system may monitor tissue oxygenation changes correlated with compensatory mechanisms including clot formation, vasoconstriction, and tachycardia during hemorrhage. When the body experiences blood loss, physiological compensatory mechanisms may be activated to maintain blood pressure and tissue perfusion despite the reduced blood volume. Clot formation may occur at the site of vascular injury to limit further blood loss. Vasoconstriction may occur in peripheral vascular beds to redirect blood flow toward vital organs and maintain central blood pressure. Tachycardia may occur as the heart rate increases to maintain cardiac output despite reduced stroke volume resulting from decreased venous return.

The tissue oxygenation changes measured by the NIRS system during hemorrhage may reflect the activation and effectiveness of these compensatory mechanisms. During the early stages of hemorrhage when compensatory mechanisms are effective, tissue oxygenation may be maintained near baseline levels despite the reduction in blood volume. The vasoconstriction response may redirect blood flow from peripheral tissues toward vital organs, which may result in decreased tissue oxygenation at peripheral measurement sites while central tissue oxygenation is preserved. The tachycardia response may increase cardiac output to compensate for reduced stroke volume, helping to maintain tissue perfusion and oxygenation.

The correlation between tissue oxygenation changes and compensatory mechanisms during hemorrhage may allow the NIRS system to provide information about the physiological state of the patient that is not available from blood pressure measurements alone. Blood pressure may be maintained within normal ranges during the early stages of hemorrhage due to the effectiveness of compensatory mechanisms, potentially masking the severity of blood loss. The tissue oxygenation measurements may detect changes in peripheral perfusion that occur as vasoconstriction redirects blood flow, potentially providing earlier indication of significant blood loss than blood pressure monitoring alone.

The monitoring of tissue oxygenation during hemorrhage may allow tracking of the progression from compensated to decompensated shock states. During compensated shock, the compensatory mechanisms may be sufficient to maintain adequate tissue perfusion and oxygenation despite reduced blood volume. As blood loss continues or compensatory mechanisms become exhausted, the patient may transition to decompensated shock where tissue perfusion becomes inadequate and tissue oxygenation decreases. The continuous tissue oxygenation monitoring provided by the NIRS monitoring wearable system may detect this transition by identifying decreases in tissue oxygenation that indicate failure of compensatory mechanisms to maintain adequate tissue perfusion.

The vasoconstriction response during hemorrhage may be reflected in tissue oxygenation measurements at peripheral sites such as the forearm or thigh. As peripheral vasoconstriction occurs, blood flow to these tissues may decrease, resulting in reduced oxygen delivery and decreased tissue oxygenation. The magnitude and timing of tissue oxygenation decreases at peripheral sites may provide information about the intensity of the vasoconstriction response and the degree of blood flow redistribution occurring in response to hemorrhage. The tissue oxygenation measurements may complement blood pressure monitoring by providing information about regional perfusion changes that occur as part of the compensatory response.

The tachycardia response during hemorrhage may be detected through the pulse rate measurements derived from the photoplethysmography signals acquired by the NIRS monitoring wearable system. As heart rate increases in response to hemorrhage, the pulse rate measurements may show corresponding increases that can be correlated with the tissue oxygenation changes. The relationship between pulse rate changes and tissue oxygenation changes may provide information about the effectiveness of the tachycardia response in maintaining tissue perfusion. In cases where tachycardia is effective in maintaining cardiac output, tissue oxygenation may be preserved despite the increased heart rate. In cases where tachycardia is insufficient to compensate for reduced stroke volume, tissue oxygenation may decrease despite the elevated heart rate.

The clot formation response during hemorrhage may not be directly measured by the NIRS system, but the effectiveness of clot formation in limiting blood loss may be reflected in the overall trajectory of tissue oxygenation changes over time. Effective clot formation may limit the rate of blood loss and allow compensatory mechanisms to maintain tissue perfusion, resulting in stabilization or recovery of tissue oxygenation values. Ineffective clot formation may allow continued blood loss that eventually overwhelms compensatory mechanisms, resulting in progressive decreases in tissue oxygenation as the patient transitions toward decompensated shock.

The monitoring of compensatory mechanisms through tissue oxygenation measurements may support clinical decision-making during hemorrhage resuscitation. The tissue oxygenation trends may provide real-time feedback about the effectiveness of resuscitation interventions in restoring tissue perfusion. As blood volume is restored through fluid administration or blood transfusion, the compensatory vasoconstriction may relax and peripheral tissue oxygenation may increase as blood flow is redistributed back to peripheral tissues. The tissue oxygenation recovery may provide an indication that resuscitation is effective in restoring adequate tissue perfusion, complementing blood pressure measurements in assessing resuscitation adequacy.

The correlation between tissue oxygenation changes and compensatory mechanisms may allow the NIRS system to provide information about the physiological reserve of the patient during hemorrhage. Patients with greater physiological reserve may exhibit more robust compensatory responses and may maintain tissue oxygenation at higher levels for longer periods during hemorrhage. Patients with reduced physiological reserve due to age, comorbidities, or pre-existing cardiovascular disease may exhibit less effective compensatory responses and may show earlier decreases in tissue oxygenation during hemorrhage. The tissue oxygenation measurements may help identify patients who are at higher risk for rapid decompensation and who may require more aggressive resuscitation interventions.

The tissue oxygenation kinetics during hemorrhage may also provide information about the transition between different stages of hypovolemic shock. Hypovolemic shock may be classified into stages based on the percentage of blood volume lost and the associated physiological changes. In early stages of hemorrhage with blood volume loss of less than 15 percent, compensatory mechanisms may maintain blood pressure and tissue perfusion with minimal changes in tissue oxygenation. As blood volume loss increases to 15 to 30 percent, compensatory mechanisms may become more active, with vasoconstriction and tachycardia becoming more pronounced and tissue oxygenation at peripheral sites beginning to decrease. With blood volume loss exceeding 30 percent, compensatory mechanisms may become insufficient to maintain adequate tissue perfusion, resulting in more significant decreases in tissue oxygenation and progression toward decompensated shock.

The continuous monitoring of tissue oxygenation during hemorrhage may allow detection of changes in compensatory mechanism effectiveness over time. The compensatory mechanisms may initially be effective in maintaining tissue perfusion, but may become exhausted or overwhelmed as hemorrhage continues. The tissue oxygenation measurements may detect the transition from effective compensation to failing compensation by identifying accelerating decreases in tissue oxygenation or failure of tissue oxygenation to respond to resuscitation interventions. The early detection of failing compensatory mechanisms may allow clinical intervention before the patient progresses to severe decompensated shock with associated morbidity and mortality risks.

The NIRS system may provide tissue oxygenation measurements that trend with mean arterial pressure during hypovolemic hemorrhage, allowing continuous assessment of the efficacy of medical interventions in patients for whom continuous blood pressure monitoring may be unavailable or impractical. The correlation between tissue oxygenation and mean arterial pressure may result from the relationship between blood pressure, tissue perfusion, and oxygen delivery. As mean arterial pressure decreases during hemorrhage, tissue perfusion may decrease proportionally, resulting in decreased oxygen delivery and decreased tissue oxygenation. The tissue oxygenation measurements may serve as a surrogate indicator of perfusion pressure when direct blood pressure monitoring is not available.

The tracking of compensatory mechanisms through tissue oxygenation monitoring may support the development of resuscitation endpoints based on tissue perfusion rather than blood pressure alone. Current resuscitation protocols may target maintenance of mean arterial blood pressure above specified thresholds, but blood pressure alone may not fully reflect the adequacy of tissue perfusion. The tissue oxygenation measurements may provide additional information about whether resuscitation interventions are effectively restoring tissue perfusion, potentially allowing more individualized resuscitation endpoints based on the physiological response of each patient. The real-time estimation of tissue perfusion through continuous tissue oxygenation monitoring may provide a suitable resuscitation endpoint that reflects the ultimate goal of resuscitation, which is to restore adequate oxygen delivery to tissues.

The NIRS system may be used to monitor tissue oxygenation during delivery of chemotherapeutic agents to detect chemotherapy-induced cardiotoxicity (CIC). Chemotherapy-induced cardiotoxicity refers to adverse effects on the heart and cardiovascular system that may result from cancer treatments, particularly chemotherapy and targeted therapies. The NIRS monitoring wearable system may provide continuous monitoring of tissue oxygenation and other cardiovascular parameters during chemotherapy infusion sessions, allowing real-time observation of the effects of chemotherapy treatment on cardiovascular function as well as the magnitude of those effects.

The chemotherapeutic agents monitored by the NIRS system may include anthracyclines such as doxorubicin and epirubicin. Anthracyclines represent a class of chemotherapy drugs that are associated with cardiotoxicity, with the risk of anthracycline-induced cardiomyopathy increasing with cumulative dose. The risk of cardiomyopathy may be approximately 3 to 5 percent with cumulative doses up to 400 mg/m$^2$, and may increase to approximately 18 to 48 percent with cumulative doses of 700 mg/m$^2$. The NIRS monitoring wearable system may monitor tissue oxygenation and cardiovascular parameters during anthracycline infusion to detect acute cardiovascular changes that may indicate sensitivity to cardiotoxic effects.

The chemotherapeutic agents monitored by the NIRS system may also include taxanes such as paclitaxel and docetaxel. Taxanes represent another class of chemotherapy drugs used in breast cancer treatment that may contribute to cardiotoxicity. The combination of anthracyclines and taxanes in complex treatment regimens may result in cumulative cardiotoxic effects that require monitoring throughout the course of treatment. The NIRS system may monitor tissue oxygenation during infusion of taxane-based chemotherapy to detect cardiovascular changes associated with these agents.

The physiological mechanisms of anthracycline-induced cardiotoxicity may include generation of free radicals, oxidative stress, mitochondrial dysfunction, and damage to heart muscle cells. While the exact physiological effects of anthracycline-induced cardiotoxicity are complex and not fully understood, the proposed mechanisms of action may result in measurable changes in cardiovascular function that can be detected through tissue oxygenation monitoring. The NIRS system may detect changes in tissue oxygenation, cardiac output, stroke volume, and other cardiovascular parameters that reflect the acute effects of chemotherapy on cardiovascular function.

The NIRS system may gather comprehensive patient data throughout chemotherapy treatment including StO$_2$, SpO$_2$, cardiac output (CO), stroke volume (SV), PR, and RR. The combination of NIRS sensor devices to measure cardiac hemodynamics and both central and peripheral tissue oximetry may provide a multi-dimensional assessment of cardiovascular function during chemotherapy infusion. Blood pressure and pulse measurements may be collected for baseline validation using a non-invasive blood pressure system. The comprehensive data collection may allow monitoring of the acute effects of chemotherapy on cardiovascular function and may support identification of patients who exhibit cardiovascular sensitivity to the chemotherapeutic agents.

The NIRS system may designate patients as responders or non-responders to cardiotoxic chemotherapy based on measured cardiovascular changes during treatment. Responders may be defined as patients who exhibit measurable changes in cardiovascular parameters during or following chemotherapy infusion, indicating sensitivity to the cardiotoxic aspects of the treatment. Non-responders may be defined as patients who do not exhibit significant cardiovascular changes during or following chemotherapy infusion, indicating relative resistance to the cardiotoxic effects. The designation of patients as responders or non-responders may be based on the magnitude of cardiovascular parameter changes, the timing of changes relative to chemotherapy infusion, or the pattern of changes observed across multiple treatment sessions.

The designation of patients as responders may involve detection of earlier changes in measurable cardiovascular metrics or more severe cardiovascular responses during chemotherapy infusion. Earlier changes may include decreases in tissue oxygenation, changes in cardiac output or stroke volume, or alterations in pulse rate or respiratory rate that occur during or shortly after chemotherapy infusion. More severe responses may include larger magnitude changes in cardiovascular parameters or changes that persist for longer durations following chemotherapy infusion. The NIRS system may process and compare the cardiovascular measures gathered during treatment to determine the extent of each patient's response to chemotherapy-induced cardiotoxicity.

Patients designated as responsive to cardiotoxic chemotherapy may be referred to physicians for supplementary treatments to mitigate or avoid the development of cardiac abnormalities including heart failure. The supplementary treatments may include cardiac support medications such as beta blockers that may help protect the heart from the cardiotoxic effects of chemotherapy. The early identification of responsive patients through tissue oxygenation monitoring may allow proactive intervention before significant cardiac damage occurs, potentially improving long-term cardiovascular outcomes for patients undergoing chemotherapy treatment.

The NIRS system may provide longitudinal monitoring capability during the entire course of breast cancer treatment for cardiotoxicity assessment. Breast cancer treatment may be administered over a course of several months, with multiple chemotherapy infusion sessions occurring at regular intervals throughout the treatment period. The longitudinal monitoring capability may allow clinicians to track changes in cardiovascular function over time and adjust treatment regimens as needed between infrequent in-person clinical cardiac function assessments during therapy. The continuous monitoring of tissue oxygenation and cardiovascular parameters across multiple treatment sessions may reveal cumulative effects of chemotherapy on cardiovascular function that may not be apparent from single-session assessments.

The longitudinal monitoring capability may extend beyond the active treatment period to monitor cardiovascular function during remission. Approximately 4 to 10 percent of patients with no pre-existing cardiovascular conditions have been found to develop heart failure within a year of having chemotherapy treatment. The extended monitoring capability may allow detection of delayed cardiotoxic effects that develop after completion of chemotherapy treatment. The NIRS system may be used for periodic cardiovascular assessments during the post-treatment period to identify patients who develop cardiovascular abnormalities that may require clinical intervention.

The longitudinal monitoring during breast cancer treatment may involve comparison of cardiovascular parameters measured during successive chemotherapy infusion sessions. The NIRS system may establish baseline cardiovascular parameters during the first treatment session and may track changes in these parameters across subsequent sessions. Progressive changes in tissue oxygenation, cardiac output, stroke volume, or other cardiovascular parameters across treatment sessions may indicate cumulative cardiotoxic effects that warrant clinical attention. The longitudinal data may allow identification of trends in cardiovascular function that may predict the development of clinically significant cardiotoxicity.

The NIRS system may identify commonalities between patients with similar cardiotoxicity responses including age, race, body mass index (BMI), subtype of cancer, duration of treatment, or type of treatment. The identification of patient commonalities may involve analysis of demographic information, clinical characteristics, and treatment parameters in conjunction with the cardiovascular response data measured during chemotherapy infusion. The analysis may reveal patterns that associate certain patient characteristics with increased or decreased sensitivity to chemotherapy-induced cardiotoxicity.

Age may represent a patient factor that influences the risk of cardiac damage from chemotherapy treatment. Older patients may exhibit different cardiovascular responses to chemotherapy compared to younger patients due to age-related changes in cardiovascular function, reduced physiological reserve, or increased prevalence of underlying cardiovascular disease. The NIRS system may collect age information as part of the patient demographic data and may correlate age with measured cardiovascular responses to identify age-related patterns in cardiotoxicity sensitivity.

Race may represent another patient factor that influences cardiotoxicity responses. Different racial groups may exhibit different baseline cardiovascular characteristics, different prevalences of cardiovascular risk factors, or different pharmacokinetic responses to chemotherapeutic agents that affect cardiotoxicity risk. The NIRS monitoring wearable system may collect race information as part of the patient demographic data and may analyze cardiovascular response patterns across different racial groups to identify race-related differences in cardiotoxicity sensitivity.

Body mass index (BMI) may influence cardiotoxicity responses through effects on drug distribution, metabolism, and cardiovascular function. Patients with higher BMI may exhibit different tissue oxygenation patterns, different cardiac output responses, or different drug exposure levels compared to patients with lower BMI. The NIRS monitoring wearable system may collect height and weight information to calculate BMI and may correlate BMI with measured cardiovascular responses to identify BMI-related patterns in cardiotoxicity sensitivity.

Subtype of cancer may influence cardiotoxicity responses through effects on treatment selection, disease burden, or underlying physiological state. Different breast cancer subtypes may require different chemotherapy regimens with different cardiotoxic potential. The NIRS monitoring wearable system may collect cancer subtype information as part of the clinical data and may analyze cardiovascular response patterns across different cancer subtypes to identify subtype-related differences in cardiotoxicity sensitivity.

Duration of treatment may influence cardiotoxicity responses through cumulative exposure to cardiotoxic agents. Patients who receive longer treatment courses may accumulate higher cumulative doses of anthracyclines or other cardiotoxic agents, potentially increasing the risk of cardiovascular damage. The NIRS monitoring wearable system may track treatment duration and cumulative drug exposure and may correlate these parameters with measured cardiovascular responses to identify duration-related patterns in cardiotoxicity development.

Type of treatment may influence cardiotoxicity responses through the specific cardiotoxic potential of different chemotherapeutic agents and combinations. Treatment regimens that include anthracyclines may carry higher cardiotoxicity risk compared to regimens that do not include these agents. The combination of multiple cardiotoxic agents may result in additive or synergistic cardiotoxic effects. The NIRS monitoring wearable system may collect treatment regimen information and may analyze cardiovascular response patterns across different treatment types to identify treatment-related differences in cardiotoxicity sensitivity.

The identification of commonalities between patients with similar cardiotoxicity responses may support development of predictive models to evaluate individual patient response to cardiotoxic chemotherapy drugs. The predictive models may combine insights and conclusions from analysis of patient demographics, clinical characteristics, treatment parameters, and measured cardiovascular responses to identify patients with the greatest likelihood of developing cardiotoxicity. The predictive models may allow clinicians to identify high-risk patients before or early in the treatment course, enabling proactive interventions to mitigate cardiotoxicity risk.

The data acquired from patients undergoing chemotherapy monitoring may be combined with results of follow-up visits to refine the predictive models for cardiotoxicity risk. The follow-up data may include cardiovascular assessments performed at later appointments or clinical evaluations performed in the case of cardiac complications. The correlation of acute cardiovascular responses measured during chemotherapy infusion with long-term cardiovascular outcomes may allow validation and refinement of the predictive models. The refined models may provide improved accuracy in identifying patients who are at risk for developing clinically significant cardiotoxicity.

The NIRS system may use the change in cardiac output during delivery of chemotherapeutic agents compared to patient outcomes during chemotherapy treatment to build an initial assessment framework for estimating individual patient sensitivity to chemotherapy-induced cardiotoxicity. The assessment framework may determine whether individual patients would be suitable candidates for cardiac support medications or physician follow-up to mitigate dangerous complications associated with chemotherapy. The dangerous complications may include heart failure or arrhythmias that may develop as a result of chemotherapy-induced cardiac damage.

The greater understanding of patient cardiovascular status provided by the NIRS monitoring wearable system may aid physicians in providing the safest and most effective treatment for patients undergoing chemotherapy. The real-time monitoring of cardiovascular function during chemotherapy infusion may allow immediate detection of adverse cardiovascular responses that may require intervention. The longitudinal monitoring across the treatment course may allow tracking of cumulative cardiovascular effects and adjustment of treatment regimens to minimize cardiotoxicity risk while maintaining therapeutic efficacy. The identification of patient risk factors and development of predictive models may allow personalized treatment approaches that account for individual patient sensitivity to cardiotoxic effects.

The NIRS system may provide early and non-invasive detection of cardiotoxicity in breast cancer patients undergoing treatment, both in the clinic during chemotherapy infusion and when implemented in a home monitoring environment. The early detection capability may allow identification of cardiovascular changes at an early stage, enabling higher resolution monitoring between in-clinic evaluation with traditional markers such as echocardiography. The non-invasive nature of the NIRS monitoring may allow frequent cardiovascular assessments without the burden, cost, or scheduling constraints associated with traditional cardiac imaging modalities.

The individualized monitoring capability of the NIRS system may align with the observation that breast cancer patients may exhibit individual variations in their susceptibility to cardiotoxic effects. The NIRS monitoring may identify these individual variations through measurement of cardiovascular responses during chemotherapy infusion, allowing tailored interventions based on each patient's specific cardiotoxicity risk profile. The personalized approach to cardiotoxicity monitoring may improve cardiovascular outcomes by ensuring that patients who are most sensitive to cardiotoxic effects receive appropriate protective interventions while avoiding unnecessary interventions in patients who are less sensitive.

The implementation of the NIRS system in a home monitoring environment may enable remote patient monitoring of cardiovascular function between clinic visits. The remote monitoring capability may allow more frequent cardiovascular assessments than would be practical with in-clinic monitoring alone, potentially improving detection of cardiovascular changes that develop between scheduled clinic appointments. The home monitoring data may be transmitted to clinical monitoring systems or telemedicine platforms where clinicians can review the cardiovascular data and make clinical decisions about the need for intervention or treatment modification.

The benefits of remote patient monitoring with the NIRS system may include improved physician confidence and patient outcomes through availability of higher volume quality medical data. The remote monitoring may also increase patient throughput by allowing physicians to manage a larger group of patients through review of remotely collected cardiovascular data rather than requiring in-person clinic visits for all cardiovascular assessments. The remote monitoring capability may be particularly beneficial for patients who face barriers to accessing in-person healthcare services due to geographic distance, mobility limitations, or other factors.

The NIRS system may monitor tissue oxygenation and perfusion during treatment for massive hemorrhage and hypovolemic shock to provide resuscitation endpoints. Massive hemorrhage may occur when a patient experiences severe blood loss that threatens life and requires immediate medical intervention to stop the bleeding and restore blood volume. Hypovolemic shock may develop when blood volume loss exceeds the capacity of physiological compensatory mechanisms to maintain adequate tissue perfusion, resulting in inadequate oxygen delivery to tissues and potential organ dysfunction. The NIRS monitoring wearable system may provide continuous tissue oxygenation measurements throughout the resuscitation process, allowing clinicians to assess the adequacy of tissue perfusion and to determine when resuscitation goals have been achieved.

The resuscitation endpoints provided by the NIRS system may be based on tissue oxygenation and perfusion measurements that reflect the ultimate goal of hemorrhage resuscitation, which is to restore adequate oxygen delivery to tissues. Current resuscitation protocols may rely primarily on blood pressure measurements to guide resuscitation efforts, but blood pressure alone may not fully reflect the adequacy of tissue perfusion. The tissue oxygenation measurements provided by the NIRS system may complement blood pressure monitoring by providing direct information about whether resuscitation interventions are effectively restoring oxygen delivery to the tissues being monitored.

The complexity of compensatory responses to massive blood loss may result in difficulty setting objective resuscitation endpoints for each patient's physiology at the time of need. Compensatory mechanisms including vasoconstriction, tachycardia, and blood flow redistribution may maintain blood pressure within acceptable ranges even when tissue perfusion is inadequate. The tissue oxygenation measurements provided by the NIRS monitoring wearable system may detect inadequate tissue perfusion that occurs despite maintenance of blood pressure, potentially providing a more sensitive indicator of resuscitation adequacy than blood pressure measurements alone.

Real-time estimation of tissue perfusion through continuous tissue oxygenation monitoring may provide a suitable resuscitation endpoint that accounts for individual patient variability in compensatory responses and physiological reserve. Different patients may require different amounts of fluid or blood product administration to achieve adequate tissue perfusion, depending on factors including the severity of blood loss, the effectiveness of compensatory mechanisms, and the presence of underlying cardiovascular disease. The tissue oxygenation measurements may allow individualized resuscitation endpoints based on the physiological response of each patient rather than fixed targets that may not be appropriate for all patients.

The NIRS monitoring wearable system may monitor tissue oxygenation trends with mean arterial pressure during hypovolemic hemorrhage to assess efficacy of medical interventions. The tissue oxygenation measurements may trend with mean arterial pressure during hemorrhage because both parameters may be affected by the reduction in blood volume and the activation of compensatory mechanisms. As blood volume decreases during hemorrhage, mean arterial pressure may decrease and tissue perfusion may become inadequate, resulting in decreased tissue oxygenation. The correlation between tissue oxygenation and mean arterial pressure may allow the NIRS monitoring wearable system to provide continuous assessment of perfusion status in patients for whom continuous blood pressure monitoring may be unavailable or impractical.

The correlation between tissue oxygenation and mean arterial pressure during hemorrhage may allow the NIRS monitoring wearable system to serve as a surrogate indicator of perfusion pressure. In emergency settings where continuous blood pressure monitoring may not be feasible due to equipment limitations, patient condition, or the need to maintain access to the patient for life-saving interventions, the tissue oxygenation measurements may provide ongoing information about the patient's hemodynamic status. The tissue oxygenation trends may indicate whether the patient's condition is stable, improving, or deteriorating, allowing clinicians to adjust resuscitation efforts accordingly.

The assessment of medical intervention efficacy through tissue oxygenation monitoring may involve tracking tissue oxygenation changes in response to specific resuscitation interventions. When fluid administration or blood transfusion is provided to restore blood volume, the tissue oxygenation measurements may increase as tissue perfusion improves. When vasopressor medications are administered to support blood pressure, the tissue oxygenation measurements may indicate whether the blood pressure support is translating into improved tissue perfusion. The real-time feedback provided by the tissue oxygenation measurements may allow clinicians to assess the effectiveness of each intervention and to adjust the resuscitation approach based on the observed physiological response.

The tissue oxygenation trends during hemorrhage resuscitation may provide information about the trajectory of the patient's condition over time. A patient whose tissue oxygenation is increasing in response to resuscitation interventions may be on a trajectory toward recovery, while a patient whose tissue oxygenation continues to decrease despite resuscitation efforts may require more aggressive intervention or may have ongoing bleeding that has not been controlled. The trend information may be more informative than single-point measurements because the trend may indicate whether the patient's condition is improving, stable, or deteriorating.

The monitoring of tissue oxygenation trends with mean arterial pressure may allow detection of dissociation between blood pressure and tissue perfusion that may occur in some patients. In some cases, blood pressure may be maintained through intense vasoconstriction while tissue perfusion remains inadequate due to the redistribution of blood flow away from peripheral tissues. The tissue oxygenation measurements may detect this dissociation by showing decreased tissue oxygenation despite maintenance of blood pressure within target ranges. The detection of blood pressure-perfusion dissociation may prompt clinicians to adjust resuscitation strategies to address the underlying perfusion deficit rather than relying solely on blood pressure targets.

The NIRS system may use permissive hypotension targets during hemorrhage control and healthy mean arterial pressure targets after bleeding resolution. Permissive hypotension refers to a resuscitation strategy where blood pressure is maintained at levels lower than normal during the period when hemorrhage is not yet controlled. The permissive hypotension approach may reduce the risk of exacerbating bleeding by avoiding aggressive fluid resuscitation that could increase blood pressure and disrupt clot formation at the bleeding site. The tissue oxygenation monitoring provided by the NIRS system may support the permissive hypotension approach by providing information about tissue perfusion adequacy during the period of controlled hypotension.

The permissive hypotension target may maintain blood pressure above 70 mmHg until hemorrhage is controlled. The 70 mmHg threshold may represent a minimum blood pressure level that provides adequate perfusion to vital organs while avoiding the risks associated with higher blood pressure during active bleeding. The tissue oxygenation measurements provided by the NIRS monitoring wearable system may help clinicians assess whether the permissive hypotension target is providing adequate tissue perfusion for the individual patient. If tissue oxygenation decreases to concerning levels during permissive hypotension, clinicians may need to adjust the blood pressure target or provide additional interventions to support tissue perfusion.

The permissive hypotension strategy may be maintained until the source of bleeding can be resolved through surgical intervention, application of hemostatic agents, or other bleeding control measures. During the period of permissive hypotension, the NIRS system may provide continuous monitoring of tissue oxygenation to ensure that the controlled hypotension is not resulting in inadequate tissue perfusion that could lead to organ dysfunction. The tissue oxygenation measurements may provide early warning of perfusion inadequacy that may prompt adjustment of the resuscitation approach before irreversible tissue damage occurs.

After bleeding resolution, the resuscitation goal may transition to restoration and maintenance of healthy mean arterial blood pressure above 90 mmHg. The 90 mmHg threshold may represent a blood pressure level that provides adequate perfusion pressure for normal tissue function and organ perfusion. The transition from permissive hypotension targets to healthy blood pressure targets may occur once the source of bleeding has been controlled and the risk of exacerbating hemorrhage through increased blood pressure has been eliminated. The NIRS system may monitor tissue oxygenation during this transition to assess whether the restoration of normal blood pressure is translating into improved tissue perfusion.

The tissue oxygenation measurements during the transition from permissive hypotension to healthy blood pressure targets may provide information about the recovery of tissue perfusion following hemorrhage control. As blood pressure is restored to normal levels and blood volume is replenished through continued fluid or blood product administration, tissue oxygenation may increase as oxygen delivery to tissues improves. The rate and magnitude of tissue oxygenation recovery may provide information about the adequacy of resuscitation and the restoration of normal cardiovascular function.

The NIRS system may support the massive transfusion protocol that may be performed in severe cases of hemorrhage to completely restore a body's entire blood volume within 24 hours. During massive transfusion, hospitals may utilize a rapid infuser to warm and supplement blood components including fibrinogen, complete blood count components, prothrombin time factors, partial thromboplastin time factors, international normalized ratio components, and ionized calcium as needed. The tissue oxygenation monitoring provided by the NIRS system may track the effectiveness of the massive transfusion in restoring tissue perfusion throughout the transfusion process.

The tissue oxygenation measurements during massive transfusion may provide feedback about the adequacy of blood component replacement in restoring oxygen-carrying capacity and tissue perfusion. As red blood cells are transfused, the oxygen-carrying capacity of the blood may increase, potentially resulting in improved tissue oxygenation if cardiac output and tissue perfusion are adequate. The tissue oxygenation trends during massive transfusion may indicate whether the transfusion is effectively restoring tissue oxygen delivery or whether additional interventions may be needed to support cardiovascular function.

The recovery of vital fluid levels during hemorrhage resuscitation may occur through administration of saline, whole blood, or plasma depending on the conditions of the hemorrhagic injury. The choice of resuscitation fluid may affect the oxygen-carrying capacity of the blood and the effectiveness of resuscitation in restoring tissue oxygenation. Crystalloid solutions such as saline may restore blood volume but do not provide oxygen-carrying capacity, while blood products may restore both volume and oxygen-carrying capacity. The tissue oxygenation measurements provided by the NIRS monitoring wearable system may help clinicians assess the effectiveness of different resuscitation fluid choices in restoring tissue perfusion and oxygen delivery.

The tissue oxygenation monitoring during hemorrhage resuscitation may allow correlation of physiological responses with individual treatment actions including fluid administration, wound closure, and administration of pro-coagulation medications. The ability to monitor tissue oxygenation changes in response to specific interventions may allow easier analysis of the immediate effects of each aspect of resuscitation treatment. The insight provided by the tissue oxygenation monitoring may confirm clinical treatment efficacy while also enabling patient-specific adjustments of procedures to improve effectiveness.

The tissue oxygenation measurements during hemorrhage resuscitation may be correlated with blood pressure trends collected separately to provide a comprehensive assessment of cardiovascular status. The correlation of tissue oxygenation with blood pressure may allow assessment of the relationship between systemic hemodynamics and regional tissue perfusion during resuscitation. In cases where tissue oxygenation and blood pressure trends diverge, the divergence may indicate that blood pressure alone is not adequately reflecting tissue perfusion status and that additional assessment or intervention may be needed.

The NIRS system may provide unobtrusive monitoring of patient status throughout the resuscitation process from hemorrhagic shock. The sensor may be applied to an unobtrusive site such as the thigh to minimize interference with medical staff during resuscitation procedures. The thigh placement may provide access to peripheral tissue oxygenation measurements while allowing medical staff unrestricted access to the chest, abdomen, and upper extremities where life-saving interventions may be performed. The unobtrusive monitoring capability may allow continuous tissue oxygenation data collection without impeding the resuscitation efforts of the medical team.

The tissue oxygenation monitoring during hemorrhage resuscitation may enable observation of the initial reduction and subsequent recovery in tissue oxygenation correlated with life-saving medical treatment. The initial reduction in tissue oxygenation may reflect the effects of blood loss and inadequate tissue perfusion during the acute phase of hemorrhage. The subsequent recovery in tissue oxygenation may reflect the effectiveness of resuscitation interventions in restoring blood volume, cardiac output, and tissue perfusion. The observation of tissue oxygenation recovery may provide direct and real-time insight into the efficacy of resuscitation treatment.

The tissue oxygenation data collected during hemorrhage resuscitation may be combined with accessory patient vital statistics including blood pressure, pulse rate, and other parameters collected via standard emergency room protocols for comparison and analysis. The combination of tissue oxygenation data with standard vital signs may provide a more comprehensive picture of patient status during resuscitation than either data source alone. The integrated data may allow correlation of tissue oxygenation changes with changes in other physiological parameters, potentially revealing relationships that inform clinical decision-making and improve understanding of the physiological response to hemorrhage and resuscitation.

The tissue oxygenation monitoring during hemorrhage resuscitation may provide information about the body's response to and recovery from hypovolemic shock. The physiological response to hypovolemic shock may involve complex interactions between compensatory mechanisms, tissue oxygen consumption, and cardiovascular function that may be reflected in the tissue oxygenation measurements. The recovery from hypovolemic shock may involve restoration of blood volume, relaxation of compensatory vasoconstriction, and normalization of tissue perfusion that may be tracked through continuous tissue oxygenation monitoring.

The tissue oxygenation measurements during hemorrhage resuscitation may potentially lead to more efficient use of life-saving resources in the future by providing information about the effectiveness of different resuscitation approaches. The correlation of tissue oxygenation responses with specific resuscitation interventions may allow identification of approaches that are most effective in restoring tissue perfusion for different patient populations or injury types. The insights gained from tissue oxygenation monitoring during hemorrhage resuscitation may inform the development of improved resuscitation protocols that optimize the use of blood products, fluids, and other resources while achieving adequate tissue perfusion.

The NIRS system may provide tissue oxygenation monitoring that has potential to improve hemorrhage intervention and management in both military and civilian settings, particularly in environments with reduced or limited medical access. The wearable and low cube form factor of the NIRS system may allow deployment in far forward field settings where traditional monitoring equipment may not be available or practical. The ability to monitor tissue oxygenation during patient transport across different roles of care may provide continuous assessment of patient status throughout the evacuation and treatment process.

The tissue oxygenation monitoring capability may be particularly valuable in settings where continuous blood pressure monitoring is unavailable or impractical. In pre-hospital settings, during patient transport, or in mass casualty situations where monitoring resources are limited, the NIRS system may provide tissue oxygenation measurements that serve as a surrogate indicator of perfusion status. The tissue oxygenation trends may allow triage decisions and resuscitation prioritization based on objective physiological data rather than relying solely on clinical assessment of patient condition.

The tissue oxygenation measurements provided by the NIRS system during hemorrhage resuscitation may be compared to computational models or simulated models to validate research findings and advise future clinical cases. The comparison of clinical tissue oxygenation data with model predictions may allow validation of the models and refinement of model parameters to improve accuracy. The validated models may then be used to predict patient responses to different resuscitation approaches, potentially allowing optimization of resuscitation strategies before implementation in clinical practice.

The NIRS system may monitor patients with cardiogenic shock and classify patients according to Society for Cardiovascular Angiography and Interventions (SCAI) Stages A through E based on tissue oxygenation and perfusion measurements. Cardiogenic shock represents a state characterized by low cardiac output resulting in life-threatening end-organ hypoperfusion and hypoxia. Diagnostic criteria for cardiogenic shock may include persistent hypotension and signs of compromised end-organ perfusion such as cold extremities, oliguria, or altered mental status despite correction of co-existent hypovolemia. The NIRS monitoring wearable system may provide continuous tissue oxygenation and perfusion monitoring that can aid in the assessment and classification of cardiogenic shock patients according to the SCAI staging system.

The SCAI staging system may categorize cardiogenic shock patients into five stages designated as Stage A through Stage E based on the severity of hemodynamic compromise and clinical presentation. Stage A may represent patients who are at risk for cardiogenic shock but do not yet exhibit signs of hemodynamic compromise. Stage B may represent patients who exhibit beginning hemodynamic compromise with hypotension or tachycardia without hypoperfusion. Stage C may represent patients with established cardiogenic shock who exhibit hypoperfusion requiring intervention beyond volume resuscitation. Stage D may represent patients with deteriorating cardiogenic shock who are not responding to initial interventions. Stage E may represent patients with extremis cardiogenic shock who are experiencing cardiac arrest or are supported by multiple interventions. The NIRS monitoring wearable system may provide tissue oxygenation and perfusion measurements that correlate with these SCAI stages and may support classification of patients based on objective physiological data.

The classification of cardiogenic shock patients according to SCAI stages based on tissue oxygenation and perfusion measurements may involve analysis of multiple physiological parameters measured by the NIRS system. The StO$_2$ measurements may reflect the adequacy of oxygen delivery to tissues and may decrease as cardiogenic shock progresses from early stages to more severe stages. The perfusion measurements derived from the optical signals may reflect microvascular blood flow and may indicate the degree of end-organ hypoperfusion associated with each SCAI stage. The combination of tissue oxygenation and perfusion measurements may provide a multi-dimensional assessment of cardiogenic shock severity that supports classification according to the SCAI staging system.

The NIRS system may differentiate between SCAI Stage A patients who are at risk for cardiogenic shock and SCAI Stage B patients who exhibit beginning hemodynamic compromise based on tissue oxygenation and perfusion measurements. Stage A patients may exhibit tissue oxygenation and perfusion values that remain within normal ranges because hemodynamic compromise has not yet developed. Stage B patients may exhibit early decreases in tissue oxygenation or perfusion that reflect the beginning of hemodynamic compromise, even when blood pressure and other traditional vital signs remain within acceptable ranges. The tissue oxygenation and perfusion measurements may provide earlier detection of the transition from at-risk status to beginning hemodynamic compromise than blood pressure monitoring alone.

The NIRS system may differentiate between SCAI Stage B patients with beginning hemodynamic compromise and SCAI Stage C patients with established cardiogenic shock based on tissue oxygenation and perfusion measurements. Stage C patients may exhibit more pronounced decreases in tissue oxygenation and perfusion compared to Stage B patients, reflecting the progression from early hemodynamic compromise to established shock with hypoperfusion. The tissue oxygenation measurements in Stage C patients may indicate inadequate oxygen delivery to tissues that requires intervention beyond volume resuscitation. The perfusion measurements may indicate microvascular dysfunction that accompanies the macrovascular hemodynamic compromise characteristic of established cardiogenic shock.

The NIRS system may differentiate between SCAI Stage C patients with established cardiogenic shock and SCAI Stage D patients with deteriorating cardiogenic shock based on tissue oxygenation and perfusion measurements. Stage D patients may exhibit continued decreases in tissue oxygenation and perfusion despite initial interventions, indicating failure to respond to treatment and progression of shock severity. The tissue oxygenation trends in Stage D patients may show continued decline or failure to improve following interventions that would be expected to restore tissue perfusion in responsive patients. The perfusion measurements may indicate worsening microvascular dysfunction that accompanies the deterioration of cardiovascular function in Stage D patients.

The NIRS system may identify SCAI Stage E patients with extremis cardiogenic shock based on tissue oxygenation and perfusion measurements that indicate severe end-organ hypoperfusion. Stage E patients may exhibit the lowest tissue oxygenation values and the most severely compromised perfusion measurements, reflecting the extreme hemodynamic compromise associated with cardiac arrest or near-arrest states. The tissue oxygenation measurements in Stage E patients may indicate inadequate oxygen delivery that threatens immediate survival without aggressive intervention. The perfusion measurements may indicate near-complete failure of microvascular blood flow that accompanies the cardiovascular collapse characteristic of extremis cardiogenic shock.

The SCAI staging system may not utilize a consistent quantitative measure to categorize patient cardiogenic shock stage in current clinical practice. The NIRS system may provide quantitative tissue oxygenation and perfusion measurements that can serve as objective criteria for SCAI stage classification. The quantitative measurements may reduce variability in stage classification that may result from subjective clinical assessment and may provide reproducible criteria that can be applied consistently across different clinical settings and providers. The objective quantitative criteria based on tissue oxygenation and perfusion measurements may complement the clinical assessment criteria currently used for SCAI staging.

The patient populations with greater risk of cardiogenic shock may include patients with pre-existing systolic heart failure and patients with large acute myocardial infarction. The NIRS system may be applied to these at-risk patient populations to provide continuous monitoring of tissue oxygenation and perfusion that can detect early signs of cardiogenic shock development. The early detection capability may allow intervention before patients progress from at-risk status (SCAI Stage A) to established cardiogenic shock (SCAI Stage C or higher), potentially improving outcomes by enabling earlier treatment.

The NIRS system may provide early detection of microvascular changes due to changes in cardiac output, stroke volume, and blood pressure during simulated hypovolemic shock protocols. Simulated hypovolemic shock protocols may include lower body negative pressure (LBNP) testing that uses a sealed vacuum chamber applied below a patient's waist to simulate the effects of hypovolemia on the upper body. The LBNP system may accurately reproduce the same effects of blood volume loss with safe and reversible measures, allowing investigation of the physiological responses to hypovolemia without the risks associated with actual blood loss.

During simulated hypovolemic shock protocols using LBNP, the NIRS monitoring wearable system may detect early microvascular changes that occur as cardiac output decreases in response to the simulated hypovolemia. As negative pressure is applied to the lower body, blood may pool in the lower extremities, reducing venous return to the heart and decreasing cardiac output. The decreased cardiac output may result in reduced tissue perfusion that can be detected through changes in tissue oxygenation measured by the NIRS monitoring wearable system. The tissue oxygenation changes may occur before significant changes in blood pressure are observed, providing early detection of the hemodynamic effects of simulated hypovolemia.

The NIRS system may detect early microvascular changes due to changes in stroke volume during simulated hypovolemic shock protocols. As venous return decreases during LBNP testing, stroke volume may decrease due to reduced ventricular filling. The decreased stroke volume may result in reduced cardiac output and tissue perfusion even before compensatory tachycardia develops to maintain cardiac output. The tissue oxygenation measurements provided by the NIRS system may detect the perfusion changes associated with decreased stroke volume, providing early indication of hemodynamic compromise.

The NIRS system may detect early microvascular changes due to changes in blood pressure during simulated hypovolemic shock protocols. As the simulated hypovolemia progresses and compensatory mechanisms become insufficient to maintain blood pressure, mean arterial pressure may decrease. The decreased blood pressure may result in reduced tissue perfusion pressure and decreased oxygen delivery to tissues. The tissue oxygenation measurements may track with blood pressure changes during the simulated hypovolemic shock protocol, demonstrating the correlation between systemic hemodynamics and tissue oxygenation that allows the NIRS system to serve as a surrogate indicator of perfusion status.

The early detection of microvascular changes during simulated hypovolemic shock protocols may demonstrate the capability of the NIRS system to identify hemodynamic compromise before traditional vital signs indicate significant abnormality. During LBNP testing, the NIRS system may show reduction of tissue oxygenation and elevation of heart rate that follow predicted trends as the simulated hypovolemia progresses. The tissue oxygenation changes may precede significant blood pressure changes because compensatory mechanisms may initially maintain blood pressure while tissue perfusion is already compromised. The early detection capability demonstrated during simulated hypovolemic shock protocols may translate to clinical applications where early identification of hemodynamic compromise can enable earlier intervention.

The NIRS system may demonstrate early detection of microvascular changes at presyncope during simulated hypovolemic shock protocols. Presyncope represents the point at which compensatory mechanisms are no longer able to maintain adequate cerebral perfusion, resulting in symptoms such as lightheadedness, visual disturbances, or impending loss of consciousness. The tissue oxygenation measurements at multiple body sites including the sternum, upper distal and proximal limb, and forehead may correlate with hypovolemic status and may show characteristic changes before and at presyncope. The detection of tissue oxygenation changes at presyncope may demonstrate the sensitivity of the NIRS system to hemodynamic compromise that threatens consciousness and organ function.

The rapid recovery of tissue oxygenation following release of the LBNP system may demonstrate the responsiveness of the NIRS system to changes in hemodynamic status. When the negative pressure is released and blood redistributes from the lower extremities back to the central circulation, venous return, cardiac output, and tissue perfusion may rapidly recover. The tissue oxygenation measurements may show corresponding rapid recovery to baseline levels, demonstrating that the NIRS monitoring wearable system can track both deterioration and recovery of tissue perfusion during hemodynamic challenges.

The NIRS system may be used for monitoring patients with cardiogenic shock, specifically monitoring hemodynamic changes that occur during cardiogenic shock, including decreased cardiac output, decreased stroke volume, and decreased blood pressure. The NIRS system can detect early microvascular changes during simulated hypovolemic shock as well as early microvascular changes in patients developing or experiencing cardiogenic shock.

The NIRS system may assess cardiogenic shock risk by using multiple model systems tested in animals and humans that are validated with current clinical standard equipment. The validation studies may demonstrate that the NIRS system can accurately measure tissue oxygenation and detect hemodynamic changes across different experimental conditions and patient populations. The validation against clinical standard equipment may provide confidence that the tissue oxygenation and perfusion measurements provided by the NIRS system are accurate and clinically meaningful for assessment of cardiogenic shock patients.

The monitoring of tissue oxygenation and perfusion in cardiogenic shock patients may help identify tissue hypoxia or compromised microvascular perfusion earlier than traditional monitoring methods, allowing for timely intervention. Current tools such as blood lactate measurements may be lagging indicators of inadequate tissue oxygenation and perfusion because elevations of lactate indicate a shift to anaerobic metabolism due to hypoperfusion and tissue hypoxia that has already occurred. Since lactate measurement is invasive and requires a specific blood draw, lactate is most commonly prescribed for patients once cardiogenic shock is established, missing the transition from compensated states to those of decompensation and cardiogenic shock. The continuous tissue oxygenation monitoring provided by the NIRS monitoring wearable system may detect inadequate tissue perfusion before lactate elevation occurs, enabling earlier intervention.

The NIRS system may provide early detection of cardiogenic shock in patients by continuously monitoring tissue perfusion trends. The continuous monitoring capability may allow detection of gradual changes in tissue oxygenation and perfusion that indicate developing cardiogenic shock before acute decompensation occurs. The trend analysis of tissue oxygenation measurements may identify patients whose tissue perfusion is deteriorating over time, even when individual measurements remain within acceptable ranges. The early detection of deteriorating tissue perfusion trends may allow proactive intervention to prevent progression to more severe stages of cardiogenic shock.

The NIRS system may use measured changes in physiological data to predict deterioration of a patient's clinical state to enable earlier intervention in cardiogenic shock management. The predictive capability may involve analysis of tissue oxygenation trends, perfusion measurements, and other physiological parameters to identify patterns that precede clinical deterioration. The prediction of clinical deterioration may allow clinicians to intervene before patients progress to more severe SCAI stages, potentially improving outcomes by enabling treatment at earlier stages of cardiogenic shock when interventions may be more effective.

The combination of tissue oxygenation monitoring with cardiac hemodynamic measurements including cardiac output and stroke volume may provide a comprehensive assessment of cardiovascular function in cardiogenic shock patients. The NIRS system may incorporate impedance cardiography capabilities that allow measurement of stroke volume and cardiac output in addition to tissue oxygenation. The simultaneous measurement of cardiac pumping function and tissue oxygen delivery may provide complementary information about cardiovascular performance that supports assessment of cardiogenic shock severity and response to treatment.

The NIRS system may combine near-infrared spectroscopy, photoplethysmography, electrocardiography, and impedance cardiography to enable simultaneous measurement of tissue perfusion, cardiac electrical function, and cardiac mechanical function. The combination of these sensing modalities may provide a comprehensive assessment of the cardiovascular system that is not available from any single monitoring technique alone. The unified readout from multiple sensing modalities may allow timely intervention for patients with various stages of cardiogenic shock by providing integrated information about tissue perfusion status and cardiac function.

The electrocardiography and impedance cardiography capabilities of the NIRS system may be used individually or in combination to assess cardiac function in cardiogenic shock patients. Electrocardiography may provide information about cardiac rhythm, conduction abnormalities, and ischemic changes that may contribute to or result from cardiogenic shock. Impedance cardiography may provide information about cardiac mechanical function including stroke volume and cardiac output that directly reflects the pumping capability of the heart. The combination of electrical and mechanical cardiac assessment with tissue oxygenation monitoring may provide a multi-dimensional characterization of cardiovascular status in cardiogenic shock patients.

The NIRS system may provide real-time, noninvasive, and continuous monitoring that may prevent degradation in a hemodynamically stable patient who is not experiencing signs of cardiogenic shock but is at risk for cardiogenic shock development. The continuous monitoring capability may detect early changes in tissue oxygenation and perfusion that indicate developing hemodynamic compromise before clinical signs of cardiogenic shock become apparent. The early detection may allow intervention during the transition from compensated states to decompensation, potentially preventing progression to established cardiogenic shock.

The most precise method to continuously monitor cardiovascular performance in cardiogenic shock patients, pulmonary artery catheterization, is an invasive procedure with controversial use given the lack of evidence supporting a change in mortality with pulmonary artery catheterization use and the risk of complications from the procedure and subsequent use. The NIRS system may provide a noninvasive alternative for continuous cardiovascular monitoring that avoids the risks associated with pulmonary artery catheterization while providing tissue oxygenation and perfusion information that reflects cardiovascular performance. The noninvasive nature of the NIRS system may allow application to a broader range of patients, including those for whom the risks of invasive monitoring may not be justified.

While noninvasive monitors exist for cardiovascular assessment, none have been validated in the ongoing assessment of patients with potential or established cardiogenic shock prior to the NIRS system. The validation of the NIRS system during simulated hypovolemic shock protocols and in clinical studies of heart failure patients may support the application of the system for ongoing assessment of cardiogenic shock patients. The demonstrated capability to detect early microvascular changes due to changes in cardiac output, stroke volume, and blood pressure may indicate that the NIRS system can provide clinically meaningful information for cardiogenic shock assessment and management.

Cardiogenic shock carries a poor prognosis and is the leading cause of death in patients with acute myocardial infarction. Cardiogenic shock patients may be treated with vasopressors to improve mean arterial blood pressure and perfusion, or with inotropic agents to improve cardiac output. Patients may undergo percutaneous coronary intervention in the case of ST-segment elevation myocardial infarction. Despite the partial efficacy of these treatments, cardiogenic shock mortality rates remain high at approximately 35 to 50 percent and have not meaningfully changed in more than 20 years of study. The NIRS monitoring wearable system may contribute to improved outcomes by enabling earlier detection of cardiogenic shock and more precise monitoring of treatment response, potentially allowing optimization of therapy before irreversible organ damage occurs.

Implementing systems to continuously monitor cardiovascular function and hemodynamic parameters may improve the outcomes of cardiogenic shock patients by enabling earlier intervention. The NIRS monitoring wearable system may provide the continuous monitoring capability that allows detection of hemodynamic changes as they develop rather than after clinical deterioration has occurred. The real-time tissue oxygenation and perfusion measurements may provide immediate feedback about the effectiveness of interventions, allowing rapid adjustment of treatment strategies to optimize cardiovascular support and tissue perfusion.

The NIRS system may assess changes in patient cardiogenic shock status using tissue oxygenation and perfusion measurements. By studying patients and applying the NIRS system early in the course of care, the correlation between the measures captured by the device and the actual clinical course may be observed. The correlation analysis may allow assessment of whether the NIRS system metrics provide a leading indicator of worsening cardiogenic shock that can enable proactive intervention before clinical deterioration becomes apparent through traditional monitoring methods.

The data acquired from cardiogenic shock patients using the NIRS system may be used to develop and refine a predictive model of shock status from individual patient response trends. The predictive model may identify patients with the greatest likelihood of developing advanced cardiogenic shock based on trend changes in tissue oxygenation, stroke volume, cardiac output, and other physiological parameters. The predictive model may enable physicians to treat patients preemptively and avoid worsening in the clinical stage by identifying deterioration trends before they result in progression to more severe SCAI stages.

The trend changes in tissue oxygenation and other vital statistics measured by the NIRS system may be observed and used to predict changes in the patient's cardiogenic shock stage. The prediction of stage changes may involve analysis of the rate and direction of tissue oxygenation trends, the relationship between tissue oxygenation changes and cardiac function parameters, and the response of tissue oxygenation to interventions. The prediction of impending stage progression may allow clinicians to intensify treatment before the patient actually progresses to a more severe stage, potentially preventing the morbidity and mortality associated with advanced cardiogenic shock.

The NIRS system may evaluate the feasibility of using noninvasive monitoring devices to appropriately characterize cardiogenic shock patient state. Using the NIRS system in patients with varying stages of cardiogenic shock from at-risk Stage A to established cardiogenic shock Stage C and higher, physiological measurement data including tissue oxygenation, cardiac output, stroke volume, pulse oximetry, pulse rate, and respiratory rate may be gathered throughout treatment. The feasibility evaluation may also assess patient tolerance to the applied sensors and gain usability feedback from clinical staff to support optimization of the monitoring approach for cardiogenic shock applications.

The NIRS system may automatically adapt optical system parameters for changes in patient physiology due to hemorrhage or hypothermia. The automatic adaptation may involve adjustment of light source drive currents, detector gain settings, signal processing parameters, or other optical system parameters based on detected changes in tissue optical properties or signal characteristics that indicate altered physiological state. The automatic adaptation capability may allow the NIRS system to maintain accurate measurements across a range of physiological conditions that may affect tissue optical properties and signal quality.

During hemorrhage, the physiological changes that occur may affect the optical properties of tissue and the characteristics of the optical signals measured by the NIRS system. The reduction in blood volume may result in decreased hemoglobin content in the measured tissue volume, which may affect the absorption of near-infrared light and the amplitude of the detected optical signals. The activation of compensatory vasoconstriction may alter blood distribution in the tissue, potentially affecting the ratio of arterial to venous blood in the measured volume and the resulting tissue oxygenation values. The NIRS system may detect these changes in signal characteristics and may automatically adjust optical parameters to maintain accurate measurements despite the altered physiological state.

The automatic adaptation for hemorrhage-related physiological changes may involve adjustment of light source drive currents to compensate for changes in tissue optical absorption. As hemoglobin content decreases during hemorrhage, the optical absorption of the tissue may decrease, potentially resulting in increased signal amplitudes at the detectors. The NIRS system may detect the increased signal amplitudes and may reduce the light source drive currents to maintain optimal signal levels and prevent detector saturation. Conversely, if vasoconstriction results in reduced blood volume in the measured tissue and decreased signal amplitudes, the system may increase light source drive currents to maintain adequate signal levels for accurate measurements.

The automatic adaptation for hemorrhage-related physiological changes may also involve adjustment of detector gain settings to accommodate changes in signal amplitude. The dynamic gain adjustment capability of the avalanche photodiodes may allow the NIRS system to maintain optimal signal levels across a range of tissue optical properties and hemodynamic states. When signal amplitudes decrease due to hemorrhage-related changes in tissue perfusion, the detector gain may be increased to maintain adequate signal-to-noise ratio. When signal amplitudes increase due to changes in tissue optical properties, the detector gain may be decreased to prevent signal saturation and maintain linear detector response.

During hypothermia, the physiological changes that occur may also affect the optical properties of tissue and the characteristics of the optical signals measured by the NIRS system. Hypothermia may result in peripheral vasoconstriction as the body attempts to conserve heat by reducing blood flow to the skin and extremities. The vasoconstriction may reduce blood volume in the superficial tissue layers, potentially affecting the amplitude and characteristics of the optical signals. Hypothermia may also affect tissue metabolism and oxygen consumption, potentially altering the relationship between tissue oxygenation measurements and underlying physiological state.

The automatic adaptation for hypothermia-related physiological changes may involve adjustment of optical parameters to compensate for the effects of vasoconstriction on signal characteristics. The reduced blood volume in superficial tissue layers during hypothermia may result in decreased pulsatile signal amplitude, potentially affecting the accuracy of pulse oximetry measurements that rely on detection of pulsatile arterial blood signals. The NIRS system may detect the reduced pulsatile signal amplitude and may adjust signal processing parameters to maintain accurate $SpO_2$ measurements despite the reduced signal quality. The system may also adjust light source drive currents or detector gain settings to compensate for changes in overall signal amplitude associated with hypothermia-induced vasoconstriction.

The automatic adaptation for changes in patient physiology may involve continuous monitoring of signal quality metrics and automatic adjustment of optical parameters when signal quality degrades below acceptable thresholds. The signal quality metrics may include signal amplitude, signal-to-noise ratio, pulsatile signal amplitude, and the presence of artifacts or interference. When the signal quality metrics indicate that the current optical parameters are not providing optimal signal quality for the current physiological state, the NIRS monitoring wearable system may automatically adjust the optical parameters to improve signal quality and maintain measurement accuracy.

The automatic adaptation capability may allow the NIRS system to maintain accurate measurements in patients suffering from hemorrhage and other mechanisms of shock, hypothermia, and other peripheral vasoconstricting conditions. The adaptation may occur without requiring manual intervention by clinical staff, allowing the system to continue providing accurate physiological measurements even as the patient's condition changes during treatment. The automatic adaptation may be particularly valuable in emergency and critical care settings where patient physiology may change rapidly and where clinical staff may be focused on life-saving interventions rather than monitoring equipment adjustment.

The automatic adaptation for various skin tones including shading densities may also be implemented by the NIRS system. Different skin tones and shading densities may exhibit different optical properties due to variations in melanin content, which affects the absorption of light in the visible and near-infrared wavelength ranges. The NIRS system may detect the optical characteristics of the tissue being measured during an initial calibration period and may adjust optical parameters to optimize signal quality and measurement accuracy for the specific skin characteristics encountered.

The calibration methods implemented by the NIRS system may include initial calibration sequences performed when the sensor is first applied to the user's body. The initial calibration sequence may involve measurement of baseline optical signals at multiple wavelengths and source-detector distances to characterize the optical properties of the tissue being measured. The baseline measurements may be used to determine appropriate light source drive currents, detector gain settings, and signal processing parameters for the specific tissue site and user characteristics. The calibration parameters determined during the initial calibration sequence may be stored and applied to subsequent measurements during the monitoring session.

The calibration methods may also include ongoing calibration updates that occur during continuous monitoring to track changes in tissue characteristics or sensor-tissue coupling that may affect measurement accuracy. The ongoing calibration updates may involve periodic reassessment of signal quality metrics and adjustment of optical parameters as needed to maintain optimal signal quality. The ongoing calibration may also involve comparison of measurements from different source-detector distances or different wavelengths to detect and compensate for changes in tissue optical properties that may occur during extended monitoring periods.

The validation and calibration methods implemented by the NIRS system may support accurate physiological measurements across a range of patient populations, physiological conditions, and clinical applications. The validation against clinical standard devices may demonstrate that the NIRS system provides measurements that agree with established reference devices. The validation using controlled physiological challenges including ROBD and LBNP protocols may demonstrate that the system can accurately track physiological changes across a range of conditions. The automatic adaptation for changes in patient physiology and the calibration methods may ensure that the system maintains accurate measurements even as patient condition changes during monitoring.

The NIRS system may implement statistical analysis methods for processing and interpreting physiological data collected during cardiovascular assessments. The statistical analysis methods may be applied to tissue oxygenation measurements, slope parameters extracted from vascular occlusion testing, and other physiological metrics to characterize patient populations and identify differences between groups with different disease severity levels. The statistical analysis may be performed by the processor of the NIRS monitoring wearable system, by external computing devices that receive data from the system, or by data analysis software that processes stored physiological measurements.

The NIRS system may use Shapiro-Wilk tests to assess normality of collected data prior to applying parametric statistical methods. The Shapiro-Wilk test may be a statistical test that evaluates whether a sample of data comes from a normally distributed population. The test may calculate a test statistic based on the correlation between the data and the corresponding normal scores, with the test statistic compared to critical values to determine whether the null hypothesis of normality can be rejected. The Shapiro-Wilk test may be applied to tissue oxygenation measurements, reperfusion slope values, deoxygenation rate measurements, and other physiological parameters collected by the NIRS system to determine whether the data distribution meets the assumptions required for parametric statistical analysis.

The assessment of data normality using Shapiro-Wilk tests may inform the selection of appropriate statistical methods for subsequent analysis. When the Shapiro-Wilk test indicates that the data are normally distributed, parametric statistical methods that assume normal distribution may be applied to the data. When the Shapiro-Wilk test indicates that the data are not normally distributed, non-parametric statistical methods that do not require the assumption of normality may be selected instead. The appropriate selection of statistical methods based on data distribution characteristics may improve the validity and reliability of statistical conclusions drawn from the physiological measurements.

The Shapiro-Wilk test may be performed separately for each group of subjects being compared, such as subjects classified into different NYHA heart failure classes. The normality assessment for each group may determine whether the data within that group follow a normal distribution, which may be relevant for selecting appropriate group comparison methods. In some cases, the data may be normally distributed within some groups but not within others, which may require consideration of robust statistical methods that can accommodate mixed distribution characteristics across groups.

The NIRS system may use Spearman ranked tests for correlation analysis when the assumption of normality is not maintained or when the relationship between variables may be monotonic but not linear. The Spearman ranked correlation test may be a non-parametric statistical method that assesses the strength and direction of the monotonic relationship between two variables. The Spearman test may calculate a correlation coefficient based on the ranks of the data values rather than the actual values, making the test robust to outliers and non-normal distributions. The Spearman ranked test may be applied to assess correlations between tissue oxygenation parameters and clinical measures such as blood pressure, NYHA classification, or distance traveled during the six-minute walk test.

The Spearman ranked correlation coefficient may range from negative one to positive one, with values near positive one indicating a strong positive monotonic relationship, values near negative one indicating a strong negative monotonic relationship, and values near zero indicating no monotonic relationship between the variables. The statistical significance of the Spearman correlation coefficient may be assessed using a p-value that indicates the probability of observing the calculated correlation coefficient if no true correlation exists in the population. The Spearman ranked test may be used to assess within-group correlations between different physiological metrics and across-group correlations between tissue oxygenation parameters and disease severity measures.

The NIRS system may use Pearson tests for correlation analysis when the assumption of normality is maintained and when the relationship between variables is expected to be linear. The Pearson correlation test may be a parametric statistical method that assesses the strength and direction of the linear relationship between two continuous variables. The Pearson test may calculate a correlation coefficient based on the covariance of the two variables divided by the product of their standard deviations. The Pearson correlation coefficient may be more powerful than the Spearman coefficient for detecting linear relationships when the normality assumption is satisfied, but may be less robust to outliers and non-normal distributions.

The selection between Spearman ranked tests and Pearson tests for correlation analysis may be based on the results of the Shapiro-Wilk normality assessment and on the expected nature of the relationship between the variables being analyzed. When the Shapiro-Wilk test indicates that both variables are normally distributed and the relationship is expected to be linear, the Pearson test may be selected for correlation analysis. When the Shapiro-Wilk test indicates that one or both variables are not normally distributed, or when the relationship may be monotonic but not strictly linear, the Spearman ranked test may be selected instead.

The correlation analysis using Spearman ranked tests or Pearson tests may be applied to assess relationships between tissue oxygenation metrics and standard clinical metrics collected during cardiovascular assessments. The tissue oxygenation metrics may include reperfusion slopes from vascular occlusion testing, deoxygenation rates during physical activity, baseline tissue oxygenation values, and tissue oxygenation changes during postural transitions. The standard clinical metrics may include blood pressure measurements, pulse rate, pulse oximetry values, and distance traveled during the six-minute walk test. The correlation analysis may identify relationships between the tissue oxygenation measurements provided by the NIRS system and established clinical measures of cardiovascular function.

The NIRS system may use analysis of variance (ANOVA) tests for group-wise comparisons between subjects classified into different NYHA heart failure classes. The ANOVA test may be a parametric statistical method that compares the means of three or more groups to determine whether there are statistically significant differences between the group means. The ANOVA test may partition the total variance in the data into variance between groups and variance within groups, with the ratio of these variance components used to calculate an F-statistic that is compared to critical values to assess statistical significance.

The ANOVA test may be applied to compare tissue oxygenation parameters across NYHA Class I, Class II, and Class III heart failure patients. The comparison may assess whether the mean reperfusion slopes, mean deoxygenation rates, mean baseline tissue oxygenation values, or other tissue oxygenation parameters differ significantly between the NYHA classes. The ANOVA test may provide an overall assessment of whether differences exist among the groups, but may not identify which specific pairs of groups differ from each other.

The NIRS system may use Tukey's multiple comparison tests following ANOVA to identify which specific pairs of NYHA classes differ significantly from each other. Tukey's multiple comparison test may be a post-hoc analysis method that compares all possible pairs of group means while controlling the family-wise error rate to account for the multiple comparisons being performed. The Tukey test may calculate a critical difference value based on the studentized range distribution, with pairs of group means that differ by more than the critical difference considered statistically significantly different.

The combination of ANOVA tests with Tukey's multiple comparison tests may provide a comprehensive assessment of differences in tissue oxygenation parameters between NYHA heart failure classes. The ANOVA test may first determine whether any significant differences exist among the groups, and if significant differences are detected, the Tukey test may identify which specific pairs of groups differ. The Tukey test may compare NYHA Class I versus Class II, Class I versus Class III, and Class II versus Class III to determine which class comparisons show statistically significant differences in tissue oxygenation parameters.

The NIRS system may use multiple unpaired t-tests with Welch's correction as an alternative to ANOVA for group-wise comparisons between NYHA classes. The unpaired t-test may be a parametric statistical method that compares the means of two independent groups to determine whether there is a statistically significant difference between the group means. Welch's correction may be applied to the t-test to account for unequal population variances between the groups being compared, which may occur when the groups have different sample sizes or different degrees of variability in the measured parameters.

Welch's correction may modify the calculation of the t-statistic and the degrees of freedom used for determining statistical significance to account for unequal variances between groups. The standard unpaired t-test may assume that the two groups being compared have equal population variances, and violation of this assumption may lead to inaccurate p-values and incorrect conclusions about statistical significance. Welch's correction may provide more accurate results when the equal variance assumption is not satisfied, making the test more robust for comparing groups that may have different variability characteristics.

The multiple unpaired t-tests with Welch's correction may be applied to compare tissue oxygenation parameters between each pair of NYHA classes. The comparisons may include NYHA Class I versus Class II, Class I versus Class III, and Class II versus Class III, with each comparison performed using a separate t-test with Welch's correction. The multiple t-test approach may provide flexibility in comparing specific pairs of groups of interest, but may require adjustment for multiple comparisons to control the overall Type I error rate when multiple tests are performed.

The selection between ANOVA with Tukey's multiple comparison tests and multiple unpaired t-tests with Welch's correction may depend on the specific analysis requirements and the characteristics of the data being analyzed. The ANOVA approach may be preferred when comparing three or more groups simultaneously and when the assumption of equal variances across groups is satisfied. The multiple t-tests with Welch's correction may be preferred when comparing specific pairs of groups, when the groups have unequal variances, or when the sample sizes differ substantially between groups.

The statistical analysis methods may be applied to calculate mean, median, and standard deviation for each NYHA class group and each collected metric. The mean may represent the arithmetic average of the measured values within each group, providing a measure of central tendency. The median may represent the middle value when the data are sorted in order, providing a measure of central tendency that is robust to outliers. The standard deviation may represent the dispersion of the data around the mean, providing a measure of variability within each group.

The calculation of mean, median, and standard deviation for each group may provide descriptive statistics that characterize the distribution of tissue oxygenation parameters within each NYHA class. The descriptive statistics may be presented along with the results of inferential statistical tests to provide a comprehensive summary of the data. The standard error of the mean may also be calculated to characterize the precision of the mean estimate, with the standard error used to construct confidence intervals and to assess the reliability of group mean comparisons.

The statistical analysis methods may be applied to datasets separated by assessment protocols including vascular occlusion testing, six-minute walk test, and postural reactivity testing. The separation of datasets by protocol may allow protocol-specific analysis of tissue oxygenation parameters and comparison of results across different cardiovascular stressor conditions. The statistical analysis may identify which assessment protocols provide the most discriminating tissue oxygenation parameters for differentiating between NYHA heart failure classes.

The statistical analysis methods may incorporate the timing information recorded during cardiovascular assessments to synchronize tissue oxygenation measurements with clinical events and blood pressure measurements. The synchronization may allow extraction of tissue oxygenation parameters at specific time points relative to protocol events such as cuff inflation, cuff release, test onset, or postural transition. The time-synchronized analysis may improve the accuracy of slope calculations and other kinetic parameters by ensuring that the analysis windows correspond to the intended phases of each assessment protocol.

The statistical significance threshold for the analysis may be set at a p-value of 0.05, indicating that results with p-values less than 0.05 may be considered statistically significant. The 0.05 significance level may represent a conventional threshold that balances the risk of Type I errors (false positive findings) against the risk of Type II errors (false negative findings). The anticipated p-values for comparisons between NYHA classes may be greater than 0.05 in some cases, indicating that larger sample sizes may be needed to achieve statistical significance for detecting differences between groups.

The statistical analysis methods implemented by the NIRS system may support the characterization of heart failure patients based on tissue oxygenation kinetics and the identification of relationships between tissue oxygenation parameters and disease severity. The combination of normality assessment using Shapiro-Wilk tests, correlation analysis using Spearman ranked tests or Pearson tests, and group comparison using ANOVA with Tukey's tests or multiple t-tests with Welch's correction may provide a comprehensive statistical framework for analyzing physiological data collected during cardiovascular assessments. The statistical analysis may support clinical decision-making by identifying tissue oxygenation parameters that differentiate between patients with different heart failure severity levels and by quantifying the relationships between tissue oxygenation measurements and established clinical measures of cardiovascular function.

The NIRS system may be integrated into various medical settings to provide continuous tissue oxygenation and perfusion monitoring across different clinical environments and patient care scenarios. The medical settings where the NIRS system may be deployed include emergency medicine departments, cardiac and intensive care units (C/ICU), outpatient monitoring clinics, primary care facilities, and remote patient locations. The integration of the NIRS monitoring wearable system into these diverse medical settings may leverage the compact form factor, wireless communication capabilities, extended battery life, and robust construction of the system to provide tissue oxygenation monitoring in environments where traditional monitoring equipment may be impractical or unavailable.

The integration of the NIRS system into emergency medicine settings may support rapid assessment and continuous monitoring of patients presenting with acute conditions that affect tissue oxygenation and perfusion. Emergency medicine departments may encounter patients with hemorrhage, shock, respiratory compromise, cardiac emergencies, and other conditions where tissue oxygenation monitoring may provide clinically relevant information. The NIRS system may be applied to patients upon arrival in the emergency department to begin continuous tissue oxygenation monitoring while other diagnostic and therapeutic interventions are performed. The unobtrusive placement of the sensor on locations such as the thigh may allow monitoring to continue without interfering with emergency medical procedures performed on the chest, abdomen, or upper extremities.

The emergency medicine integration may support triage decisions by providing objective physiological data about tissue perfusion status. In mass casualty situations or during periods of high patient volume, the tissue oxygenation measurements provided by the NIRS system may help emergency medicine staff prioritize patients based on physiological status rather than relying solely on clinical assessment. Patients with compromised tissue oxygenation may be identified for more urgent evaluation and treatment, while patients with preserved tissue oxygenation may be monitored while awaiting further assessment. The continuous monitoring capability may also detect deterioration in patient condition that may not be immediately apparent from intermittent vital sign measurements.

The integration of the NIRS system into cardiac and intensive care units may support continuous monitoring of critically ill patients who require close surveillance of cardiovascular function and tissue perfusion. Cardiac and intensive care units may care for patients with cardiogenic shock, heart failure exacerbations, post-cardiac surgery recovery, sepsis, and other conditions where tissue oxygenation monitoring may provide information about the adequacy of oxygen delivery to tissues. The NIRS monitoring wearable system may complement existing monitoring modalities in the C/ICU by providing continuous tissue oxygenation data that reflects microvascular perfusion and oxygen extraction at the tissue level.

The C/ICU integration may support early detection of hemodynamic deterioration by providing continuous tissue oxygenation trends that may change before traditional vital signs indicate significant abnormality. The tissue oxygenation measurements may detect inadequate tissue perfusion that occurs despite maintenance of blood pressure within acceptable ranges, potentially providing earlier warning of developing shock or cardiovascular decompensation. The continuous monitoring capability may also support assessment of response to therapeutic interventions such as fluid administration, vasopressor titration, or inotropic support by providing real-time feedback about the effects of these interventions on tissue perfusion.

The integration of the NIRS system into outpatient monitoring settings may support assessment of patients with chronic cardiovascular conditions during clinic visits and standardized functional assessments. Outpatient monitoring clinics may perform vascular occlusion testing, six-minute walk tests, postural reactivity assessments, and other standardized protocols that evaluate cardiovascular function and exercise capacity. The NIRS system may be applied during these assessments to provide tissue oxygenation kinetics data that complements traditional measures such as blood pressure, heart rate, and distance traveled. The tissue oxygenation data may provide additional information about microvascular function and cardiovascular reserve that may not be captured by traditional assessment methods.

The outpatient monitoring integration may support longitudinal tracking of cardiovascular function in patients with heart failure, peripheral vascular disease, or other chronic conditions. Patients may undergo periodic assessments using the NIRS system to track changes in tissue oxygenation kinetics over time. The longitudinal data may reveal trends in cardiovascular function that indicate disease progression, response to treatment, or need for therapy adjustment. The standardized assessment protocols performed with the NIRS system may provide reproducible measurements that can be compared across multiple clinic visits to assess changes in patient status.

The integration of the NIRS system into primary care settings may support cardiovascular screening and monitoring in the general patient population. Primary care facilities may serve as the first point of contact for patients with cardiovascular symptoms or risk factors, and the NIRS system may provide tissue oxygenation assessment capabilities that support initial evaluation and ongoing monitoring. The compact and easy-to-use design of the NIRS system may allow deployment in primary care settings without requiring specialized training or dedicated monitoring infrastructure.

The primary care integration may support identification of patients who may benefit from referral to cardiology or other specialty services based on tissue oxygenation findings. Patients with abnormal tissue oxygenation kinetics during standardized assessments may be identified for further evaluation, while patients with normal findings may be reassured and monitored with routine follow-up. The tissue oxygenation measurements may provide objective data that supports clinical decision-making about the need for additional testing, specialist referral, or treatment initiation.

The integration of the NIRS system into remote patient locations may support monitoring of patients who are not physically present in healthcare facilities. Remote patient locations may include patient homes, assisted living facilities, rehabilitation centers, rural clinics, and other settings where patients may require cardiovascular monitoring but may not have access to traditional monitoring infrastructure. The wireless communication capabilities of the NIRS system may allow transmission of tissue oxygenation data from remote locations to clinical monitoring systems where healthcare providers can review the data and make clinical decisions.

The remote patient monitoring integration may leverage the extended battery life and autonomous data storage capabilities of the NIRS system to support continuous monitoring over multiple days without requiring frequent intervention. Patients in remote locations may wear the NIRS system during daily activities, with the system continuously recording tissue oxygenation data that can be transmitted to healthcare providers at regular intervals or upon detection of abnormal findings. The remote monitoring capability may allow patients to remain in their home environments while receiving cardiovascular surveillance that would otherwise require hospitalization or frequent clinic visits.

The NIRS system may support remote patient monitoring (RPM) programs that provide ongoing surveillance of patients with chronic cardiovascular conditions. Remote patient monitoring programs may use connected medical devices to collect physiological data from patients in their home environments and transmit the data to healthcare providers for review and clinical decision-making. The NIRS system may be incorporated into RPM programs to provide tissue oxygenation monitoring that complements other remotely monitored parameters such as blood pressure, weight, and symptoms. The tissue oxygenation data may provide information about cardiovascular function and tissue perfusion that supports early detection of clinical deterioration and timely intervention.

The RPM integration may support management of heart failure patients by providing continuous tissue oxygenation monitoring that can detect changes in cardiovascular status between scheduled clinic visits. Heart failure patients may experience gradual decompensation that manifests as changes in tissue oxygenation before symptoms become severe enough to prompt the patient to seek medical attention. The continuous tissue oxygenation monitoring provided by the NIRS system may detect these early changes and alert healthcare providers, allowing intervention before hospitalization becomes necessary. The RPM approach may reduce hospital readmissions and improve outcomes for heart failure patients by enabling proactive management based on objective physiological data.

The RPM integration may also support monitoring of patients recovering from cardiac procedures, patients with peripheral vascular disease, and patients with other cardiovascular conditions that may benefit from continuous tissue oxygenation surveillance. The flexibility of the NIRS system to be placed on multiple body locations may allow monitoring of tissue oxygenation at sites relevant to each patient's specific condition. The wireless data transmission capabilities may allow healthcare providers to receive tissue oxygenation data from multiple patients simultaneously, supporting efficient management of patient populations enrolled in RPM programs.

The NIRS system may support telemedicine integration that enables real-time physician-supported medical decision making for patients who may have low accessibility to healthcare services. Telemedicine platforms may provide video or audio communication between patients and healthcare providers, allowing clinical consultations to occur without requiring in-person visits. The NIRS system may be integrated with telemedicine platforms to provide tissue oxygenation data that healthcare providers can review during telemedicine consultations, enhancing the clinical information available for remote decision-making.

The telemedicine integration may allow healthcare providers to review tissue oxygenation trends, assess cardiovascular function, and make treatment recommendations based on objective physiological data collected by the NIRS system. During telemedicine consultations, healthcare providers may view real-time tissue oxygenation values, review historical trends, and correlate tissue oxygenation findings with patient-reported symptoms and other clinical information. The availability of tissue oxygenation data during telemedicine consultations may improve physician confidence in remote clinical decision-making by providing objective physiological measurements that supplement the clinical history and visual assessment available through video communication.

The telemedicine integration may be particularly valuable for patients with low healthcare accessibility due to geographic distance, mobility limitations, transportation barriers, or other factors that make in-person healthcare visits difficult. Patients in rural areas may live far from healthcare facilities and may face long travel times for routine appointments. Patients with mobility limitations may have difficulty traveling to healthcare facilities for monitoring visits. The combination of the NIRS monitoring wearable system with telemedicine platforms may allow these patients to receive cardiovascular monitoring and clinical consultation without requiring travel to healthcare facilities, improving access to care for underserved populations.

The implementation of advanced medical monitoring capabilities into existing RPM and telemedicine networks may improve physician confidence and patient outcomes by providing a higher volume of quality medical data available at the network edge. The tissue oxygenation measurements provided by the NIRS system may represent advanced physiological monitoring capabilities that extend beyond the basic vital sign monitoring typically available in RPM programs. The availability of tissue oxygenation data may allow healthcare providers to make more informed clinical decisions and to detect cardiovascular abnormalities that may not be apparent from basic vital sign monitoring alone.

The RPM and telemedicine integration with advanced medical wearables such as the NIRS system may increase patient throughput by allowing physicians to manage a larger group of patients in need. The continuous data collection and automated alert generation capabilities of the NIRS system may allow healthcare providers to efficiently monitor multiple patients simultaneously, with attention directed to patients whose tissue oxygenation data indicates potential clinical concerns. The efficient monitoring approach may allow healthcare providers to extend cardiovascular surveillance to more patients than would be possible with traditional in-person monitoring approaches, improving access to care for patient populations with cardiovascular monitoring needs.

The integration of the NIRS system across emergency medicine, C/ICU, outpatient monitoring, primary care, and remote patient settings may provide continuity of tissue oxygenation monitoring as patients transition between different levels of care. A patient who presents to the emergency department with a cardiovascular emergency may have the NIRS system applied during initial evaluation, with monitoring continuing as the patient is admitted to the C/ICU for intensive management. As the patient stabilizes and transitions to step-down care and eventually outpatient follow-up, the tissue oxygenation monitoring may continue using the same NIRS system, providing longitudinal data that tracks the patient's cardiovascular recovery across the care continuum.

The continuity of monitoring across care settings may support clinical decision-making by providing healthcare providers with tissue oxygenation data that spans the patient's entire clinical course. The longitudinal tissue oxygenation data may reveal patterns of recovery, identify setbacks or complications, and support assessment of treatment effectiveness across different phases of care. The standardized measurement approach provided by the NIRS system may ensure that tissue oxygenation measurements obtained in different care settings are comparable, supporting meaningful interpretation of changes in tissue oxygenation over time.

The integration of the NIRS monitoring wearable system into various medical settings may be facilitated by the system's compatibility with existing clinical workflows and information systems. The wireless communication capabilities may allow the NIRS monitoring wearable system to transmit tissue oxygenation data to clinical monitoring systems, electronic health records, or data management platforms used in each care setting. The data integration may allow tissue oxygenation measurements to be incorporated into the patient's clinical record alongside other physiological measurements, laboratory results, and clinical documentation, supporting comprehensive clinical assessment and decision-making.

The deployment of the NIRS monitoring wearable system across diverse medical settings may leverage the system's robust construction and biocompatible materials to support reliable operation in different clinical environments. The waterproof, crush-resistant, and drop-resistant construction may allow the system to withstand the demands of emergency medicine and critical care environments where equipment may be exposed to fluids, mechanical stress, and handling during urgent clinical situations. The biocompatible materials and adhesive system may support extended wear during outpatient monitoring and remote patient monitoring applications where patients may wear the system for multiple days during daily activities.

The clinical deployment of the NIRS monitoring wearable system across various medical settings may support improved patient care by providing continuous tissue oxygenation monitoring capabilities that complement existing clinical assessment methods. The tissue oxygenation measurements may provide information about microvascular perfusion and oxygen delivery that is not available from traditional vital sign monitoring, potentially enabling earlier detection of cardiovascular abnormalities and more informed clinical decision-making. The integration into emergency medicine, C/ICU, outpatient monitoring, primary care, and remote patient settings may extend the benefits of tissue oxygenation monitoring to diverse patient populations across the healthcare continuum.

*Example Operational Workflow*

The NIRS monitoring wearable system may operate through coordinated interaction of the light sources, detectors, flexible substrate, processor, memory, wireless communication module, and power management circuitry to perform continuous physiological monitoring. The operational workflow may begin with sensor placement and skin preparation, proceed through data acquisition and signal processing, continue with biometric calculation and data storage, and conclude with wireless transmission of physiological measurements to external devices. The coordinated operation of the system components may enable multi-depth tissue analysis, real-time oxygenation monitoring, and clinical assessment across various medical applications including heart failure evaluation, cardiotoxicity monitoring, and shock assessment.

The operational workflow may begin with preparation of the skin surface at the intended sensor placement location. The skin preparation may involve cleaning the skin surface with a gentle cleanser to remove oils, debris, and other substances that could interfere with adhesive bonding or optical coupling between the sensor and the tissue. The cleaning may be performed using soap and warm water or using alcohol wipes and skin prep wipes when soap and water are not available. The cleaned skin may be allowed to air dry completely before adhesive application to ensure optimal adhesive performance and secure sensor attachment during extended monitoring periods.

Following skin preparation, the adhesive may be applied to the NIRS monitoring wearable system by removing the patch-side liner and overlaying the exposed adhesive onto the silicone surface of the sensor. The optical cutouts in the adhesive may be aligned with the optical windows of the sensor to ensure that the light sources and detectors are not obstructed by adhesive material. The adhesive may be pressed firmly onto the sensor surface to ensure uniform contact and secure attachment. The skin-side liner may remain in place during adhesive application to the sensor, protecting the skin-side adhesive surface until the sensor is ready to be applied to the user's body.

The sensor may then be applied to the prepared skin surface at the intended measurement location by removing the skin-side liner and placing the sensor on the skin. The sensor may be pressed firmly onto the skin to ensure uniform contact between the adhesive and the skin surface, with pressure applied around the edge of the optical shroud to ensure good contact in the regions surrounding the optical windows. The flexible substrate connecting the first rigid component and the second rigid component may allow the sensor to conform to the local surface curvature of the body part, improving optical coupling between the sensor and the tissue by reducing air gaps and ensuring consistent contact pressure across the sensor footprint.

Upon sensor placement, the NIRS monitoring wearable system may be energized by pressing the power button for at least three seconds. The power management circuitry may activate the system components and may illuminate the battery indicator LED to indicate the current battery charge level. The processor may initialize the system firmware, configure the optical system parameters, and prepare the memory buffers for data acquisition. The wireless communication module may enter a discoverable state that allows pairing with external devices such as mobile phones or tablets running the NIRS monitoring application software.

The wireless communication module may establish a connection with an external device through a pairing procedure that authenticates and authorizes communication between the NIRS monitoring wearable system and the external device. The pairing procedure may involve selecting the NIRS monitoring wearable system from a list of available devices displayed on the external device, with the system identified by a unique identifier that matches the identifier printed on the sensor label. Upon successful pairing, the wireless communication module may maintain a bidirectional communication link that allows transmission of physiological data from the sensor to the external device and reception of control commands from the external device to the sensor.

Following connection establishment, the NIRS monitoring wearable system may perform an initial calibration sequence to characterize the optical properties of the tissue being measured and to determine appropriate optical system parameters for the specific tissue site and user characteristics. The initial calibration sequence may involve measurement of baseline optical signals at multiple wavelengths and source-detector distances. The processor may analyze the baseline measurements to determine appropriate light source drive currents, detector gain settings, and signal processing parameters that optimize signal quality and measurement accuracy for the specific tissue characteristics encountered.

The initial calibration sequence may include automatic adaptation of optical system parameters for various skin tones and shading densities. The processor may detect the optical characteristics of the tissue through analysis of the baseline optical signals and may adjust the light source drive currents to compensate for variations in tissue optical absorption associated with different melanin content. The detector gain settings may be adjusted to maintain optimal signal levels across the range of tissue optical properties that may be encountered. The calibration parameters determined during the initial calibration sequence may be stored in memory and applied to subsequent measurements during the monitoring session.

Data acquisition may begin when the user initiates a monitoring session through the external device application or through activation of the duty cycle sampling mode on the NIRS monitoring wearable system. The control circuit (e.g., part of the processor(s)) may begin sequentially activating the multiple light sources and collecting corresponding signals from the multiple photodetectors with synchronized timing control. The time-multiplexed operation may activate each light source sequentially rather than simultaneously, with the detector signals sampled during each light source activation period to capture the optical signals while minimizing interference from ambient light or signals from other light sources.

The sequential activation of light sources may follow a predetermined sequence that cycles through all wavelengths and source-detector combinations to collect a complete set of multi-wavelength, multi-distance measurements. The control circuit may generate timing signals that control when each light source is activated and when the corresponding detector signals are sampled. The synchronized timing control may ensure that the detector signals are sampled at the appropriate times relative to the light source activation to capture the optical signals with optimal signal-to-noise ratio.

The adaptive exposure adjustment implemented by the control circuit may adjust the optical exposure parameters during data acquisition to maintain optimal signal levels across different measurement conditions. When the detected signal levels are too low, the control circuit may increase the light source drive current or extend the detector integration time to improve signal strength. When the detected signal levels are too high and approaching saturation, the control circuit may decrease the light source drive current or reduce the detector integration time to prevent signal clipping. The adaptive exposure adjustment may occur automatically based on detected signal levels without requiring manual intervention.

The optical signals collected by the photodetectors may be digitized by analog-to-digital converters and stored in memory buffers for subsequent signal processing. The simultaneous multi-channel acquisition may capture optical signals from all photodetectors at the same time or in rapid succession, enabling synchronized measurement of tissue oxygenation at multiple depths and wavelengths. The digitized signals may be stored in the dedicated buffering regions of the memory, which may maintain continuous data acquisition and streaming operations without data loss during periods of high processing demand or wireless transmission delays.

The processor may apply signal processing algorithms to the optical signals to determine tissue oxygenation levels. The signal processing algorithms may include preprocessing steps such as filtering, baseline correction, and normalization that prepare the raw optical signals for oxygenation calculation. The filtering may remove high-frequency noise and low-frequency drift from the optical signals, improving the signal-to-noise ratio for subsequent analysis. The baseline correction may compensate for variations in optical coupling between the sensor and the tissue surface that may occur during monitoring. The normalization may adjust the signal amplitudes to account for variations in light source output power and detector sensitivity.

The processor may apply machine learning-based artifact rejection to identify and remove or attenuate signal components that do not represent true physiological changes. The machine learning models may be trained on datasets containing labeled examples of artifact-contaminated signals and clean signals, allowing the models to learn patterns that distinguish artifacts from physiological signals. The artifact rejection may identify motion artifacts, ambient light interference, sensor coupling variations, and other non-physiological signal components that could degrade measurement accuracy if not removed from the analysis.

The processor may apply adaptive filtering algorithms combined with inertial measurement unit data for enhanced motion artifact rejection. The accelerometer or other inertial measurement unit may detect motion of the sensor and the user's body, providing acceleration data for three axes that can be correlated with motion-induced variations in the optical signals. The adaptive filtering algorithms may use the inertial measurement unit signals as a reference signal to identify and remove motion artifact components from the optical signals while preserving the physiological signal components. The combination of machine learning-based artifact rejection and adaptive filtering with inertial measurement unit data may provide enhanced motion artifact rejection compared to either approach alone.

Following artifact rejection, the processor may apply differential path length factor corrections to the optical signals to account for variations in optical path lengths through tissue at different wavelengths and source-detector separations. The differential path length factor represents the ratio of the actual optical path length through tissue to the geometric source-detector distance, accounting for the increased path length that results from multiple scattering of light in biological tissue. The processor may store differential path length factor values for each wavelength and source-detector distance combination, and may apply these corrections during the signal processing to convert the measured optical attenuation values into tissue oxygenation estimates.

The processor may execute multi-depth tissue analysis algorithms that process signals from detectors at different source-detector distances to determine oxygenation levels at corresponding tissue depths. The signals from detectors positioned at the 8 mm source-detector distance may provide information about shallow tissue oxygenation, while signals from detectors positioned at the 35 mm and 40 mm source-detector distances may provide information about deeper tissue oxygenation. The multi-depth tissue analysis algorithms may apply depth-specific differential path length factor corrections to the signals from each source-detector distance and may account for the different tissue volumes sampled at each distance.

The processor may apply spectroscopic analysis techniques with real-time spectral deconvolution to determine tissue oxygenation from the multi-wavelength optical measurements. The spectral deconvolution may decompose the multi-wavelength optical measurements into the individual chromophore contributions by solving a system of equations that relates the measured optical attenuation at each wavelength to the known absorption spectra of oxygenated hemoglobin and deoxygenated hemoglobin and their unknown concentrations in the tissue. The tissue oxygen saturation may be calculated as the ratio of oxygenated hemoglobin concentration to total hemoglobin concentration.

The processor may calculate pulse oximetry values from the pulsatile components of the optical signals detected at the shorter source-detector distances. The pulse oximetry calculation may analyze the ratio of pulsatile signal amplitudes at red and near-infrared wavelengths, using the different absorption characteristics of oxygenated and deoxygenated hemoglobin at these wavelengths to calculate arterial oxygen saturation. The pulse rate may be derived from the periodic oscillations in the optical signals that correspond to cardiac pulsations, with the pulse rate calculated by detecting the peaks or other characteristic features in the photoplethysmography waveform and measuring the time intervals between successive cardiac cycles.

The processor may calculate respiratory rate from respiratory modulations in the optical signals. The respiratory modulations may appear as low-frequency variations in the photoplethysmography waveform amplitude or baseline that correspond to the respiratory cycle. The respiratory rate may be calculated by analyzing the frequency content of these respiratory modulations or by detecting individual respiratory cycles in the signal.

The processor may calculate additional biometric properties using algorithms trained on multi-modal physiological data. The biometric properties may include perfusion indices derived from the photoplethysmography signal amplitude, blood volume estimates derived from total hemoglobin content measured by near-infrared spectroscopy, and hemodynamic parameters derived from analysis of the optical signal characteristics. When impedance cardiography capabilities are incorporated, the processor may calculate stroke volume and cardiac output from the impedance changes that occur during each cardiac cycle. When electrocardiography capabilities are incorporated, the processor may analyze cardiac rhythm and timing parameters from the electrical signals.

The calculated biometric properties may be stored in the memory of the NIRS monitoring wearable system along with the raw optical signals and timestamps that allow synchronization with clinical events and external data sources. The data storage component may employ compression algorithms to reduce the storage space required for physiological measurements and analysis results, enabling extended data retention within the available memory capacity. The compression algorithms may employ differential encoding, predictive coding, or other techniques optimized for the characteristics of physiological data to achieve efficient compression while preserving the information content of the stored measurements.

The wireless communication module may transmit the calculated biometric properties to the external device in real-time as the measurements are acquired and processed. The encrypted data transmission may protect the confidentiality and integrity of physiological data during wireless transmission. The adaptive bandwidth management may adjust data transmission parameters based on available communication bandwidth and data transmission requirements, reducing the data transmission rate when bandwidth is limited and increasing the rate when bandwidth is abundant.

The external device may receive the transmitted physiological data and may display the data through the user interface of the NIRS monitoring application. The user interface may display real-time physiological status information including tissue oxygenation values, pulse oximetry values, pulse rate, respiratory rate, and other biometric properties. The user interface may also display raw optical data from the photodetectors, accelerometer data, and trend graphs showing changes in physiological parameters over time. The customizable visualization modes may allow users to select different display formats and data presentations based on their preferences and the requirements of specific monitoring applications.

The power management circuitry may monitor the power requirements of the various system components throughout the operational workflow and may adjust power delivery to optimize battery life while maintaining measurement performance. The adaptive power consumption capability may reduce power consumption during periods of lower activity or when full measurement capability is not required. The duty cycle sampling mode may sample at the target rate during active sampling periods and reduce power consumption during inactive periods between sampling bursts, extending battery life during extended monitoring periods.

The operational workflow may continue in a continuous loop of data acquisition, signal processing, biometric calculation, data storage, and wireless transmission for the duration of the monitoring session. The processor may continuously process the optical signals and update the calculated biometric properties at the sampling rate of the system. The memory may continuously buffer incoming data and store processed measurements. The wireless communication module may continuously transmit physiological data to the external device when connected, or the system may store data locally when operating in save mode disconnected from external devices.

The system components may interact to support multi-depth tissue analysis by coordinating the operation of light sources at multiple wavelengths with detectors positioned at multiple source-detector distances. The control circuit may sequence through the light source activations and detector signal collections to acquire a complete set of multi-wavelength, multi-distance measurements within each sampling period. The processor may apply the multi-depth tissue analysis algorithms to the acquired measurements to calculate oxygenation values at shallow, mid-depth, and deep tissue compartments. The multi-depth measurements may be displayed separately on the external device or may be combined to provide a comprehensive assessment of tissue oxygenation status across the full range of accessible tissue depths.

The system components may interact to support real-time oxygenation monitoring by maintaining continuous data acquisition and processing throughout the monitoring session. The control circuit may maintain the target sampling rate by coordinating the timing of light source activations and detector signal collections. The processor may complete the signal processing and biometric calculations within the time available between successive measurement samples. The memory buffering may accommodate variations in processing load and communication bandwidth to maintain continuous data acquisition without data loss. The wireless communication module may transmit physiological data with minimal latency to provide real-time display on the external device.

The system components may interact to support clinical assessment across heart failure, cardiotoxicity, and shock monitoring applications by providing the measurement capabilities and data analysis functions required for each application. For heart failure assessment, the system may provide tissue oxygenation kinetics measurements during vascular occlusion testing, six-minute walk tests, and postural reactivity assessments. The processor may extract deoxygenation and reoxygenation slopes from the tissue oximetry signals and may compare the extracted parameters to reference values or threshold criteria to characterize cardiovascular function and differentiate patients based on disease severity.

For cardiotoxicity monitoring during chemotherapy treatment, the system may provide continuous tissue oxygenation and cardiovascular parameter monitoring during chemotherapy infusion sessions. The processor may track changes in tissue oxygenation, cardiac output, stroke volume, and other cardiovascular parameters during and following chemotherapy administration. The analysis engine may compare pre-treatment and during-treatment cardiovascular parameters to identify patients who exhibit sensitivity to cardiotoxic effects of chemotherapeutic agents.

For shock monitoring applications including hemorrhagic shock and cardiogenic shock, the system may provide continuous tissue oxygenation monitoring that tracks with hemodynamic status and reflects the adequacy of tissue perfusion. The processor may analyze tissue oxygenation trends to detect deterioration in perfusion status, to assess the effectiveness of resuscitation interventions, and to provide resuscitation endpoints based on tissue perfusion rather than blood pressure alone. The analysis engine may classify patients according to shock severity staging systems based on tissue oxygenation and perfusion measurements.

The operational workflow may be adapted for different clinical assessment protocols by configuring the data acquisition parameters, signal processing algorithms, and analysis functions appropriate for each protocol. For vascular occlusion testing, the system may be configured to record tissue oxygenation continuously during cuff inflation and deflation, with the analysis engine configured to extract deoxygenation slopes during the occlusion period and reoxygenation slopes during the reperfusion period. The timing of cuff inflation and deflation may be recorded through event markers that synchronize the tissue oxygenation data with the protocol events.

For six-minute walk test assessment, the system may be configured to record tissue oxygenation at multiple body sites during the walking activity, with the analysis engine configured to calculate baseline tissue oxygenation values, percent change in tissue oxygenation during activity, and deoxygenation rates during the initial phase of the walk test. The test onset time and distance traveled may be recorded in conjunction with the tissue oxygenation data to allow correlation of oxygenation kinetics with functional exercise capacity.

For postural reactivity assessment, the system may be configured to record tissue oxygenation continuously during the supine baseline period, during the transition from supine to standing, and during the standing period. The analysis engine may be configured to calculate tissue oxygenation differences between supine and standing positions and to extract deoxygenation rates during the postural transition. The blood pressure measurements recorded at specified time points may be synchronized with the tissue oxygenation data to allow correlation of tissue oxygenation changes with blood pressure changes during the postural challenge.

The operational workflow may support autonomous operation when the NIRS monitoring wearable system is disconnected from external devices. The duty cycle sampling mode may be activated by pressing a button on the sensor for a specified duration, causing the system to independently collect data until the battery is depleted or the system is powered off. The data may be stored on the internal flash memory and may be retrieved later by reconnecting the system to an external device and initiating a data transfer operation. The autonomous operation capability may support extended monitoring during daily activities, ambulatory monitoring, and remote patient monitoring applications where continuous connection to an external device may not be practical.

The operational workflow may support data transfer from the internal flash memory to external devices or cloud storage services following autonomous monitoring sessions. The data transfer may be initiated through the external device application, with the stored data transmitted from the internal flash memory to the external device over the wireless communication interface. The data transfer process may include verification steps that confirm successful transfer of all stored data before clearing the internal flash memory to make capacity available for subsequent monitoring sessions. The transferred data may be uploaded to cloud storage services for remote access, long-term archival, and sharing with healthcare providers.

The system components may interact to support predictive analytics for trend analysis by maintaining historical data and applying analysis algorithms that identify patterns and predict future values. The processor may analyze the time series of physiological measurements to identify trends, detect changes from baseline values, and forecast future values based on observed patterns. The predictive analytics may generate alerts when trend analysis indicates that physiological parameters are likely to exceed threshold values within a specified time horizon, enabling proactive clinical intervention.

The system components may interact to support automated alert generation by monitoring physiological parameters against predetermined thresholds and activating feedback mechanisms when threshold violations occur. The processor may continuously compare calculated biometric properties to configured threshold values and may generate alerts when parameters exceed or fall below the thresholds. The alerts may be presented through visual indicators on the external device display, audible alarms, haptic feedback through vibration of the sensor or external device, or notifications transmitted to clinical monitoring systems.

The coordinated interaction of the light sources, detectors, flexible substrate, processor, memory, wireless communication module, and power management circuitry may enable the NIRS monitoring wearable system to provide continuous physiological monitoring across diverse clinical applications and care settings. The operational workflow from sensor placement through data acquisition, signal processing, biometric calculation, data storage, and wireless transmission may support real-time assessment of tissue oxygenation and cardiovascular function. The system architecture may enable multi-depth tissue analysis, adaptive calibration for diverse patient populations, and integration with clinical workflows and information systems to support improved patient care through continuous tissue oxygenation monitoring.

*Example Hardware Components and Design*

The NIRS monitoring wearable system may comprise hardware components configured to provide tissue oxygenation and pulse oximetry measurements through near-infrared spectroscopy and photoplethysmography sensing modalities. The hardware architecture may include a sensor subsystem, an adhesive patch subsystem, and a human interface display subsystem that work together to enable continuous physiological monitoring.

The sensor subsystem may comprise an optics printed circuit board (PCB) and a control PCB connected by a flexible circuit region. The optics PCB may be responsible for optical sampling and biosignal measurement, implementing time-multiplexed emitter driver circuitry that sequences through multiple emitter channels across multiple detectors. The control PCB may house communication, control, memory, and user interface modules that manage device operation and data transmission.

The optics PCB may include NIRS emitters configured to emit light at wavelengths of approximately 735 nm, 810 nm, and 850 nm for tissue oxygenation measurements. The optics PCB may also include PPG emitters configured as multiwavelength light sources emitting at approximately 535 nm, 660 nm, and 940 nm for photoplethysmography and pulse oximetry measurements. In some aspects, the system may include two multiwavelength PPG emitters to provide redundancy and improved signal quality.

The optics PCB may include four detectors positioned at source-detector distances of approximately 8 mm, 30 mm, 35 mm, and 40 mm from the emitters. The detector positioned at approximately 8 mm may be configured primarily for PPG signal acquisition, while the detectors positioned at approximately 30 mm, 35 mm, and 40 mm may be configured for NIRS tissue oxygenation measurements at varying tissue depths. The multi-distance detector configuration may enable simultaneous measurement of superficial and deep tissue oxygenation.

The optics PCB may include emitter current control circuitry capable of providing current up to approximately 120 mA to drive the optical emitters. The emitter brightness may be adjusted through a digital-to-analog converter (DAC) current sink that controls the emitter drive current. A time-multiplexed emitter driver MOSFET bank may sequentially activate individual emitters to prevent optical crosstalk between different wavelength channels.

The optics PCB may include dual-stage amplification circuitry with ambient light cancellation for processing the detected optical signals. The amplification circuitry may include a transimpedance amplifier (TIA) stage and a programmable gain amplifier (PGA) stage to convert the photodetector current signals to voltage signals suitable for analog-to-digital conversion. Low dropout voltage regulators may be used to reduce noise on sensitive analog signals, and areas under sensitive signal traces may be left clear of additional traces to minimize interference.

The optics PCB may include a six-channel simultaneous sampling analog-to-digital converter (ADC) that digitizes optical signals at multiple points in the signal chain. The simultaneous sampling capability may enable synchronized acquisition of signals from multiple detectors, reducing timing errors that could affect multi-depth tissue analysis calculations. Precision resistors may be used throughout the analog signal chain to improve measurement accuracy.

The control PCB may include a dual-core processor comprising a processing core and a Bluetooth Low Energy (BLE) core. The processing core may execute the sampling state machine, control peripherals, manage sample scheduling, and calculate tissue oxygenation and pulse oximetry values using signal processing algorithms. The BLE core may transmit calculated metrics and telemetry data to the human interface display over a wireless communication link.

The control PCB may include a GPIO expander that controls sampling of optical attenuation by managing the sequencing of emitter activations and detector signal acquisitions. The GPIO expander may interface with the emitter driver circuitry on the optics PCB to implement the time-multiplexed sampling scheme.

The control PCB may include non-volatile memory (NVM) that stores calibration coefficients for analog circuit components. The calibration coefficients may include values for resistor calibration, detector calibration, and emitter calibration that are determined during manufacturing using spectroradiometer measurements. The stored calibration coefficients may be applied during signal processing to compensate for component-to-component variations.

The control PCB may include a real-time clock (RTC) that provides timestamping of operational data. The timestamps may be used to synchronize physiological measurements with clinical events, to calculate time-based parameters such as pulse rate and respiratory rate, and to support data logging and retrieval functions.

The control PCB may include flash memory with a capacity of approximately 4 Gb that stores performance and telemetry events. The flash memory capacity may be sufficient to store approximately 50 days of performance and telemetry data, supporting extended autonomous monitoring and data logging applications.

The control PCB may include power and function pushbuttons that allow users to power the device on and off and to illuminate status indicators. The power button may be configured to activate the device when pressed for a specified duration, and the function button may be configured to display battery status or initiate other user-selectable functions.

The control PCB may include battery, status, and heartbeat indicator LEDs that show system status details to the user on demand. The battery indicator may display the current battery charge level through a multi-segment LED display. The status indicator may illuminate in different colors to indicate normal operation, fault conditions, or other operational states. The heartbeat indicator may provide visual confirmation that the device is actively sampling and processing data.

The control PCB may include a system-on-chip (SoC) with encryption capabilities that enable secure data transmission over the BLE wireless link. The encryption capabilities may protect patient data during wireless transmission and may support compliance with healthcare data security requirements.

The control PCB may include circuitry that supports over-the-air firmware updates. The firmware update capability may allow the device software to be updated in the field without requiring physical connection to a programming interface, supporting ongoing improvements and bug fixes after device deployment.

The sensor subsystem may include system monitoring circuitry that oversees safety parameters during device operation. The system monitoring circuitry may include current monitors for system current and emitter functionality monitoring. A total sensor current monitor may detect abnormal power consumption that could indicate component failures or fault conditions. An emitter current monitor may verify that each emitter is functioning correctly during the sampling sequence.

The sensor subsystem may include thermistors for board temperature detection positioned near the emitters and detectors. The temperature sensors may detect overheating conditions that could affect measurement accuracy or patient safety. The processor may read temperature sensor values and may generate operational error alerts when temperature thresholds are exceeded.

The sensor subsystem may include a watchdog timer for system crash recovery. The watchdog timer may reset the processor if the firmware fails to execute properly, providing automatic recovery from software faults that could otherwise require manual intervention.

The sensor subsystem may include electrostatic discharge (ESD) protection circuitry positioned near ports, buttons, and emitters. ESD diodes may protect sensitive electronic components from damage due to electrostatic discharge events that could occur during handling or use. Components may be spaced away from the USB connector to provide additional ESD immunity.

The sensor subsystem may include an accelerometer that provides motion data for artifact detection and rejection. The accelerometer may detect patient movement and sensor motion that could affect optical signal quality. The motion data may be used by signal processing algorithms to identify and reject motion-contaminated data segments.

The sensor subsystem may include power management circuitry comprising a power management integrated circuit (PMIC) and additional linear regulators. The PMIC may control battery charging and may contain a battery gauge for monitoring battery state of charge. Independent voltage regulators may power separate rails for digital and analog circuits to ensure sufficient power and minimal noise coupling between circuit domains.

The power management circuitry may implement power optimization features including independent voltage regulator disablement between sampling periods and voltage settings at minimum operational levels to minimize quiescent power draw. The processing core and BLE core may enter sleep states during inactive periods to reduce power consumption and extend battery life.

The sensor subsystem may include an internal battery that provides portable power for device operation. The battery may be a lithium-ion or lithium-polymer battery with IEC 62133 certification and integrated short-circuit protection. In some aspects, the battery may have a capacity of approximately 350 mAh, providing sufficient energy for extended monitoring sessions. The battery may be rechargeable via a USB-C connector through the PMIC charge controller.

The sensor subsystem may include port isolation circuitry that isolates the USB charger from control PCB connections. The port isolation may be implemented through do-not-populate (DNP) jumpers that prevent direct electrical connection between the charging interface and the internal circuitry during normal operation, requiring software counters for security rather than hardware jumpers.

The sensor subsystem may include electromagnetic interference (EMI) shielding to protect sensitive electronic components from external electromagnetic fields. A shield can may be positioned over the control PCB to provide electromagnetic shielding for sensitive signals. The PCB design may include ground plane shielding and EMI film over the flex region to support electromagnetic compatibility (EMC) requirements.

The sensor subsystem may include a six-layer rigid-flex PCB with dedicated ground planes that provide electromagnetic shielding and signal integrity for sensitive analog and digital signals. The flex region of the PCB may include EMI film to maintain shielding continuity across the flexible portion of the circuit.

The sensor housing may comprise enclosure components that surround, locate, and protect the internal components while providing the form factor and user contact surfaces. The housing may include a housing top and housing bottom portions manufactured from polyamide material using three-dimensional printing processes.

The housing top may be manufactured from PA-12 polyamide material and may protect the device exterior from impact. The housing top may provide a harder plastic exterior surface over the encapsulant material and may withstand wipe down from disinfecting wipes. The housing top may include integrated features that protect the flexible circuit board region from overbending beyond approximately plus or minus 20 degrees through tensile living hinge bend stops.

The housing top may include a circular rib feature that provides headspace for battery swelling and thermal insulation. The circular rib may create an air space around the battery that accommodates volume changes during charging and discharging cycles while enhancing water tightness of the enclosure.

The housing bottom may be manufactured from PA-12 black polyamide material and may protect the device exterior from impact. The housing bottom may provide a harder plastic exterior surface over the encapsulant material and may withstand wipe down from disinfecting wipes. The housing bottom may facilitate sufficient adhesion to the adhesive securement patch for attachment to the patient.

The sensor housing may include optical lugs manufactured from PA-12 polyamide material that position and retain glass optical windows above the emitters and detectors. The optical lugs may include integrated runners on the backside and injection ports on the outer face for epoxy filling during manufacturing. The optical lugs may facilitate UV epoxy filling and curing of the optical path to eliminate air gaps between the optical components and the glass windows.

The sensor housing may include detector glass and emitter glass components manufactured from optical-grade glass material. The glass components may protect the optoelectronics from water intrusion while allowing uninterrupted optical signal transmission. The glass components may facilitate sufficient optical coupling to the patient for vital signs collection.

The glass components may include a black silkscreen on the backside that isolates optical signals from each other. The silkscreen may ensure that the optical path travels through patient tissue rather than through unintended optical paths within the optical subassembly, preventing direct light short-circuiting from emitter to detector.

The sensor housing may include UV-curable epoxy that fills the optical path between the optical components and the glass windows. The UV epoxy may act as a moisture barrier and protection for the optoelectronics, contributing to IPX7 water resistance. The UV epoxy may adhere the glass components to the optical lugs while maintaining high optical transmission at near-infrared wavelengths. The UV epoxy may be urethane-based, optically clear, and USP Class VI biocompatible for patient contact applications.

The sensor housing may include encapsulant material that fills the internal volume not occupied by other components. The encapsulant may be a polyamide-based hot melt adhesive that provides moisture barrier protection, mechanical strength, ingress protection, and impact resistance. The encapsulant may adhere all housing components together during a low-pressure molding process.

The encapsulant material may have a UL 94 V0 flammability rating and may maintain flexibility across an operating temperature range of approximately -40 to +100 degrees Celsius. The encapsulant may provide electrical insulation and dielectric protection for compliance with electrical safety standards. The encapsulant may withstand wipe down from disinfecting wipes during clinical use.

The sensor housing may include a battery insulation component manufactured from thermoplastic polyurethane (TPU) material. The battery insulation may position the battery during the encapsulation process and may act as thermal insulation to protect the battery against the elevated temperature of the encapsulant material during manufacturing. The battery insulation may minimize battery swelling and help retain battery life by preventing thermal damage during the encapsulation process.

The sensor housing may include a control PCB spacer manufactured from TPU material. The spacer may fit between the control PCB and optics PCB without causing interference with the slim stack connectors that join the two boards. The spacer may displace the thick wall of encapsulant that would otherwise occupy the space between the boards, helping to lower the temperature of the user interface half of the device during encapsulation. The spacer may also reduce the likelihood of board decoupling due to torsion or pressure applied to the device during use.

The sensor housing may include user interface symbol components manufactured from natural-colored PA-12 polyamide material. The UI symbols may act as light pipes for the underlying LEDs, forming recognizable symbols for battery fuel gauge, Bluetooth pairing, and caution or warning signals. The UI symbols may seal against the PCB to prevent encapsulant intrusion over the LEDs during the encapsulation process while acting as a moisture barrier for IPX7 water resistance.

The sensor housing may include button caps manufactured from TPU material that provide flexible covers for the tactile switches. The power button cap may include a legible power symbol for user recognition. The button caps may minimize debris traps for infection prevention and may withstand wipe down from disinfecting wipes.

The sensor subsystem may include a device label that provides identification, regulatory compliance markings, traceability, and user safety information. The label may include a unique serial number, date of manufacture, manufacturer information, and regulatory reference numbers. The label may include a QR code that facilitates quick sensor-to-HID pairing by allowing the human interface display to scan and establish a wireless connection with the sensor.

The adhesive patch subsystem may comprise a multi-layer lamination stackup of biocompatible materials that provides mechanical attachment of the sensor to user contact surfaces. The adhesive patch may be configured for single use and may support wear times of up to approximately 72 hours.

The adhesive patch may include a skin-side release liner that protects the exposed skin-side adhesive layer prior to application. The skin-side release liner may be manufactured from biocompatible material with a silicone-coated release surface that enables easy peel-off when the patch is ready for use. The skin-side release liner may include a printable surface for instructions for use or orientation indicators.

The adhesive patch may include a skin-side adhesive layer that provides gentle attachment to the patient's skin. The skin-side adhesive layer may be a double-coated adhesive that aids skin adhesion and patch lamination. The skin-side adhesive layer may be optically transparent to facilitate enhanced sensor coupling and may support skin attachment for up to approximately 72 hours of wear time.

The adhesive patch may include lamination adhesive layers that facilitate ambient light blocking through multiple non-transparent material layers. The lamination adhesive layers may be breathable and conformable to user contact surfaces. The lamination adhesive layers may include sensor optical cutouts that allow the extended optics to achieve closer proximity to the user contact surface.

The adhesive patch may include a device-side adhesive layer that provides secure attachment between the patch and the sensor housing. The device-side adhesive layer may include an aggressive adhesive compatible with the bottom sensor material for sensor attachment up to approximately 72 hours of wear time. The device-side adhesive layer may include sensor optical cutouts that allow the extended optics to interface with the patient's skin.

The adhesive patch may include a device-side release liner that protects the exposed device-side adhesive layer prior to application. The device-side release liner may be manufactured from biocompatible material with a silicone-coated release surface that enables easy peel-off when the patch is ready for use.

The human interface display (HID) subsystem may comprise a portable display and user interface platform used by clinicians to configure monitoring, view measurements and trends, acknowledge alarms, and access instructions for use. The HID may be mechanically and electronically independent from the sensor and may communicate with the sensor wirelessly using BLE 4.0.

The HID may present real-time measurements, trends, and alarms through a patient-facing application on a validated tablet display. Charts may be rendered at clinically relevant update rates with configurable threshold overlays and historical trace retrieval capabilities.

The HID may provide configuration screens for threshold settings, sensor pairing and management, and session start and stop functions. The HID may implement role-based access control and audit logging for security and compliance purposes.

The HID may provide audible alarm annunciation through the tablet speaker or an optional external wired speaker. The HID may implement alarm escalation, muting, and acknowledgement flows according to an alarm taxonomy defined in the system requirements.

The HID may provide secure connectivity through BLE encrypted pairing with the sensor. Session logs may be stored on the device with secure export capabilities in signed CSV or JSON formats, or upload via approved network connections. Local log retention may be configured according to system requirements and enterprise policy.

The system may be configured for use in multiple configurations including a monitoring configuration and a charging configuration. In the monitoring configuration, the sensor may be attached to the patient within the patient environment in a medically used room, operating as a Type BF defibrillation-proof internally powered device. In the charging configuration, the sensor and HID may be connected to charging blocks outside the patient environment, which may be in a non-medically used room.

The system may provide essential performance including tissue oximetry accuracy with root mean square error less than 10 percent over the declared range, pulse oximetry accuracy with root mean square error less than or equal to 4 percent over the 70 to 100 percent saturation range, and pulse rate accuracy with error less than or equal to plus or minus 3 beats per minute or plus or minus 3 percent, whichever is greater. The system may provide indication of abnormal operation including signal inadequacy indicators, probe fault indicators, and data update period indicators.

The system may maintain basic safety and essential performance during electromagnetic compatibility immunity tests, including no component failures, no change in programmable parameters or settings, no reset to default settings, no change of operating mode, and less than 4 percent deviation of tissue oximetry and pulse oximetry outputs.

In some examples, disclosed systems or methods may involve one or more of the following clauses:

Clause 1: A system for detecting oximetry parameters of a user, the system comprising: a reusable, wireless, and flexible near-infrared spectroscopy (NIRS) monitoring wearable system configured to be placed on a body part of a user, the NIRS system comprising: a first component comprising: a plurality of light sources configured to emit visible and infrared light at a plurality of wavelengths including at least five distinct wavelengths; and a first detector configured to detect a first plurality of signals from the plurality of light sources at a first source-detector distance of approximately 6 millimeters (mm) to 10 mm, wherein the first component has a first length of about 25 mm to about 45 mm, a first width of about 25 mm to about 45 mm, a first height of about 8 mm to about 16 mm, and comprises integrated optical isolation barriers; a second component comprising a plurality of second detectors configured to detect a second plurality of signals from the plurality of light sources at multiple source-detector distances ranging from about 25 mm to about 45 mm, wherein the second component has a second length of about 25 mm to about 45 mm, a second width of about 25 mm to about 45 mm, and a second height of about 8 mm to about 16 mm; a processor configured to execute one or more multi-depth tissue analysis algorithms; a memory configured to utilize a multi-threading processing approach and comprising dedicated buffering for continuous data streaming; and a rechargeable battery having a lifespan of at least two days with power management circuitry for adaptive power consumption, wherein the NIRS system is configured to: continuously process the plurality of signals using differential path length factor corrections; and responsive to continuously processing the plurality of signals: simultaneously assess characteristics of optical tissue density at a plurality of tissue depths of the body part; and calculate one or more biometric properties of the user.

Clause 2: The system of clause 1, wherein the plurality of wavelengths include a first wavelength at approximately 705 nanometers (nm) to 765 nm, a second wavelength at approximately 805 to 865 nm, a third wavelength at approximately 505 nm to 550 nm, a fourth wavelength at approximately 635 nm to 685 nm, and a fifth wavelength at approximately 900 nm to 960 nm.

Clause 3: The system of any of clauses 1-2, wherein the NIRS system is waterproof, crush resistant, drop resistant, and low pressure over-molded, and comprises biocompatible materials.

Clause 4: The system of any of clauses 1-3, wherein the NIRS system has an Ingress Protection (IP) rating of at least IP67.

Clause 5: The system of any of clauses 1-4, wherein the NIRS system comprises: an overall width of about 25 mm to about 45 mm; an overall length of about 50 mm to about 108 mm; and an overall height of about 8 mm to about 16 mm.

Clause 6: The system of any of clauses 1-5, wherein the rechargeable battery has a lifespan of at least 3 days and comprises fast-charging capability with thermal protection.

Clause 7: The system of any of clauses 1-6, wherein the plurality of tissue depths comprise up to approximately 3.0 cm in depth.

Clause 8: The system of any of clauses 1-7, wherein at least two detectors of the plurality of second detectors are configured to detect a first signal associated with tissue oxygenation (StO2) of the body part of the user.

Clause 9: The system of any of clauses 1-8, wherein at least the first detector is configured to detect a second signal associated with pulse oximetry (SpO2) of the body part of the user.

Clause 10: The system of any of clauses 1-9, wherein: at least two light sources of the plurality of light sources are configured to emit wavelengths associated with StO2, and at least six light sources of the plurality of light sources are configured to emit wavelengths associated with SpO2.

Clause 11: The system of any of clauses 1-10, wherein the NIRS system further comprises: a flexible hinge connecting the first and second components thereby enabling the NIRS system to conform to a shape of the body part of the user while maintaining optical alignment.

Clause 12: The system of any of clauses 1-11, wherein the NIRS system further comprises: glass disposed on one or more surfaces of the plurality of light sources, the glass configured to reduce optical crosstalk between the plurality of light sources and comprising: anti-reflective coatings with wavelength-specific filtering; and a silkscreen with precision-etched optical barriers and wavelength-selective transmission properties, wherein the glass is about 90 percent opaque with controlled light transmission characteristics for optimal signal-to-noise ratio.

Clause 13: The system of any of clauses 1-12, wherein the NIRS system has a power usage of about 45 to about 85 milliwatts (mW) with dynamic power scaling based on measurement requirements.

Clause 14: The system of any of clauses 1-13, wherein the one or more biometric properties comprise one or more of tissue oxygenation (StO2), pulse oximetry (SpO2), pulse rate (PR), heart rate, respiratory rate (RR), perfusion, blood pressure, blood volume, total hemoglobin, macrovascular and microvascular hemodynamic coherence, or combinations thereof.

Clause 15: A system for continuous physiological monitoring of a user, the system comprising: a wearable near-infrared spectroscopy (NIRS) device configured to be positioned on a body part of the user, the NIRS device comprising: a plurality of light emitting diodes (LEDs) configured to emit light at multiple wavelengths including green, red, and near-infrared wavelengths, wherein the plurality of LEDs are arranged in a spatially distributed pattern with integrated thermal management and wavelength-specific power control; a plurality of photodetectors positioned at varying distances from the plurality of LEDs to enable multi-depth tissue analysis, wherein the plurality of photodetectors comprise photodiodes with dynamic gain adjustment; a flexible substrate connecting the plurality of LEDs and the plurality of photodetectors, wherein the flexible substrate enables the NIRS device to conform to contours of the body part; a processor configured to: receive optical signals from the plurality of photodetectors with simultaneous multi-channel acquisition; apply signal processing algorithms to the optical signals to determine tissue oxygenation levels; and generate real-time physiological measurements based on the tissue oxygenation levels; a wireless communication module configured to transmit the real-time physiological measurements to an external device using encrypted data transmission with adaptive bandwidth management; and a power source configured to provide continuous power to the NIRS device for extended monitoring periods with intelligent power management.

Clause 16: The system of clause 15, wherein: the plurality of LEDs comprises at least six LEDs configured to emit light at wavelengths optimized for hemoglobin absorption characteristics and arranged in a hexagonal array pattern with individual current control for each LED, and the plurality of photodetectors comprises at least four photodetectors positioned at distances ranging from about 5 mm to about 45 mm from the plurality of LEDs with angular positioning configured for tissue penetration depth analysis.

Clause 17: The system of any of clauses 15-16, wherein the wireless communication module supports one or more of Bluetooth, Wi-Fi, cellular communication protocols, or combinations thereof.

Clause 18: The system of any of clauses 15-17, wherein the power source comprises a rechargeable lithium-ion battery with wireless charging capability and integrated fuel gauge for accurate battery life prediction.

Clause 19: A system for multi-parameter physiological assessment, the system comprising: a portable near-infrared spectroscopy (NIRS) monitoring device configured for attachment to a user's body, the NIRS monitoring device comprising: an optical sensor array comprising multiple light sources and multiple photodetectors arranged in a predetermined pattern configured to enable multi-depth tissue analysis; optical isolation structures positioned between adjacent light sources to minimize optical crosstalk, wherein the optical isolation structures comprise wavelength-selective barriers with gradient refractive index profiles; a processor configured to: selectively activate the multiple light sources and collect corresponding signals from the multiple photodetectors with synchronized timing control and adaptive exposure adjustment; process the collected signals; responsive to processing the collected signals, determine multiple physiological parameters including tissue oxygenation; and provide continuous monitoring output with automated alert generation based on physiological thresholds; a data storage component configured to store physiological measurements and analysis results with compression algorithms for extended data retention; and a user interface configured to display real-time physiological status information with customizable visualization modes and alerts.

Clause 20: The system of clause 19, wherein the optical sensor array comprises at least six light sources emitting at wavelengths between about 535 nm and about 940 nm.

Clause 21: The system of any of clauses 1-14, wherein at least three detectors of the plurality of second detectors are configured to detect a first signal associated with tissue oxygenation (StO2) of the body part of the user.

Clause 22: The system of any of clauses 1-14, 21, wherein a first distance between a first light source of the plurality of light sources and the first detector is about 5 millimeters (mm) to about 11 mm.

Clause 23: The system of any of clauses 1-14, 21-22, wherein a second distance between a second light source of the plurality of light sources and a second detector of the plurality of second detectors is about 25 mm to about 32 mm.

Clause 24: The system of any of clauses 1-14, 21-23, wherein a third distance between a third light source of the plurality of light sources and a third detector of the plurality of second detectors is about 33 mm to about 37 mm.

Clause 25: The system of any of clauses 1-14, 21-24, wherein a fourth distance between a fourth light source of the plurality of light sources and a fourth detector of the plurality of second detectors is about 38 mm to about 42 mm.

Clause 26: The system of any of clauses 1-14, 21-25, wherein the NIRS system is configured to calculate the one or more biometric properties using one or more algorithms trained on multi-modal physiological data.

Clause 27: The system of any of clauses 19-20, wherein the optical isolation structures comprise opaque barriers with anti-reflective surfaces and micro-structured geometries for enhanced light containment.

Clause 28: The system of any of clauses 19-20, 27, wherein the processor is configured to operate the multiple light sources in a time-multiplexed manner to avoid interference between measurements with adaptive timing sequences based on signal quality metrics.

Clause 29: The system of any of clauses 19-20, 27-28, wherein the processor is configured to apply one or more algorithms trained on physiological data to enhance measurement accuracy with continuous learning capabilities for personalized calibration.

Clause 30: The system of any of clauses 19-20, 27-29, wherein the user interface comprises a display screen and tactile feedback elements for user interaction.

A number of implementations have been described. Nevertheless, it will be understood that various modifications may be made without departing from the spirit and scope of the disclosure. Accordingly, other implementations are within the scope of the following claims.

## Claims

1. A system for detecting oximetry parameters of a user, the system comprising:
a reusable, wireless, and flexible near-infrared spectroscopy (NIRS) monitoring wearable system (400) configured to be placed on a body part of a user, the NIRS system (400) comprising:

   a first component (402) comprising:

   a plurality of light sources (404a, 404b, 404c) configured to emit visible and infrared light at a plurality of wavelengths including at least five distinct wavelengths; and
   a first detector (408a) configured to detect a first plurality of signals from the plurality of light sources at a first source-detector distance of approximately 6 millimeters (mm) to 10 mm,

wherein the first component (402) has a first length (L1) of about 25 mm to about 45 mm, a first width (W1) of about 25 mm to about 45 mm, a first height (H1) of about 8 mm to about 16 mm, and comprises integrated optical isolation barriers;

a second component (406) comprising a plurality of second detectors (408b, 408c, 408d) configured to detect a second plurality of signals from the plurality of light sources at multiple source-detector distances ranging from about 25 mm to about 45 mm,

wherein the second component (406) has a second length (L2) of about 25 mm to about 45 mm, a second width (W1) of about 25 mm to about 45 mm, and a second height (H1) of about 8 mm to about 16 mm;

a processor (310) configured to execute one or more multi-depth tissue analysis algorithms;

a memory (324) configured to utilize a multi-threading processing approach and comprising dedicated buffering for continuous data streaming; and

a rechargeable battery (322) having a lifespan of at least two days with power management circuitry for adaptive power consumption,

wherein the NIRS system (400) is configured to:

continuously process the plurality of signals using differential path length factor corrections; and

responsive to continuously processing the plurality of signals:

simultaneously assess characteristics of optical tissue density at a plurality of tissue depths of the body part; and

calculate one or more biometric properties of the user.

2. The system of claim 1, wherein the plurality of wavelengths include a first wavelength at approximately 705 nanometers (nm) to 765 nm, a second wavelength at approximately 805 to 865 nm, a third wavelength at approximately 505 nm to 550 nm, a fourth wavelength at approximately 635 nm to 685 nm, and a fifth wavelength at approximately 900 nm to 960 nm.

3. The system of any of claims 1-2, wherein the NIRS system (400) is waterproof, crush resistant, drop resistant, and low pressure over-molded, and comprises biocompatible materials.

4. The system of any of claims 1-3, wherein the NIRS system (400) has an Ingress Protection (IP) rating of at least IP67.

5. The system of any of claims 1-4, wherein the NIRS system (400) comprises:

an overall width (W1) of about 25 mm to about 45 mm;
an overall length (L3) of about 50 mm to about 108 mm; and
an overall height (H1) of about 8 mm to about 16 mm.

6. The system of any of claims 1-5, wherein the rechargeable battery has a lifespan of at least 3 days and comprises fast-charging capability with thermal protection.

7. The system of any of claims 1-6, wherein the plurality of tissue depths comprise up to approximately 3.0 cm in depth.

8. The system of any of claims 1-7, wherein at least two detectors of the plurality of second detectors are configured to detect a first signal associated with tissue oxygenation (StO2) of the body part of the user.

9. The system of any of claims 1-8, wherein at least the first detector is configured to detect a second signal associated with pulse oximetry (SpO2) of the body part of the user.

10. The system of any of claims 1-9, wherein:

at least two light sources of the plurality of light sources are configured to emit wavelengths associated with StO2, and

at least six light sources of the plurality of light sources are configured to emit wavelengths associated with SpO2.

11. The system of any of claims 1-10, wherein the NIRS system (400) further comprises:
a flexible hinge (410) connecting the first and second components (402, 406) thereby enabling the NIRS system to conform to a shape of the body part of the user while maintaining optical alignment.

12. The system of any of claims 1-11, wherein the NIRS system (400) further comprises:
glass disposed on one or more surfaces of the plurality of light sources, the glass configured to reduce optical crosstalk between the plurality of light sources and comprising:

anti-reflective coatings with wavelength-specific filtering; and
a silkscreen with precision-etched optical barriers and wavelength-selective transmission properties,
wherein the glass is about 90 percent opaque with controlled light transmission characteristics for optimal signal-to-noise ratio.

13. The system of any of claims 1-12, wherein the NIRS system (400) has a power usage of about 45 to about 85 milliwatts (mW) with dynamic power scaling based on measurement requirements.

14. The system of any of claims 1-13, wherein the one or more biometric properties comprise one or more of tissue oxygenation (StO2), pulse oximetry (SpO2), pulse rate (PR), heart rate, respiratory rate (RR), perfusion, blood pressure, blood volume, total hemoglobin, macrovascular and microvascular hemodynamic coherence, or combinations thereof.

15. The system of any of claims 1-14, wherein the NIRS system (400) further comprises a user interface configured to display real-time physiological status information with customizable visualization modes and alerts.

100

SUBSTRATE 202

NETWORK
102

ELECTRONICS MODULE 302

FIG. 1

FIG. 2

ELECTRONICS MODULE 302

PROCESSOR
310

ENVIRONMENTAL SENSOR
312

COMMUNICATION INTERFACE
314

SOURCE
316

DETECTOR
318

I/O
320

BATTERY
322

324

MEMORY

OS
326

PROGRAM
328

330

FIG. 3

FIG. 4

FIG. 5

FIG. 6

EP 4 775 134 A1

406

400

408a

402

408b  408c  408d

404a  404b  404c

FIG. 7

FIG. 8

FIG. 9

600

START

↓

CONTINUOUSLY PROCESS A PLURALITY OF SIGNALS FROM A PLURALITY OF LIGHT SOURCES USING DIFFERENTIAL PATH LENGTH FACTOR CORRECTIONS — 602

↓

SIMULTANEOUSLY ASSESS CHARACTERISTICS OF OPTICAL TISSUE DENSITY AT A PLURALITY OF TISSUE DEPTHS OF A BODY PART OF A USER — 604

↓

CALCULATE ONE OR MORE BIOMETRIC PROPERTIES OF THE USER — 606

↓

END

FIG. 10

700

START

RECEIVE OPTICAL SIGNALS FROM A PLURALITY OF PHOTODETECTORS WITH SIMULTANEOUS MULTI-CHANNEL ACQUISITION — 702

APPLY SIGNAL PROCESSING ALGORITHMS TO THE OPTICAL SIGNALS TO DETERMINE TISSUE OXYGENATION LEVELS — 704

GENERATE REAL-TIME PHYSIOLOGICAL MEASUREMENTS BASED ON THE TISSUE OXYGENATION LEVELS — 706

END

FIG. 11

800

START

SELECTIVELY ACTIVATE MULTIPLE LIGHT SOURCES AND COLLECT CORRESPONDING SIGNALS FROM MULTIPLE PHOTODETECTORS WITH SYNCHRONIZED TIMING CONTROL AND ADAPTIVE EXPOSURE ADJUSTMENT — 802

PROCESS THE COLLECTED SIGNALS — 804

DETERMINE MULTIPLE PHYSIOLOGICAL PARAMETERS INCLUDING TISSUE OXYGENATION — 806

PROVIDE CONTINUOUS MONITORING OUTPUT WITH AUTOMATED ALERT GENERATION BASED ON PHYSIOLOGICAL THRESHOLDS — 808

END

FIG. 12

Europäisches
Patentamt

European
Patent Office

Office européen
des brevets

# EUROPEAN SEARCH REPORT

**Application Number**

EP 26 15 1036

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 556 291 A1 (UNIV MASSACHUSETTS [US]) 23 October 2019 (2019-10-23) * paragraphs [0005], [0052] - [0053], [0080], [0086] - [0087], [0098] - [0100], [0109] * * paragraphs [0123], [0143], [0156], [0198] - [0199], [0212] - [0213] * * figures 1B, 11, 13A, 34 * | 1-15 | INV. A61B5/00 A61B5/1455 |
| A | KIM KWANG BOK ET AL: "Photoplethysmography in Wearable Devices: A Comprehensive Review of Technological Advances, Current Challenges, and Future Directions", ELECTRONICS, [Online] vol. 12, no. 13, 3 July 2023 (2023-07-03), page 2923, XP093220190, Basel, Switzerland ISSN: 2079-9292, DOI: 10.3390/electronics12132923 Retrieved from the Internet: URL:https://www.mdpi.com/2079-9292/12/13/2923/pdf> [retrieved on 2026-02-10] * page 7 * * figure 3 * | 1-15 | |
| X | US 2024/081697 A1 (ALLEN JARED DALE [US] ET AL) 14 March 2024 (2024-03-14) * paragraphs [0073] - [0086], [0100] - [0101] * * figures 3C, çA-çB * | 1-15 | TECHNICAL FIELDS SEARCHED (IPC) A61B |
| A | WO 2024/253936 A1 (NIRSENSE LLC [US]) 12 December 2024 (2024-12-12) * paragraph [0064] * | 1-15 | |

-/--

The present search report has been drawn up for all claims

| Place of search | Date of completion of the search | Examiner |
|---|---|---|
| The Hague | 10 February 2026 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

## EUROPEAN SEARCH REPORT

**Application Number**

EP 26 15 1036

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 3 057 138 A1 (TAIWAN BIOPHOTONIC CORP [CN]) 17 August 2016 (2016-08-17)<br>* paragraphs [0012], [0070] *<br>----- | 1-15 | |

TECHNICAL FIELDS
SEARCHED     (IPC)

The present search report has been drawn up for all claims

| Place of search | Date of completion of the search | Examiner |
|---|---|---|
| The Hague | 10 February 2026 | Costa Angeli, M |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 26 15 1036

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2026

| Patent document cited in search report | | | Publication date | Patent family member(s) | | | Publication date |
|---|---|---|---|---|---|---|---|
| EP 3556291 | A1 | | 23-10-2019 | CA | 2732996 | A1 | 14-05-2010 |
| | | | | CN | 102176864 | A | 07-09-2011 |
| | | | | EP | 2323554 | A2 | 25-05-2011 |
| | | | | EP | 3556291 | A1 | 23-10-2019 |
| | | | | JP | 5671460 | B2 | 18-02-2015 |
| | | | | JP | 2011530351 | A | 22-12-2011 |
| | | | | KR | 20110053993 | A | 24-05-2011 |
| | | | | US | 2011205535 | A1 | 25-08-2011 |
| | | | | WO | 2010053617 | A2 | 14-05-2010 |
| US 2024081697 | A1 | | 14-03-2024 | EP | 4312765 | A1 | 07-02-2024 |
| | | | | US | 2024081697 | A1 | 14-03-2024 |
| | | | | US | 2024398292 | A1 | 05-12-2024 |
| | | | | WO | 2022212951 | A1 | 06-10-2022 |
| WO 2024253936 | A1 | | 12-12-2024 | NONE | | | |
| EP 3057138 | A1 | | 17-08-2016 | CN | 105891136 | A | 24-08-2016 |
| | | | | CN | 105895594 | A | 24-08-2016 |
| | | | | CN | 105895595 | A | 24-08-2016 |
| | | | | CN | 105895641 | A | 24-08-2016 |
| | | | | CN | 105895642 | A | 24-08-2016 |
| | | | | CN | 105895643 | A | 24-08-2016 |
| | | | | EP | 3057138 | A1 | 17-08-2016 |
| | | | | EP | 3057139 | A2 | 17-08-2016 |
| | | | | EP | 3057140 | A1 | 17-08-2016 |
| | | | | EP | 3057141 | A2 | 17-08-2016 |
| | | | | EP | 3057142 | A2 | 17-08-2016 |
| | | | | EP | 3059765 | A1 | 24-08-2016 |
| | | | | JP | 6211636 | B2 | 11-10-2017 |
| | | | | JP | 6219987 | B2 | 25-10-2017 |
| | | | | JP | 6219988 | B2 | 25-10-2017 |
| | | | | JP | 6219989 | B2 | 25-10-2017 |
| | | | | JP | 6441839 | B2 | 19-12-2018 |
| | | | | JP | 6496256 | B2 | 03-04-2019 |
| | | | | JP | 2016147052 | A | 18-08-2016 |
| | | | | JP | 2016148657 | A | 18-08-2016 |
| | | | | JP | 2016148658 | A | 18-08-2016 |
| | | | | JP | 2016149540 | A | 18-08-2016 |
| | | | | JP | 2016149541 | A | 18-08-2016 |
| | | | | JP | 2016149542 | A | 18-08-2016 |
| | | | | TW | 201629437 | A | 16-08-2016 |
| | | | | TW | 201629438 | A | 16-08-2016 |
| | | | | TW | 201629439 | A | 16-08-2016 |
| | | | | TW | 201629464 | A | 16-08-2016 |
| | | | | TW | 201629520 | A | 16-08-2016 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 26 15 1036

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2026

| Patent document cited in search report | Publication date | Patent family member(s) | Publication date |
|---|---|---|---|
| | | TW 201630209 A | 16-08-2016 |
| | | US 2016238439 A1 | 18-08-2016 |
| | | US 2016238440 A1 | 18-08-2016 |
| | | US 2016238441 A1 | 18-08-2016 |
| | | US 2016238443 A1 | 18-08-2016 |
| | | US 2016238444 A1 | 18-08-2016 |
| | | US 2016240721 A1 | 18-08-2016 |
| | | US 2019137332 A1 | 09-05-2019 |

EPO FORM P0459

For more details about this annex : see Official Journal of the European Patent Office, No. 12/82

page 2 of 2

**REFERENCES CITED IN THE DESCRIPTION**

**Patent documents cited in the description**

- US 63743499